# EUROPEAN PATENT APPLICATION

(11) **EP 1 437 593 A1**
(43) Date of publication of application: **14.07.2004**
(21) Application number: 02765579.4
(22) Date of filing: 18.09.2002
(51) Int. Cl.: G01N 33/53, C12N 15/86, C12N 15/45, C12N 7/01

(54) **METHOD OF EXAMINING (-)-STRAND RNA VIRUS VECTOR HAVING LOWERED ABILITY TO FORM GRAINS AND METHOD OF CONSTRUCTING THE SAME**

(30) Priority: 18.09.2001 JP 2001283451; 21.11.2001 JP 2001356336
(71) Applicant: Dnavec Research Inc., Tsukuba-shi, Ibaraki 305-0856 (JP)
(72) Inventor: INOUE, Makoto, c/o DNAVEC RESEARCH INC., Tsukuba-shi, Ibaraki 305-0856 (JP); TOKUSUMI, Yumiko, c/o DNAVEC RESEARCH INC., Tsukuba-shi, Ibaraki 305-0856 (JP); IIDA, Akihiro, c/o DNAVEC RESEARCH INC., Tsukuba-shi, Ibaraki 305-0856 (JP); HASEGAWA, Mamoru, c/o DNAVEC RESEARCH INC., Tsukuba-shi, Ibaraki 305-0856 (JP)
(74) Representative: VOSSIUS & PARTNER
(86) International application number: PCT/JP2002/009558
(87) International publication number: WO 2003/025570

(57) **Abstract**

The present invention provides methods for testing and producing (-)strand RNA virus vectors with reduced or eliminated particle formation ability or cytotoxicity. It was revealed that a deficiency in M protein localization in cells introduced with such a (-)strand RNA virus vector could result in the suppression of virus-like particle (VLP) formation in the cells. The present invention provides methods for testing and screening for a (-)strand RNA virus vector in which particle formation ability has been reduced or eliminated, and methods for producing a recombinant (-)strand RNA virus vector in which particle formation ability has been reduced or eliminated. Such a vector, in which VLP formation has been reduced or eliminated, is extremely useful as a vector for gene therapy, since it neither induces cytotoxicity nor immune response due to the secondary release of viruses from cells in which it has been introduced.

SEQUENCE LISTING

<110> DNAVEC RESEARCH INC.

<120> METHODS FOR TESTING AND PRODUCING (-)-STRAND RNA VIRUS VECTORS WITH
REDUCED PARTICLE FORMATION ABILITY

<130> D3-A0105Y1P

<140>
<141>

<150> JP 2001-283451
<151> 2001-09-18

<150> JP 2001-356336
<151> 2001-11-21

<160> 38

<170> PatentIn Ver. 2.0

<210> 1
<211> 10
<212> DNA
<213> Artificial Sequence

<220>

<223> Description of Artificial Sequence: artificially

      synthesized sequence

<400> 1

ctttcaccct                                                            10

<210> 2

<211> 15

<212> DNA

<213> Artificial Sequence

<220>

<223> Description of Artificial Sequence: artificially

      synthesized sequence

<400> 2

tttttcttac tacgg                                                      15

<210> 3

<211> 18

<212> DNA

<213> Artificial Sequence

<220>

<223> Description of Artificial Sequence: artificially
      synthesized sequence

<400> 3
cggccgcaga tcttcacg                                                    18

<210> 4
<211> 39
<212> DNA
<213> Artificial Sequence

<220>
<223> Description of Artificial Sequence: artificially
      synthesized sequence

<400> 4
gaaacaaaca accaatctag agagcgtatc tgacttgac                             39

<210> 5
<211> 39
<212> DNA
<213> Artificial Sequence

<220>
<223> Description of Artificial Sequence: artificially
      synthesized sequence

<400> 5

gtcaagtcag atacgctctc tagattggtt gtttgtttc 39

<210> 6

<211> 31

<212> DNA

<213> Artificial Sequence

<220>

<223> Description of Artificial Sequence: artificially

synthesized sequence

<400> 6

attacggtga ggagggctgt tcgagcagga g 31

<210> 7

<211> 31

<212> DNA

<213> Artificial Sequence

<220>

<223> Description of Artificial Sequence: artificially

synthesized sequence

<400> 7

ctcctgctcg aacagccctc ctcaccgtaa t 31

<210> 8
<211> 33
<212> DNA
<213> Artificial Sequence

<220>
<223> Description of Artificial Sequence: artificially
synthesized sequence

<400> 8
ggggcaatca ccatatccaa gatcccaaag acc 33

<210> 9
<211> 33
<212> DNA
<213> Artificial Sequence

<220>
<223> Description of Artificial Sequence: artificially
synthesized sequence

<400> 9
ggtctttggg atcttggata tggtgattgc ccc 33

<210> 10

<211> 37

<212> DNA

<213> Artificial Sequence

<220>

<223> Description of Artificial Sequence: artificially

      synthesized sequence

<400> 10

catgctctgt ggtgacaacc cggactaggg gttatca                                37

<210> 11

<211> 37

<212> DNA

<213> Artificial Sequence

<220>

<223> Description of Artificial Sequence: artificially

      synthesized sequence

<400> 11

tgataacccc tagtccgggt tgtcaccaca gagcatg                                37

<210> 12

<211> 41

<212> DNA

<213> Artificial Sequence

<220>

<223> Description of Artificial Sequence: artificially

synthesized sequence

<400> 12

cttgtctaga ccaggaaatg aagagtgcaa ttggtacaat a 41

<210> 13

<211> 41

<212> DNA

<213> Artificial Sequence

<220>

<223> Description of Artificial Sequence: artificially

synthesized sequence

<400> 13

tattgtacca attgcactct tcatttcctg gtctagacaa g 41

<210> 14

<211> 13

<212> PRT

<213> Artificial Sequence

<220>

<223> Description of Artificial Sequence: artificially

      synthesized sequence

<400> 14

Met Ala Asp Ile Tyr Arg Phe Pro Lys Phe Ser Tyr Glu

  1               5                   10

<210> 15

<211> 12

<212> PRT

<213> Artificial Sequence

<220>

<223> Description of Artificial Sequence: artificially

      synthesized sequence

<400> 15

Leu Arg Thr Gly Pro Asp Lys Lys Ala Ile Pro His

  1               5                   10

<210> 16

<211> 13

<212> PRT
<213> Artificial Sequence

<220>
<223> Description of Artificial Sequence: artificially
      synthesized sequence

<400> 16
Asn Val Val Ala Lys Asn Ile Gly Arg Ile Arg Lys Leu
 1               5                   10

<210> 17
<211> 34
<212> DNA
<213> Artificial Sequence

<220>
<223> Description of Artificial Sequence: artificially
      synthesized sequence

<400> 17
tcgacaccag gtatttaaat taattaatcg cgag                                34

<210> 18
<211> 34

<212> DNA

<213> Artificial Sequence

<220>

<223> Description of Artificial Sequence: artificially

synthesized sequence

<400> 18

ctagctcgcg attaattaat ttaaatacct ggtg 34

<210> 19

<211> 39

<212> DNA

<213> Artificial Sequence

<220>

<223> Description of Artificial Sequence: artificially

synthesized sequence

<400> 19

gttctatctt cctgactcta tagacctgga cacgcttac 39

<210> 20

<211> 39

<212> DNA

<213> Artificial Sequence

<220>

<223> Description of Artificial Sequence: artificially

synthesized sequence

<400> 20

gtaagcgtgt ccaggtctat agagtcagga agatagaac 39

<210> 21

<211> 43

<212> DNA

<213> Artificial Sequence

<220>

<223> Description of Artificial Sequence: artificially

synthesized sequence

<400> 21

ctacctattg agccccttag ttgacgaaga taaagatagg cta 43

<210> 22

<211> 43

<212> DNA

<213> Artificial Sequence

<220>

<223> Description of Artificial Sequence: artificially

synthesized sequence

<400> 22

tagcctatct ttatcttcgt caactaaggg gctcaatagg tag 43

<210> 23

<211> 41

<212> DNA

<213> Artificial Sequence

<220>

<223> Description of Artificial Sequence: artificially

synthesized sequence

<400> 23

caagataatc gatcaggtaa agagagtaga gtctctggga g 41

<210> 24

<211> 41

<212> DNA

<213> Artificial Sequence

<220>

<223> Description of Artificial Sequence: artificially

synthesized sequence

<400> 24

ctcccagaga ctctactctc tttacctgat cgattatctt g                41

<210> 25

<211> 38

<212> DNA

<213> Artificial Sequence

<220>

<223> Description of Artificial Sequence: artificially

      synthesized sequence

<400> 25

ctcaaacgca tcacgtctct ttccctccaa agagaagc                38

<210> 26

<211> 38

<212> DNA

<213> Artificial Sequence

<220>

<223> Description of Artificial Sequence: artificially

      synthesized sequence

<400> 26

gcttctcttt ggagggaaag agacgtgatg cgtttgag 38

<210> 27

<211> 48

<212> DNA

<213> Artificial Sequence

<220>

<223> Description of Artificial Sequence: artificially

synthesized sequence

<400> 27

agagtcactg accaactaga tcgtgcacga ggcatcctac catcctca 48

<210> 28

<211> 48

<212> DNA

<213> Artificial Sequence

<220>

<223> Description of Artificial Sequence: artificially

synthesized sequence

<400> 28

tgaggatggt aggatgcctc gtgcacgatc tagttggtca gtgactct 48

<210> 29

<211> 55

<212> DNA

<213> Artificial Sequence

<220>

<223> Description of Artificial Sequence: artificially

      synthesized sequence

<400> 29

tctcgagtcg ctcggtacga tgaaaaagcc tgaactcacc gcgacgtctg tcgag        55

<210> 30

<211> 83

<212> DNA

<213> Artificial Sequence

<220>

<223> Description of Artificial Sequence: artificially

      synthesized sequence

<400> 30

aatgcatgat cagtaaatta caatgaacat cgaaccccag agtcccgcct attcctttgc 60

cctcggacga gtgctggggc gtc                                          83

<210> 31

<211> 22

<212> DNA

<213> Artificial Sequence

<220>

<223> Description of Artificial Sequence: artificially

synthesized sequence

<400> 31

ccaatctacc atcagcatca gc 22

<210> 32

<211> 21

<212> DNA

<213> Artificial Sequence

<220>

<223> Description of Artificial Sequence: artificially

synthesized sequence

<400> 32

ttcccttcat cgactatgac c 21

<210> 33

<211> 22

<212> DNA

<213> Artificial Sequence

<220>
<223> Description of Artificial Sequence: artificially
      synthesized sequence

<400> 33
agagaacaag actaaggcta cc                                                22

<210> 34
<211> 54
<212> DNA
<213> Artificial Sequence

<220>
<223> Description of Artificial Sequence: artificially
      synthesized sequence

<400> 34
tctcgagtcg ctcggtacga tggccaagtt gaccagtgcc gttccggtgc tcac             54

<210> 35
<211> 85
<212> DNA
<213> Artificial Sequence

<220>

<223> Description of Artificial Sequence: artificially

synthesized sequence

<400> 35

aatgcatgat cagtaaatta caatgaacat cgaaccccag agtcccgctc agtcctgctc 60

ctcggccacg aagtgcacgc agttg 85

<210> 36

<211> 24

<212> DNA

<213> Artificial Sequence

<220>

<223> Description of Artificial Sequence: artificially

synthesized sequence

<400> 36

cagatgaact tcagggtcag cttg 24

<210> 37

<211> 21

<212> DNA

<213> Artificial Sequence

<220>

<223> Description of Artificial Sequence: artificially
      synthesized sequence

<400> 37
tgggtgaatg agagaatcag c                                                21

<210> 38
<211> 16
<212> PRT
<213> Sendai virus

<400> 38
Lys Ala Cys Thr Asp Leu Arg Ile Thr Val Arg Arg Thr Val Arg Ala
 1               5                  10                  15

## Description

### Technical Field

The present invention relates to methods for testing and producing (-)strand RNA viral vectors in which particle formation ability has been reduced or eliminated.

### Background Art

In recent years, the development of genetic recombination technology for (-)strand RNA viruses, such as the Sendai virus (SeV) , has seen their enhanced use as gene transfer vectors (WO00/70055 and WO00/70070). However, a problem with these vectors is the secondary release of viruses from target cells, following vector introduction. Virions form in the cells infected with replicative viruses, and daughter viruses are then released. Virus-like particles (VLPs) have also been found to be released from cells in which F-deficient non-replicative SeV has been introduced, and such. Thus, development of viral vectors that do not produce VLPs is desired.

### Disclosure of the Invention

The present invention provides methods for testing and producing (-)strand RNA viral vectors in which particle formation ability has been reduced or eliminated.

Matrix (M) protein has been reported to play a central role in virion formation in the Sendai virus (SeV) and other (-)strand RNA viruses. For example, it has been found that over expression of the M protein of vesicular stomatitis virus (VSV) causes the budding of VLPs (Justice, P.A. *et al*., J. Virol. 69; 3156-3160 (1995)). Parainfluenza virus VLP formation is also reported to occur on mere overexpression of M protein (Coronel, E.C. *et al*., J. Virol. 73; 7035-7038 (1999)). While this kind of VLP formation, caused by M protein alone, is not observed in all (-)strand RNA viruses, M protein can be recognized as a virion formation core, shared by all (-)strand RNA viruses (Garoff, H. *et al*., Microbiol. Mol. Biol. Rev. 62; 1171-1190 (1998)).

The specific role of M protein in virion formation is summarized as follows: Virions are formed in so-called lipid rafts on the cell membrane (Simons, K. and Ikonen, E. Nature 387; 569-572 (1997)). These were originally identified as lipid fractions that were insoluble with non-ionic detergents such as Triton X-100 (Brown, D.A. and Rose, J.K. Cell 68; 533-544 (1992)). Virion formation in lipid rafts has been demonstrated for the influenza virus (Ali, A. *et al*., J. Virol. 74; 8709-8719 (2000)), measles virus (MeV; Manie, S.N. *et al*., J. Virol. 74; 305-311 (2000)), SeV (Ali, A. and Nayak, D.P. Virology 276; 289-303 (2000)), and others. At these lipid raft sites, M protein enhances virion formation, concentrating envelope proteins (also referred to as spike proteins) and ribonucleoprotein (RNP). In other words, M protein may function as a driving force for virus assembly and budding (Cathomen, T. *et al*., EMBO J. 17; 3899-3908 (1998), Mebatsion, T. *et al*., J. Virol. 73; 242-250 (1999)). In fact, M protein has been revealed to bind to the cytoplasmic tail of influenza virus spike proteins and so on (Zhang, J. *et al*., J. Virol. 74; 4634-4644 (2000)), SeV (Sanderson, C.M. *et al*. J. Virol. 67; 651-663 (1993)). It also binds with the RNP of the influenza virus (Ruigrok, R.W. *et al*., Virology 173; 311-316 (1989)), parainfluenza virus, SeV (Coronel, E.C. *et al*., J. Virol. 75; 1117-1123 (2001)), etc. Further, M proteins have been reported to form oligomers with themselves in the case of SeV (Heggeness, M.H. *et al*., Proc. Natl. Acad. Sci. USA 79; 6232-6236 (1982) and vesicular stomatitis virus, etc (VSV; Gaudin, Y. *et al*., Virology 206; 28-37 (1995), Gaudin, Y. *et al*., J. Mol. Biol. 274; 816-825 (1997)). Thus, due to the capacity of many of these virus components to bind to lipids, M protein can function as the driving force for virus assembly and budding.

Based on these findings, the present inventors thought they should focus on M protein for modifications aiming to suppress secondary particle (VLP) release. In addition, some reports suggest that envelope protein (spike protein) modification may also suppress VLP release. The following experimental examples are specific reports in which virion formation was actually suppressed: G protein deficiency in rabies virus (RV) resulted in a 1/30 reduction of VLP formation (Mebatsion, T. *et al*., Cell 84; 941-951 (1996)). When M protein was deficient, this level dropped to 1/500,000 or less (Mebatsion, T. *et al*., J. Virol. 73; 242-250 (1999)). Further, in the case of the measles virus (MeV) , cell-to-cell fusion was enhanced when M protein was deficient (Cathomen, T. *et al*., EMBO J. 17; 3899-3908 (1998)). This can be assumed to result from the suppression of virion formation. In addition, similar fusion enhancement arose with mutations in the cytoplasmic tail of F or H protein (the tail on the cytoplasmic side) (Cathomen, T. *et al*., J. Virol. 72; 1224-1234 (1998)). Specifically, the following has also been clarified with regards to SeV: When SeV proteins F and HN are on secretory pathways (specifically, when they are located in Golgi bodies, etc.), the cytoplasmic tails (of F and HN proteins) bind with M protein (Sanderson, C.M. *et al*., J. Virol. 67; 651-663 (1993), Sanderson, C.M. *et al*., J. Virol. 68; 69-76 (1994)). The present inventors assumed that this binding was important for the efficient transfer of M protein to cell membrane lipid rafts, where virions are formed. M protein was thought to bind to F and HN proteins in the cytoplasm, and as a result to be transferred to the cell membrane via F and HN protein secretory pathways.

The present inventors thought that deletion of this 'core' M protein would be the most effective way to carry out modifications aiming to suppress secondary particle release, namely, VLP release. However if the modified virus is to be used industrially, for example in gene therapy, then the process of virus production must be considered. As described above, obtaining a high titer virus is difficult using previously reported RV and MeV systems, namely, systems where expression is induced by vaccinia virus (VV)-driven T7 polymerase (Mebatsion, T. *et al*., J. Virol. 73; 242-250 (1999); Cathomen, T. *et al*., EMBO J. 17; 3899-3908 (1998)). VV used to induce expression will inevitably contaminate prepared solutions of M-deficient virus. Thus, production of a practically applicable virus is difficult.

Effective methods other than the M protein deletion method include deletion of F and HN proteins, which are considered to play roles in the transfer of M protein to cell membrane lipid rafts; or a method in which mutations are introduced to delete only the cytoplasmic tails of these proteins. However, some reports describe the presence of many VLPs , particularly in the cases of F-deficient (WO00/70070) and HN-deficient SeV (Stricker, R. and Roux, L. , J. Gen. Virol. 72; 1703-1707 (1991)). Thus this method is not thought to be effective. So far, with the exception of the cytoplasmic tail, spike protein regions expected to affect VLP formation have not been identified. Similarly, M protein regions definitely expected to affect VLP formation have not been identified. Further, virus production for industrial application should be considered, and the design of such is not simple.

To solve the problem of constructing vectors with suppressed VLP release, the present inventors considered the use of temperature-sensitive mutations in the viral gene. Mutant viral strains that can be grown at low but not high temperatures have been reported. The present inventors conceived that a mutant protein in which virion formation is suppressed at high temperature, particularly a mutant M or spike protein, could be used to suppress VLP formation in such a way that virus production could be carried out at a low temperature (for example, at 32°C), but practical application of the virus, such as for gene therapy, could be carried out at a higher temperature (for example, at 37°C). For this purpose, the present inventors constructed recombinant F gene-deficient Sendai viral vectors, which encode mutant M and mutant HN proteins, and which comprise a total of six temperature-sensitive mutations reported in M and HN proteins (three for M protein, and three for HN protein). VLP release for this virus was tested, and the level was determined to be about 1/10 or less of that of the wild-type virus. Further, immunostaining with an anti-M antibody was used to analyze M protein subcellular localization in cells in which the Sendai virus vector with suppressed VLP release had been introduced. The results showed that introduction of the virus with suppressed VLP release significantly reduced M protein aggregation on cell surfaces, compared to cells containing the introduced wild type virus. In particular, M protein condensation patterns were extremely reduced at a high temperature (38°C). The subcellular localization of M and HN proteins in cells infected with SeV containing a temperature-sensitive mutant M gene was closely examined using a confocal laser microscope. M protein localization on cell surfaces was significantly reduced, even at a low temperature (32°C), and was observed to have morphology similar to that of a microtubule. At a high temperature (37°C), M protein was localized near the central body of microtubules, that is, near the Golgi body. The addition of a microtubule-depolymerizing agent resulted in the disruption of the M protein localization structure. This occurred both in SeV comprising the temperature-sensitive M gene, and in SeV comprising the wild-type M gene. This raised the possibility that M protein actually functions localized along microtubules. These findings assert that the reduced level of secondary particle release in the case of viruses comprising introduced temperature-sensitive mutations was due to insufficient intracellular localization of M protein, which is believed to play a central role in particle formation. Thus, VLP formation can be effectively suppressed by preventing the normal intracellular localization of M protein. Furthermore, interaction with microtubules may be important for M protein function. For example, secondary particle release can be reduced by causing a problem with M protein subcellular localization, achieved by using a gene mutation or pharmaceutical agent developed to inhibit M protein transport along microtubules from Golgi bodies into the cell. Namely, the present inventors found that recombinant (-)strand RNA viral vectors, in which particle formation ability had been reduced or eliminated, could be provided by preparing (-)strand RNA viral vectors comprising a mutation leading to defective M protein localization.

For example, the M gene, or genes encoding spike proteins, such as the F gene and HN gene, are first mutagenized. Viral vectors carrying these mutant genes are then screened for vectors exhibiting aberrant M protein localization, and particularly for vectors showing reduced or eliminated M protein aggregation on the cell surface. Thus, recombinant Sendai viral vectors in which particle formation ability has been reduced or eliminated can be effectively obtained. For example, such vectors can be used to test VLP production suppression, caused by modifying the cytoplasmic tail or other regions of the spike protein. Further, various mutant viruses, including those with an M protein modification, can also be screened based on the presence or absence of M protein localization on the cell membrane. These methods can be applied to (-)strand RNA viruses as well as to SeV.

By introducing mutations to the P gene and L gene, the present inventors constructed F-deficient vectors that suppress the secondary release of viral particles, lower the degree of cytotoxicity, and maintain expression of the inserted genes over a longer period. The present inventors used the SeV P protein gene, comprising an E86K or L511F substitution mutation, and the SeV L protein gene, comprising an N1197S and K1795E substitution mutation. When compared to vectors without gene mutations, vectors with both P and L gene mutations significantly suppressed the decrease in the number of cells expressing the introduced genes after vector introduction to these cells. The degree of cytotoxicity and VLP secondary release were also clearly reduced. The present inventors also constructed viruses with mutations in the four proteins, M, HN, P and L. They did this by combining mutations in the P protein and L protein with the above-mentioned temperature-sensitive mutations in the M protein and HN protein. Use of these recombinant mutant viruses resulted in remarkably reduced cytotoxicity. In particular, the secondary release of particles was significantly decreased in SeV comprising P protein gene with an amino acid substitution at L511.

The present inventors also aimed to construct a virus in which M protein aggregation on the surface of cells introduced with the virus was completely deficient. To this end, and for the first time, the present inventors constructed cells with sustained M protein expression, able to be utilized in the production of the M-deficient virus. By using these cells it is possible for the first time to produce genetic therapy vectors confirmed to be free from contamination with other viruses, even at high titers. The M-deficient SeV production system of the present invention could supply, for the first time, practically applicable M-deficient (-)strand RNA viruses. Infective viral particles lacking the M gene were recovered in the culture supernatant of the virus-producing cells at titer of 10⁷ CIU/ml or more. Secondary release of VLPs from cells introduced with the above-mentioned virus was almost completely suppressed. Cytotoxicity was also reduced. Furthermore, the present inventors newly produced helper cells that express both M and F proteins. Using these helper cells, they succeeded for the first time in recovering infective viral particles lacking both M and F genes. The virus was recovered in the culture supernatant of virus-producing cells at titer of up to 10⁸ CIU/ml or more. Secondary particle production was almost absent in the viruses thus obtained. Cytotoxicity due to the viral vector lacking both M and F genes was significantly lower than for those lacking just one or the other of the M or F genes. This viral vector was shown to be highly efficient at both *in vivo* and *in vitro* gene transfer to nerve cells. This virus can be expected to be used as a gene-transfer vector comprising the ability to infect many kinds of cells, including non-dividing cells.

The present invention relates to methods for testing and producing (-)strand RNA viral vectors in which particle formation ability has been reduced or eliminated. More specifically, the present invention relates to:
(1) a method for testing particle formation ability of a (-)strand RNA virus vector, wherein the method comprises detecting localization of M protein in cells in which the vector has been introduced;
(2) a method of screening for a (-)strand RNA virus vector whose particle formation ability has been reduced or eliminated, comprising the steps of:
   (a) detecting localization of M protein in cells into which the vector has been introduced; and
   (b) selecting the vector by which localization has been reduced or eliminated;
(3) the method according to (1) or (2), wherein the localization of M protein is an aggregation of M proteins on the cell surface;
(4) a method of screening for a gene which reduces or eliminates particle formation ability of a (-)strand RNA virus vector, comprising the steps of:
   (a) detecting localization of M protein in cells into which the (-)strand RNA virus vector comprising a test gene has been introduced; and
   (b) selecting the gene which reduces or eliminates localization;
(5) the method according to (4), wherein the localization of M protein is an aggregation of M proteins on the cell surface;
(6) the method according to (4) or (5), wherein the test gene is a mutant of a gene selected from the group consisting of M, F, and HN genes of a (-)strand RNA virus;
(7) a method for producing a recombinant (-)strand RNA virus vector whose particle formation ability has been reduced or eliminated, wherein the method comprises reconstituting the (-)strand RNA virus vector comprising a gene which can be identified or isolated by a method according to any one of (4) to (6), under a condition where the reduction or elimination of M protein localization by the gene is continuously complemented;
(8) a method for producing a recombinant (-)strand RNA virus vector whose particle formation ability has been reduced or eliminated, wherein the method comprises reconstituting the (-)strand RNA virus vector by which the localization of the M gene expression product is reduced or eliminated as a result of the deletion or mutation of the M gene, under a condition where functional M protein is continuously expressed;
(9) the method according to (8), wherein the step comprises reconstituting, at a permissive temperature, the (-)strand RNA virus vector comprising a temperature-sensitive mutant M gene by which the aggregation of gene products on the cell surface has been reduced or eliminated;
(10) the method according to (9), wherein the temperature-sensitive mutant M gene is a gene encoding a (-)strand RNA virus M protein, in which an amino acid corresponding to at least one amino acid position selected from the group consisting of G69, T116 and A183 of a Sendai virus M protein has been substituted with another amino acid;
(11) the method according to (8), wherein the step comprises reconstituting the (-)strand RNA virus vector whose M gene is deleted, under a condition where the M gene, which has been introduced in the chromosome of the cells used for reconstitution, is expressed;
(12) a method according to any one of (7) to (11), wherein the (-)strand RNA virus vector further comprises the deletion of HN and/or F genes, or comprises a temperature-sensitive mutant HN and/or F genes;
(13) the method according to (12), wherein the temperature-sensitive mutant HN gene is a gene encoding a (-)strand RNA virus HN protein, in which an amino acid corresponding to at least one amino acid position selected from the group consisting of A262, G264, and K461 of a Sendai virus HN protein, has been substituted with another amino acid;
(14) a method according to any one of (7) to (13), wherein the (-)strand RNA virus vector further comprises a mutation in the P and/or L gene;
(15) the method according to (14), wherein the mutation in the P gene is a substitution of an amino acid position of the (-)strand RNA virus P protein, corresponding to E86 and/or L511 of a Sendai virus P protein, with another amino acid;
(16) the method according to (14) or (15), wherein the mutation in the L gene is a substitution of an amino acid position of the (-)strand RNA virus L protein, corresponding to N1197 and/or K1795 of a Sendai virus L protein, with another amino acid;
(17) a method according to any one of (7) to (16), wherein the method comprises reconstituting a vector at 35°C or a lower temperature;
(18) a method according to any one of (1) to (17), wherein the (-)strand RNA virus is a paramyxovirus;
(19) the method according to (18), wherein the paramyxovirus is a Sendai virus;
(20) a recombinant (-)strand RNA virus vector produced by a method according to any one of (7) to (14), wherein the particle formation ability of the vector has been reduced or eliminated;
(21) a recombinant (-)strand RNA virus, comprising a functional M protein, but whose M protein-encoding sequence is deleted in the genome of the virus;
(22) a recombinant (-)strand RNA virus comprising at least one feature selected from the group consisting of the following (a) to (d) :
   (a) the M protein encoded in the genome of the virus comprises a substitution of an amino acid, corresponding to at least one amino acid position selected from the group consisting of G69, T116 and A183 of a Sendai virus M protein, with another amino acid;
   (b) the HN protein encoded in the genome of the virus comprises a substitution of an amino acid, corresponding to at least one amino acid position selected from the group consisting of A262, G264, and K461 of a Sendai virus HN protein, with another amino acid;
   (c) the P protein encoded in the genome of the virus comprises a substitution of an amino acid, corresponding to the amino acid position of E86 or L511 of a Sendai virus P protein, with another amino acid;
   (d) the L protein encoded in the genome of the virus comprises a substitution of an amino acid, corresponding to the amino acid position of N1197 and/or K1795 of a Sendai virus L protein or an amino acid of another (-)strand RNA virus M protein homologous thereto, with another amino acid;
(23) the virus according to (22) comprising the features of at least (a) and (b);
(24) the virus according to (22) comprising the features of at least (c) and (d);
(25) the virus according to (22) comprising the features of all of (a) to (d);
(26) a virus according to any one of (21) to (25) , wherein at least one sequence encoding a spike protein in the genome of the virus is further deleted;
(27) the virus according to (26), wherein the spike protein is an F protein;
(28) a virus according to any one of (21) to (27), wherein the (-)strand RNA virus is a paramyxovirus;
(29) the virus according to (28), wherein the paramyxovirus is a Sendai virus;
(30) a recombinant virus according to any one of (21) to (29) , which is used for reducing cytotoxicity upon gene introduction;
(31) a recombinant virus according to any one of (21) to (30), which is used for inhibiting the reduction in the expression level of an introduced gene upon gene introduction;
(32) a recombinant virus according to any one of (21) to (31), which is used for inhibiting the release of a virus-like particle (VLP) from a cell into which a virus has been introduced upon gene transduction; and
(33) an aqueous solution comprising a recombinant virus according to any one of (21) to (32) at a level of 10⁶ CIU/ml or higher.

The present invention provides a method for testing the particle formation ability of a (-)strand RNA virus vector, which comprises the step of detecting the localization of M protein in cells in which the vector has been introduced. The present inventors showed the close relationship between the localization of M protein in vector-producing cells, and the particle formation ability of (-)strand RNA virus vectors. In cells in which these particles are formed, M protein is detected at high levels on the cell surface; more strictly, it is aggregated on the cell surface. However, in cells infected with a vector with reduced particle formation ability, M protein localization on the cell surface is reduced, and the protein is detected at higher levels in the cytoplasm. When particle formation ability is eliminated, M protein is condensed around the nucleus. In this case, M protein is condensed in regions predicted to be close to the Golgi body, suggesting abnormalities in M protein transport, in which microtubules participate. Thus, since the subcellular localization of M protein correlates to the particle formation ability of the vector, this ability can be tested by detecting M protein localization. The test of the present invention is carried out by linking M protein localization with particle formation ability by detecting M protein localization in cells introduced with a vector. In other words, vectors in which M protein localization in cells has been reduced or eliminated have low particle formation ability; the greater the interference with localization, the more particle formation ability is reduced. Specifically, and as described above, the lower the level of cell-surface M protein aggregation, the more particle formation ability in the vector is reduced. Particularly, when cell-surface M protein aggregation is eliminated in a vector, that vector is assessed to have no particle formation ability. Macroscopically, as the level of M protein localized on the cell surface is reduced, the ability of the vector to form particles is judged to be lower. A vector causing M protein to be localized not on the cell surface, but in the cytoplasm or nucleus periphery, will have a reduced ability to form particles. In particular, when M protein is condensed and localized in the vicinity of the nucleus, or aggregated in regions of the Golgi body, the vector's particle formation ability is thought to be virtually or completely eliminated. On developing a vector which causes suppression of subcellular M protein localization, particularly causing suppression of cell-surface M protein aggregation, it will be possible to suppress the release of VLPs from infected cells.

In the present invention, the reduction or elimination of M protein localization in cells refers to, for example, a deficiency in M protein cellular localization. Namely, it means a significant disturbance of "M protein localization" (which is also referred to as "the normal localization of M protein") in cells infected with a paramyxovirus retaining particle or VLP formation ability (for example, a wild-type paramyxovirus and paramyxovirus retaining VLP formation ability but comprising a deficient spike-protein gene, and so on). Specifically, a deficiency in subcellular M protein localization means an alteration or elimination of M protein localization in cells infected with a vector comprising particle or VLP formation ability. M protein is localized near the cell surface; more specifically, it is aggregated on the surfaces of cells infected with paramyxoviruses retaining normal particle or VLP formation ability. In the present invention, alteration and elimination of normal M protein localization refers to reduction and elimination of M protein localization, respectively. In the present invention, a deficiency in M protein localization can include, for example, aberrant M protein localization (different to normal M protein localization). In the present invention, deficiency of M protein localization includes, for example, reduction and elimination of cell-surface aggregation, reduction of cell surface expression, increases in M protein level in the cytoplasm, condensation of M protein in the cytoplasm (e.g. condensation in the nucleus periphery), a decrease or elimination in M protein level in the entire cell, etc.

The subcellular localization of M protein can be determined by cell fractionation, or by directly detecting M protein localization using immunostaining, or so on. In immunostaining, for example, M protein stained by a fluorescently labeled antibody can be observed under a confocal laser microscope. After the cells have been lysed, a cell fraction can be prepared using a known cell fractionation method, and localization can then be determined by identifying the M protein-containing fraction using a method such as immunoprecipitation or Western blotting using an antibody against M protein. In cells infected with a vector comprising particle formation ability, M protein is localized on cell membranes. When the level of M protein localized on cell membranes is reduced, the virus particle formation is judged to be reduced. In the method of the present invention, it is preferable to test M protein localization by detecting cell-surface M protein aggregation.

Direct methods for detecting subcellular M protein localization, such as cell fractionation, immunostaining, and others, can be used to detect cell-surface M protein aggregation. Virions are formed in so-called cell membrane lipid rafts, lipid fractions that are insoluble with non-ionic detergents such as Triton X-100. M protein is believed to participate in the aggregation of viral components in the lipid rafts due to its ability to bind to spike proteins, RNP, and to M protein itself, and further to lipids. Accordingly M protein detected by electrophoresis or so on with the lipid raft fraction is assumed to reflect aggregated M protein. Namely, when the amount of detectable M protein is reduced, cell-surface M protein aggregation is determined to be reduced. M protein aggregation on cell membranes, perhaps in lipid rafts, can be directly observed using the immunocytological staining methods used by the present inventors for detecting subcellular localization. This requires an anti-M antibody able to be used for immunocytological staining. On investigation using this method, an intensely condensed image is observed near the cell membrane when M protein is aggregated. When M protein is not aggregated, there is neither a detectable condensation pattern nor a clear outline of the cell membrane. In addition, only a slight stain is observed in the cytoplasm. Thus, when little or no condensation pattern is detected, or more preferably when the cell membrane outline is indistinct and slight staining is observed throughout the cytoplasm, cell-surface M protein aggregation is judged to be reduced. Particle formation is suppressed for these viruses in which M protein aggregation on the cell surface has been reduced.

Screening for (-)strand RNA viral vectors in which particle formation ability has been reduced or eliminated can be achieved using the above detection method. This screening method comprises the steps of (a) detecting the M protein localization in cells in which the vector has been introduced, and (b) selecting a vector with which localization has been reduced or eliminated (i.e. with which normal M protein localization has been altered). As described above, reduction or elimination of localization means that normal M protein localization is significantly disturbed. Further, reduction or elimination of localization also includes complete elimination of localization and/or of M protein. This reduction and elimination in localization also includes not only cases where localization is reduced or eliminated in all cells, but also cases where localization is reduced or eliminated in a partial cell population, or where partial localization is reduced or eliminated within single cells. In addition, it includes cases where localization is reduced or eliminated under a particular condition. For example, it also includes cases where the level of localization is equivalent to that of a wild type at a particular temperature or less, but is reduced or eliminated relative to that wild type at a higher temperature. A preferable temperature at which this higher level of localization is reduced or eliminated as compared with a wild-type virus is about 37 °C to about 38 °C, which corresponds to mammalian body temperature. For example, various mutant virus strains are infected into cells, and then subcellular M protein localization is tested. Viruses in which particle formation ability has been reduced or eliminated can be isolated by selecting viruses by which M protein localization is reduced or eliminated.

In the screening method of the present invention it is preferable use a detection method that utilizes cell-surface M protein aggregation as an index to screen for (-)strand RNA viral vectors in which particle formation ability has been reduced or eliminated. This screening method comprises the steps of (a) detecting cell-surface M protein aggregation in cells in which the vector has been introduced, and (b) selecting a vector by which aggregation has been reduced or eliminated. Reduction of aggregation refers to significant reduction of aggregation. Further, reduction of aggregation also includes the complete elimination of aggregation, and of M protein itself. Reduction or elimination of aggregation includes not only cases where aggregation is reduced or eliminated in all cells, but also cases where aggregation is reduced or eliminated on the cell surface of partial cell populations, and where aggregation is reduced or eliminated on partial cell surfaces. It also includes cases where aggregation is reduced or eliminated under a particular condition. For example, it also includes cases where the level of aggregation is equivalent to that of a wild type at a particular temperature or less, but reduced or eliminated relative to that wild type at a higher temperature. A preferable temperature at which aggregation is reduced compared to the wild-type virus, or eliminated, is about 37 to 38°C, which corresponds to mammalian body temperature. For example, various mutant virus strains are infected into cells, and then M protein localization on cell surfaces is tested. Viruses in which particle formation ability has been reduced or eliminated can be isolated by selecting a virus strains in which cell-surface M protein aggregation is reduced or eliminated.

In this invention, particle formation ability refers to the ability of a vector to release infective and non-infective viral particles from cells into which a viral vector has been infected. This release is called secondary release, and the particles are called virus-like particles (VLPs). In this invention, reduction and suppression of particle formation refers to a significant reduction in particle formation ability. This reduction in particle formation ability includes complete elimination of the ability to form particles. The reduction or elimination of particle formation ability includes cases where the average particle formation ability of a viral vector is reduced. For example, it includes cases where particle formation ability is reduced or eliminated in some of the infected cells, and cases where particle formation ability is reduced or eliminated in part of the infected virus. Furthermore, it includes cases where particle formation ability is reduced or eliminated under a certain condition. For example, it includes cases where particle formation by a transgenic virus is similar to that by a wild-type virus at a specific temperature or lower, but is reduced or eliminated relative to that wild-type virus at temperatures above the specific temperature. Temperature conditions are preferably such that reduction or elimination of particle formation ability in the transgenic virus as compared to the wild-type virus occurs at 37°C to 38°C, which corresponds to mammalian body temperature (e. g., 37°C).

The reduction of particle formation ability in the viruses is statistically significant (for example, at a significant level of 5% or less). Statistical verification can be carried out using, for example, the Student t-test or the Mann-Whitney *U*-test. Particle formation ability in the virus is reduced to a level of 1/2 or less, preferably 1/5 or less, more preferably 1/10 or less, more preferably 1/30 or less, more preferably 1/50 or less, more preferably 1/100 or less, more preferably 1/300 or less, and more preferably 1/500 or less.

Elimination of particle formation ability includes quantitative or functional elimination of particle formation. Quantitative elimination of particle formation ability refers to cases where, for example, the VLPs are below the detection limit. In these cases, the number of VLPs is 10³/ml or less, preferably 10²/ml or less, and more preferably 10¹/ml or less. Functional elimination of particle formation ability refers to cases where a sample which possibly comprises VLPs is transfected to cells but does not result in detectable infectivity. Viral particles can be directly confirmed by observation under an electron microscope, etc. Alternatively, they can be detected and quantified using viral nucleic acids or proteins as indicators. For example, genomic nucleic acids in the viral particles may be detected and quantified using general methods of nucleic acid detection such as the polymerase chain reaction (PCR). Alternatively, viral particles comprising a foreign gene can be quantified by infecting them into cells and detecting expression of that gene. Non-infective viral particles (virus-like particles, etc.) can be quantified by detecting gene expression after introducing the particles into cells in combination with a transfection reagent. Specifically, a lipofection reagent such as DOSPER Liposomal Transfection Reagent (Roche, Basel, Switzerland; Cat. No. 1811169) can be used. One hundred microliters of a solution with or without viral particles is mixed with 12.5 µl of DOSPER, and allowed to stand for ten minutes at room temperature. The mixture is shaken every 15 minutes and transfected to cells confluently cultured on 6-well plates. Virus-like particles can be detected by the presence or absence of infected cells from the second day after transfection. Viral particles or infectivity can be determined by measurement using, for example, CIU assays or hemagglutination activity (HA) (Kato, A. *et al*., 1996, Genes Cells 1: 569-579; Yonemitsu, Y. & Kaneda, Y., "Hemaggulutinating virus of Japan-liposome-mediated gene delivery to vascular cells.", Ed. by Baker AH., Molecular Biology of Vascular Diseases. Method in Molecular Medicine: Humana Press: pp. 295-306, 1999). Transfection can be carried out, for example, by using lipofection reagents. Transfection can also be performed, for example, by using DOSPER Liposomal Transfection Reagent (Roche, Basel, Switzerland; Cat No. 1811169). 12.5 µl of DOSPER is mixed with 100 µl of a solution with or without VLPs, and the mixture is allowed to stand at room temperature for ten minutes. The mixed solution is shaken every 15 minutes and transfected to cells confluently cultured on 6-well plates. The presence of VLPs can be tested by detecting the presence or absence of infected cells after two days.

A test virus to be used for the screening may be a spontaneous mutant strain or so on, or an artificially created mutant. A virus selected by screening can be used as the (-)strand RNA virus vector in which particle formation ability has been reduced or eliminated.

A gene leading to the reduction or elimination of particle formation ability in a (-)strand RNA virus vector can be screened using the above detection method. This screening method comprises the steps of (a) detecting M protein localization in cells into which the (-)strand RNA virus vector containing a test gene has been introduced, and (b) selecting genes which reduce or eliminate localization. Reduction or elimination of localization can be detected as described above. For example, the localization screening method using cell-surface M protein aggregation as an index comprises the steps of (a) detecting cell-surface M protein aggregation in cells in which the (-)strand RNA virus vector containing a test gene has been introduced, and (b) selecting genes which reduce or eliminate aggregation. For example, viral vectors in which various mutant viral genes and other genes have been inserted are prepared, and these vectors are infected into cells, followed by detection of cell-surface M protein localization expressed from the infecting virus in the cells. A gene by which particle formation ability is reduced or eliminated can be isolated by selecting a gene contained in a viral vector by which cell-surface M protein aggregation of M protein has been reduced or eliminated. There is no particular limitation as to the test gene to be used in the screening, and such a gene may include a gene derived from a virus or cell, and a gene containing spontaneous or artificially created mutations. A test gene is preferably a mutant of a gene selected from the group consisting of the M, F, and HN genes of a (-)strand RNA virus. In some cases a gene functionally equivalent to the M gene of the (-)strand RNA virus is referred to as M1, and similarly, genes functionally equivalent to the F and HN genes can be referred to as G and H, respectively. In the present invention, the M gene includes the M1 gene, and the F and HN genes include the G and H genes respectively. The term "mutant" means, for example, a gene encoding a protein carrying one or more amino acid substitutions, deletions, additions, and/or insertions compared to the wild-type gene product. When the test gene is a mutant M gene, in the step (a), localization of the expression products of the mutant M gene is detected in cells in which the (-)strand RNA virus vector containing the test mutant M gene has been introduced. When the test genes are mutant F and/or HN genes, in the step (a) , localization of M protein expressed from the vector is detected in cells in which the (-)strand RNA virus vector containing the test mutant F and/or HN genes have been introduced. A gene that reduces or eliminates M protein localization is selected by detecting M protein localization in the cells. For example, when M protein localization is tested using cell-surface aggregation as an index, a gene that reduces or eliminates M protein aggregation is selected by detecting cell-surface M protein aggregation. Similar screening can be carried out by using, for example, a test gene that encodes a protein capable of interacting with viral gene products such as the M, F, or HN proteins. The gene obtained can be used for producing a (-)strand RNA virus vector in which particle formation ability has been reduced or eliminated.

Herein, the term "a gene that reduces or eliminates M protein localization in cells" means a gene that causes a reduction or elimination of subcellular M protein localization (namely, it alters the normal localization of M protein). For example, in addition to the expression of a gene which reduces or eliminates M protein localization, this phrase also includes cases where gene expression results in deficient M protein localization, or alterations of other environmental factors (for example, pH, salt concentration, temperature, addition of compounds, co-expression of other genes, etc.).

Using this screening, a gene involved in the reduction or elimination of particle formation ability can be specified from the above viruses, which were isolated by screening for (-)strand RNA virus vectors in which particle formation ability has been reduced or eliminated. Namely, genes that reduce or eliminate particle formation ability can be screened for using genes contained in an isolated virus as test genes. Genes obtained can be used for producing the recombinant virus in which particle formation ability has been reduced or eliminated.

When using a gene isolated as described above to produce a (-)strand RNA virus vector in which particle formation ability has been reduced or eliminated, the present inventors found that (-)strand RNA virus vectors retaining such a gene can be efficiently produced by a method comprising the step of reconstitution under conditions persistently complementing the reduction or elimination of M protein localization by the gene, for example, reduction or elimination of cell-surface M protein aggregation. The recombinant (-)strand RNA virus vector in which particle formation ability has been reduced or eliminated, produced by the method of the present invention, has the advantage that it does not release VLPs after introduction into target cells.

The term "conditions persistently complementing" means conditions where complementation is sustained for a period of time long enough to reconstitute the (-)strand RNA virus vector. Conditions of persistent complementation typically mean conditions where reduction or elimination of M protein localization in cells, for example, the reduction or elimination of cell-surface M protein aggregation, is continually complemented for at least two days, preferably four days or longer, more preferably seven days or longer, further preferably ten days or longer, and most preferably 14 days or longer.

For example, to produce a vector which comprises an M gene comprising a mutation by which cell-surface aggregation is reduced under a certain condition, the virus is reconstituted while persistently maintaining that condition (under which reduction of cell-surface aggregation is suppressed). For example, reconstitution may be carried out under a condition whereby the mutant phenotype is not expressed. Alternatively, the M protein mutant may be complemented by persistently expressing the wild-type M gene in cells. As a further example, where an M gene-deficient vector is produced, reconstitution can also be performed by persistently expressing the wild-type M gene in cells.

In the present invention it was revealed that when a viral vector was reconstituted at a low temperature, the reduction or elimination of normal M protein localization was suppressed, and thus the efficiency of virus reconstitution was significantly increased. Particularly, in the reconstitution of a vector comprising a mutant (or deficient) gene by which particle formation ability was reduced, reconstitution at 37°C and 38°C was inefficient, and cytotoxicity was also observed. Efficient reconstitution could be achieved at 35°C or lower, preferably at 32°C. Accordingly, in the present invention it is preferable to reconstitute viral vectors at 35°C or lower, more preferably at 34°C or lower, further preferably at 33°C or lower, most preferably at 32°C or lower.

The present invention provides, as one such method, a method for reconstituting an M gene-deficient or M gene-mutated (-)strand RNA virus vector in particular. Namely, the present invention relates to a method for producing a recombinant (-)strand RNA virus vector in which particle formation ability has been reduced or eliminated, which comprises the step of reconstituting, under a condition where the functional M protein is persistently expressed, a (-)strand RNA virus vector that reduces or eliminates M protein localization due to M gene deficiency or mutation. Specifically, for example,' the present invention relates to a (-)strand RNA virus vector that reduces or eliminates cell-surface M protein aggregation. The term "functional M protein" means the wild-type M protein or a protein comprising a function equivalent to that of this protein. Specifically, it means a protein comprising the activity of causing cell-surface aggregation, and thus of supporting particle formation by a (-)strand RNA virus vector.

Particularly preferably embodiments of these methods include a method comprising a step of reconstituting a (-)strand RNA virus vector comprising a temperature-sensitive mutant M gene that reduces or eliminates localization of its gene product in cells, at allowable temperatures. Temperature-sensitive mutation refers to a mutation that significantly reduces an activity at high temperatures (e.g., 37°C) compared to at low temperatures (e.g. , 32°C). A specific example is a method comprising the step of reconstituting a (.-)strand RNA virus vector comprising a temperature-sensitive mutant M gene that reduces or eliminates aggregation of gene product on the cell surface, at allowable temperatures. The temperature-sensitive M gene mutation is not particular limited, however includes, for example, at least one of the amino acid sites selected from the group consisting of G69, T116, and A118 from the Sendai virus M protein, preferably two sites arbitrarily selected from among these, and more preferably all three sites. Other (-)strand RNA virus M proteins comprising homologous mutations can also be used as appropriate, and use is also not limited thereto. Herein, G69 means the 69th amino acid glycine in M protein, T116 is the 116th amino acid threonine in M protein, and A183 is the 183rd amino acid alanine in M protein.

The gene encoding M protein (the M gene) is widely conserved in (-)strand RNA viruses, and is known to interact with both the viral nucleocapsid and envelope (Garoff, H. *et al*., Microbiol. Mol. Biol. Rev. 62:117-190 (1998)). The SeV M protein amino acid sequence 104 to 119 (104-KACTDLRITVRRTVRA-119 / SEQ ID NO: 38) is presumed to form an amphiphilic α-helix, and was identified as an important region for viral particle formation (Mottet, G. *et al*., J. Gen. Virol. 80:2977-2986 (1999)). This region is widely conserved in (-)strand RNA viruses. M protein amino acid sequences are similar among (-)strand RNA viruses. In particular, known M proteins in viruses belonging to the subfamily Paramyxovirus are commonly proteins with 330 to 380 amino acid residues. Their structure is similar over the whole region, however the C-end halves in particular have high homology (Gould, A. R., Virus Res. 43:17-31 (1996) , Harcourt, B. H. *et al*., Virology 271:334-349 (2000)). Therefore, for example, amino acid residues homologous to G69, T116 and A183 of the SeV M protein can be easily identified.

Amino acid residues at sites homologous to other (-)strand RNA virus M proteins corresponding to G69, T116 and A183 of the SeV M proteins can be identified by one skilled in the art using SeV M protein alignments created using an amino acid sequence homology search program (which includes an alignment forming function) such as BLAST, or an alignment forming program such as CLUSTAL W. Examples of homologous sites in M proteins that correspond to G69 in the SeV M protein include G69 in human parainfluenza virus-1 (HPIV-1) ; G73 in human parainfluenza virus-3 (HPIV-3) ; G70 in phocine distemper virus (PDV) and canine distemper virus (CDV) ; G71 in dolphin molbillivirus (DMV) ; G70 in peste-des-petits-ruminants virus (PDPR) , measles virus (MV) and rinderpest virus (RPV); G81 in Hendra virus (Hendra) and Nipah virus (Nipah); G70 in human parainfluenza virus-2 (HPIV-2); E47 in human parainfluenza virus-4a (HPIV-4a) and human parainfluenza virus-4b (HPIV-4b); and E72 in mumps virus (Mumps). (Descriptions in brackets indicate the abbreviation; letters and numbers indicate amino acids and positions.) Examples of homologous sites of M proteins corresponding to T116 in the SeV M protein include T116 in human parainfluenza virus-1 (HPIV-1); T120 in human parainfluenza virus-3 (HPIV-3); T104 in phocine distemper virus (PDV) and canine distemper virus (CDV); T105 in dolphin molbillivirus (DMV); T104 in peste-des-petits-ruminants virus (PDPR), measles virus (MV), rinderpest virus (RPV) ; T120 in Hendra virus (Hendra) and Nipah virus (Nipah); T117 in human parainfluenza virus-2 (HPIV-2) and simian parainfluenza virus 5 (SV5); T121 in human parainfluenza virus-4a (HPIV-4a) and human parainfluenza virus-4b (HPIV-4b); T119 in mumps virus (Mumps) ; and S120 in Newcastle disease virus (NDV). Examples of homologous sites of M proteins corresponding to A183 of SeV M protein are A183 in human parainfluenza virus-1 (HPIV-1); F187 in human parainfluenza virus-3 (HPIV-3) ; Y171 in phocine distemper virus (PDV) and canine distemper virus (CDV) ; Y172 in dolphin molbillivirus (DMV) ; Y171 in peste-des-petits-ruminants virus (PDPR) ; measles virus (MV) and rinderpest virus (RPV); Y187 in Hendra virus (Hendra) and Nipah virus (Nipah); Y184 in human parainfluenza virus-2 (HPIV-2); F184 in simian parainfluenza virus 5 (SV5); F188 in human parainfluenza virus-4a (HPIV-4a) and human parainfluenza virus-4b (HPIV-4b) ; F186 in mumps virus (Mumps); and Y187 in Newcastle disease virus (NDV). Of the viruses mentioned here, viruses suitable for use in the present invention include those comprising genomes which encode an M protein mutant, where amino acid residue (s) have been substituted at any one of the above-mentioned three sites, preferably at an arbitrary two of these three sites, and more preferably at all three sites.

An amino acid mutation includes substitution with any other desirable amino acid. However, substitution is preferably with an amino acid with different chemical characteristics in its side chain. Amino acids can be divided into groups such as basic amino acids (e. g. , lysine, arginine, histidine) ; acidic amino acids (e. g. , aspartic acid, glutamic acid); uncharged polar amino acids (e. g., glycine, asparagine, glutamine, serine, threonine, tyrosine, cysteine); nonpolar amino acids (e. g., alanine, valine, leucine, isoleucine, proline, phenylalanine, methionine, tryptophane); β-branched amino acids (e. g. , threonine, valine, isoleucine) ; and aromatic amino acids (e. g. , tyrosine, phenylalanine, tryptophane, histidine). One amino acid residue, belonging to a group of amino acids, may be substituted for by another amino acid, which belongs to a different group. Specific examples include but are not limited to: substitution of a basic amino acid with an acidic or neutral amino acid; substitution of a polar amino acid with a nonpolar amino acid; substitution of an amino acid of molecular weight greater than the average molecular weights of 20 naturally-occurring amino acids, with an amino acid of molecular weight less than this average; and conversely, substitution of an amino acid of molecular weight less than this average, with an amino acid of molecular weight greater than this average. For example, a mutant selected from the group consisting of G69E, T116A and A183S in the Sendai virus M protein, or comprising mutations homologous thereto, can be suitably used. Herein, G69E refers to a mutation wherein the 69th M protein amino acid glycine is substituted by glutamic acid, T116A refers to a mutation wherein the 116th M protein amino acid threonine is substituted by alanine, and A183S refers to a mutation wherein the 183rd M protein amino acid alanine is substituted by serine. In other words, G69, T116 and A183 in the Sendai virus M protein or homologous M protein sites in other viruses, can be substituted by glutamic acid (E), alanine (A) and serine (S) respectively. These mutations are preferably comprised in combination, and it is particularly preferable to comprise all three of the above-mentioned mutations. M gene mutagenesis can be carried out according to a known mutagenizing method. For example, as described in the Examples, a mutation can be introduced by using an oligonucleotide containing a desired mutation.

In the case of measles virus for example, it is possible to introduce the M gene sequence of temperature-sensitive strain P253-505, in which the epitope sequence of an anti-M protein monoclonal antibody has been altered (Morikawa, Y. *et al*., Kitasato Arch. Exp. Med., 64; 15-30 (1991)). In addition, the threonin at residue 104 of the measles virus M protein, or the threonin at residue 119 of the mumps virus M protein, which correspond to the threonin at residue 116 of the SeV M protein, may be substituted with any other amino acid (for example, alanine).

Another particularly preferable embodiment of the above method of the present invention comprises the step of reconstituting the (-)strand RNA virus vector containing the deficient M gene, under a condition where the M gene, integrated in the chromosome of the cells used for the reconstitution, is expressed. The term "M gene deficiency" refers to the deletion of a sequence encoding the functional M protein, including cases where an M gene comprising a functionally deficient mutation is present, and cases where the M gene is absent. A functionally deficient M gene mutation can be produced, for example, by deleting the M gene protein-encoding sequence, or by inserting another sequence. For example, a termination codon can be inserted partway through the M protein-encoding sequence (WO00/09700). Most preferably, the M gene-deficient vector is completely devoid of M protein-encoding sequence. Unlike a vector encoding a temperature-sensitive mutant M protein, a vector without an M protein open reading frame (ORF) cannot produce viral particles under any conditions. In the present invention, it was found that recombinant viruses could be efficiently produced by integrating the M gene into the chromosomes of host cells used for vector reconstitution, and then persistently expressing and supplying M protein. Cells in which the M gene has been chromosomally integrated can be prepared, for example, by a method described in the Examples. The M gene may be constantly expressed, or inducibly expressed on viral reconstitution. Surprisingly, it was revealed that low-temperature reconstitution markedly improved vector production efficiency, even in the presence of wild-type M protein. Thus, vector reconstitution is preferably performed at a low temperature, specifically, at 35°C or a less, more preferably at 34°C or less, further preferably at 33°C or less, and most preferably at 32°C or less.

It is preferable for the (-)strand RNA virus vector to further comprise the deficient HN and/or F genes, or temperature-sensitive mutant HN and/or F genes, in a method for producing the recombinant (-)strand RNA virus vector in which particle formation ability has been reduced or eliminated, where that method comprises the step of reconstituting a (-)strand RNA virus vector that reduces or eliminates M protein localization by M gene deficiency or mutation under a condition where the functional M protein is persistently expressed. At non-permissive temperatures, particle formation ability was revealed to be extremely reduced, particularly in the case of a vector containing a temperature-sensitive HN gene mutation in addition to an M gene deficiency or mutation. The temperature-sensitive HN gene mutation is not particularly limited, but for example comprises at least one of the amino acid sites selected from the group consisting of A262, G264, and K461 in the Sendai virus HN protein, preferably an arbitrary two of these sites, and more preferably all three sites. Other (-)strand RNA virus HN proteins comprising homologous mutations can also be suitably used, but are also not limited thereto. Herein, A262 means the 262nd HN protein amino acid alanine, G264 is the 264th HN protein amino acid glycine, and K461 is the 461st HN protein amino acid lysine. The amino acid mutation can be a substitution with any desired amino acid. However, it is preferably a substitution with an amino acid with different chemical side chain characteristics, as previously described in the case of M protein mutation. For example, it includes substitution by an amino acid belonging to a different group, as previously described. Specifically, mutants selected from the group consisting of A262T, G264R, and K461G in the Sendai virus HN protein, or comprising mutations homologous to the above-mentioned mutants, can be adequately used. Herein, A262T refers to a mutation wherein the 262nd HN protein amino acid alanine is substituted by threonine; G264R refers to a mutation in which the 264th HN protein amino acid glycine is substituted by arginine; and K461G refers to a mutation in which the 461st HN protein amino acid lysine is substituted by glycine. In other words, A262, G264, and K461 in the Sendai virus HN protein, or homologous sites in the HN protein of other viruses, can be substituted by threonine (T), arginine (R), and glycine (G), respectively. It is preferable to comprise a combination of these mutations, and is more preferable to comprise all three mutations.

An additional example refers to the Urabe AM9 strain of the mumps virus, which demonstrates temperature sensitivity and is used as a vaccine (Wright, K. E. *et al*., Virus Res. , 67; 49-57 (2000)). In the present invention, and with respect to this virus, mutations are preferably introduced at the 464th and 468th amino acids. Amino acid mutations at sites homologous to these can be applied to other (-)strand RNA viruses.

A deficient F gene is particularly preferable in a (-)strand RNA virus vector comprising a temperature-sensitive M gene. In addition, it is more preferable for the F gene-deficient vector comprising the temperature-sensitive M gene to further comprise an HN gene with a temperature-sensitive mutation. In the present invention, it was demonstrated that a high-titer F gene-deficient (-)strand RNA virus vector comprising temperature-sensitive M and HN genes'could be produced by reconstituting the viral vector at a permissive temperature using an F protein-expressing helper cell. The vector was also shown to produce significantly fewer VLPs. In the present invention, "deficiency" of a gene means that the functional gene product (a protein in the case of a protein-encoding gene) is not substantially expressed. Gene deficiency includes a null phenotype for a subject gene. Gene deficiency also includes cases in which the gene has been deleted, where the gene is not transcribed due to mutations in the transcription initiation sequence or the like, where the functional protein is not produced due to frameshift or codon mutations or the like, where the activity of the expressed protein has been substantially eliminated (or extremely reduced (for example, to 1/10 or less)) due to amino acid mutations or the like, where protein translation is eliminated or extremely reduced (for example, to 1/10 or less), etc.

This invention also refers to recombinant viruses comprising mutations that stimulate sustained infection in the P gene or L gene of (-)strand RNA viruses. These mutations more specifically include mutation of the 86th SeV P amino acid glutamine (E86), substitution to another amino acid of the 511th SeV P amino acid leucine (L511), and substitution of homologous sites of other (-)strand RNA virus P proteins. The amino acid mutation may be a substitution to any other desirable amino acid, however is preferably a substitution to an amino acid with different chemical side chain characteristics, as described above. For example, the substitution includes substitution by an amino acid belonging to a different group, as previously described. More specific examples include the substitution of E86 by lysine (E86K), and the substitution of L511 by phenylalanine (L511F). In the case of L protein, substitution(s) of the 1197th amino acid asparagine (N1197) and/or the 1795th amino acid lysine (K1795) by another amino acid, or substitutions at homologous sites of other (-)strand RNA virus L proteins, can also be included. An L protein gene comprising both of these two mutations in the L protein is especially preferable. The amino acid mutation can also be a substitution by any selected amino acid, however it is preferably a substitution by an amino acid with different chemical side chain characteristics, as previously described. For example, a substitution by an amino acid belonging to a different group can be included. More specific examples include the substitution of N1197 by serine (N1197S), and a substitution of K1795 by glutamic acid (K1795E). The presence of both P and L gene mutations remarkably increases sustained infectivity, suppression of secondary particle release, and cytotoxicity suppression. These effects can be dramatically increased by combining the above-mentioned temperature-sensitive mutant gene (s) in HN protein and/or M protein. A recombinant (-)strand RNA virus comprising at least one temperature-sensitive mutation in the M and/or HN genes, and at least one sustained-infectivity type mutation in the P and/or L genes, is preferable. In particular, a virus with mutations in all four genes (M, HN, P, and L) is further preferable. A virus comprising the three above-mentioned amino acid mutations in the M and HN genes (in SeV: G69, T116 and A183 in M; and A262, G264 and K461 in HN) , and at least one of the two above-mentioned P gene mutations (E86 or L511 in SeV) , and the two above-mentioned L gene mutations (N1197 and K1795 in SeV) , is most preferable. These recombinant viruses comprising mutations in the P and/or L gene are preferable when a (-)strand RNA virus spike protein gene (e.g. the F gene) is lost. By producing a virus strain using a deficient F-gene and an F-helper cell which expresses F protein, it is possible to obtain a viral vector that does not multiply after infecting target cells, and in which cytotoxicity is reduced and secondary particle production dramatically suppressed. Such a vector can express introduced gene (s) for longer periods than vectors with wild-type P or L genes. These viruses of the present invention are useful in gene transfer for reducing cytotoxicity; suppressing reduced expression of introduced genes, and suppressing the release of virus and virus-like particles (VLPs) from virus-infected cells.

This invention, as described in the specification, provides a method that decreases the degree of cytotoxicity in gene transfer. This method comprises the step of introducing a virus comprising mutations or deficiencies in viral genes (e. g. the M, HN, P, or L gene, or combinations thereof) into cells. This invention also provides a method for suppressing the release of VLPs from virus-introduced cells in gene transfer, where this method comprises the step of introducing such a virus into cells. The degree of cytotoxicity can be determined, for example, by measuring the release of LDH as described in the Examples. The level of expression of the introduced gene can be determined by Northern hybridization or RT-PCR using a fragment of the introduced gene as a probe or primer; by immunoprecipitation or Western blot analysis using an antibody against the product of the introduced gene; or by measuring the activity of the product of the introduced gene. VLP release can be measured, for example, by determining HA activity as shown in the Examples. Alternatively, VLPs in the supernatant solution can be quantified by recovering the supernatant solution, and measuring the expression of genes including VLPs after transfection into cells. It is preferable that reductions in cytotoxicity, introduced gene expression, and VLP release are, for example, statistically significant reductions (or suppressions) when compared to a virus without a corresponding mutation or deficiency (for example, statistical level of 5% or less). Statistical analysis can be carried out, for example, using the Student t-test or the Mann-Whitney *U*-test. Reduction or suppression should preferably be to 90% or less of the reference virus, more preferably to 80% or less, even more preferably to 70% or less, or 60% or less. Reduction or suppression is more preferably to 1/2 or less, more preferably to 1/3 or less, 1/5 or less, or 1/8 or less of the reference level.

This invention can be used in gene transfer to reduce cytotoxicity, to suppress reduced expression of introduced genes, and to suppress VLP release from cells introduced with viruses comprising mutations or deficiencies in viral genes, as described in this specification. These viruses are viruses used in gene transfer for reducing cytotoxicity, for suppressing reduced expression of introduced genes, or for suppressing VLP release from virus-introduced cells.

In the present invention, "(-)strand RNA virus" refers to an RNA virus comprising a negative strand as its genome. In the present invention, a (-)strand RNA virus preferably refers to a non-segmented (-)strand RNA virus, i.e. a single-strand RNA virus comprising a negative strand as its genome. The (-)strand RNA virus is exemplified by, for example, viruses belonging to the Paramyxoviridae family, such as the Sendai virus, Newcastle disease virus, mumps virus, measles virus, RS virus (Respiratory syncytial virus), rinderpest virus and distemper virus; viruses belonging to the Orthomyxoviridae family, such as the influenza virus; viruses belonging to the Rhabdoviridae family, such as vesicular stomatitis virus and rabies virus; etc. Herein, the (-)strand RNA virus vector is preferably a non-segmented type (-)strand RNA virus, and more preferably, a paramyxovirus.

In the present invention, preferable (-)strand RNA viruses include the Sendai virus (SeV) , human parainfluenza virus-1 (HPIV-1) , human parainfluenza virus-3 (HPIV-3) , phocine distemper virus (PDV) , canine distemper virus (CDV), dolphin molbillivirus (DMV), peste-des-petits-ruminants virus (PDPR), measles virus (MV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b) , mumps virus (Mumps) , and Newcastle disease virus (NDV). More preferably, examples include viruses selected from the group consisting of Sendai virus (SeV) , human parainfluenza virus-1 (HPIV-1), human parainfluenza virus-3 (HPIV-3), phocine distemper virus (PDV), canine distemper virus (CDV), dolphin molbillivirus (DMV) , peste-des-petits-ruminants virus (PDPR) , measles virus (MV), rinderpest virus (RPV), Hendra virus (Hendra), and Nipah virus (Nipah).

The virus genes encoded by these viruses have been known. Accession numbers are exemplified below: AF014953 (CDV) , X75961 (DMV), D01070 (HPIV-1), M55320 (HPIV-2), D10025 (HPIV-3), X85128 (Mapuera) , D86172 (Mumps), K01711 (MV), AF064091 (NDV), X74443 (PDPR), X75717 (PDV), X68311 (RPV) , X00087 (SeV), M81442 (SV5), and AF079780 (Tupaia) for N gene; X51869 (CDV), Z47758 (DMV), M74081 (HPIV-1), X04721 (HPIV-3) , M55975 (HPIV-4a), M55976 (HPIV-4b), D86173 (Mumps), M89920 (MV) , M20302 (NDV), X75960 (PDV) , X68311 (RPV), M30202 (SeV) , AF052755 (SV5), and AF079780 (Tupaia) for P gene; AF014953 (CDV), Z47758 (DMV) , M74081 (HPIV-1), D00047 (HPIV-3), AB016162 (MV), X68311 (RPV), AB005796 (SeV), and AF079780 (Tupaia) for C gene; M12669 (CDV), Z30087 (DMV), S38067 (HPIV-1), M62734 (HPIV-2), D00130 (HPIV-3), D10241 (HPIV-4a), D10242 (HPIV-4b) , D86171 (Mumps) , AB012948 (MV), AF089819 (NDV) , Z47977 (PDPR), X75717 (PDV) , M34018 (RPV), U31956 (SeV), and M32248 (SV5) for M gene; M21849 (CDV) , AJ224704 (DMV), M22347 (HPN-1) , M60182 (HPIV-2) , X05303 (HPIV-3), D49821 (HPIV-4a), D49822 (HPIV-4b) , D86169 (Mumps), AB003178 (MV), AF048763 (NDV), Z37017 (PDPR), AJ224706 (PDV), M21514 (RPV), D17334 (SeV), and AB021962 (SV5) for F gene; AF112189 (CDV), AJ224705 (DMV), U709498 (HPIV-1), D000865 (HPIV-2), AB012132 (HPIV-3), M34033 (HPIV-4A), AB006954 (HPIV-4B), X99040 (Mumps), K01711 (MV), AF204872 (NDV) , Z81358 (PDPR), Z36979 (PDV), AF132934 (RPV), U06433 (SeV), and S76876 (SV-5) for HN (H or G) gene. More than one strain is known for viral species, and genes comprising sequences other than those shown above may exist, depending on different strains.

In the present invention, "paramyxovirus" refers to viruses that belong to the family Paramyxoviridae, and to viruses derived from them. Paramyxovirus is a virus group with a non-segmented negative strand RNA genome. Paramyxoviruses in the present invention include the subfamily Paramyxovirinae (comprising the genus *Respirovirus* (also called the genus *Paramyxovirus*), the genus *Rubulavirus* and the genus *Morbillivirus*), and the subfamily Pneumovirinae (comprising the genus *Pneumovirus* and the genus Metapneumovirus). Paramyxoviruses to which the present invention can be applied include, for instance, viruses belonging to the Paramyxoviridae, such as the Sendai virus, Newcastle disease virus, mumps virus, measles virus, RS virus (Respiratory syncytial virus) , rinderpest virus, distemper virus, simian parainfluenza virus (SV5), and human parainfluenza virus type 1, 2, and 3, etc. The viruses of the present invention are preferably viruses belonging to the subfamily Paramyxovirinae (comprising the genus *Respirovirus*, the genus *Rubulavirus* and the genus *Morbillivirus*), and more preferably viruses belonging to the genus *Respirovirus* (also called the genus *Paramyxovirus*) or derivatives thereof. Examples of viruses of the genus *Respirovirus* to which the present invention can be applied include human parainfluenza virus type 1 (HPIV-1), human parainfluenza virus type 3 (HPIV-3) , bovine parainfluenza virus type 3 (BPIV-3) , Sendai virus (also called murine parainfluenza virus type 1), simian parainfluenza virus type 10 (SPIV-10), etc. The paramyxovirus of the present invention is most preferably the Sendai virus. These viruses may be derived from natural strains, wild-type strains, mutant strains, laboratory-passaged strains, artificially constructed strains, etc. Incomplete viruses such as the DI particle (J. Virol. 68, 8413-8417 (1994)), synthesized oligonucleotides, and so on, can also be utilized as material for producing the viral vector of the present invention. The (-)strand RNA viruses are suitable as vectors for gene transfer. Their transcription and replication only take place in the cytoplasm of host cells. They have no DNA phase, and thus chromosomal integration does not occur. Therefore, safety problems which depend on chromosomal aberration, such as canceration and immortalization, do not exist. These characteristics greatly contribute to the safety of (-)strand RNA viruses as vectors. The results of foreign gene expression suggest that nucleotide mutation does not take place, even during continuous multi-generational culture of SeV, indicating a highly stable genome, and stable expression of the inserted foreign gene over long periods (Yu, D. *et al*., Genes Cells 2: 457-466 (1997)). As (-)strand RNA viruses do not have capsid protein structure, they also possess advantages in packaging and size flexibility in the introduced gene. The SeV vector can be used to introduce foreign gene (s) of 4 kb or larger. By adding a transcription unit, two or more types of gene can be simultaneously expressed.

In rodents, the Sendai virus is known to be pathogenic and to cause pneumonia, but in humans it is non-pathogenic. This is supported by previous reports that the intranasal application of wild-type Sendai virus did not cause severe symptoms in non-human primates (Hurwitz, J. L. *et al*., Vaccine 15: 533-540 (1997)). Further remarkable advantages are its "high infectivity" and "high expression level". SeV vectors infect by binding to sialic acid in the sugar-chain of cell membrane proteins. As sialic acid is expressed in almost all cells, a wider infection spectrum, i.e. high infectivity, is expected. Replicative vectors based on the SeV replicon can induce the re-infection of surrounding cells by released viral particles. Distribution to daughter cells, in line with cell division, is expected to result in sustained expression of RNP produced in multiple copies in the cytoplasm of infected cells. SeV vectors can be applied to a very wide range of different tissues. This broad range of infectivity indicates that these vectors can be applied in various types of antibody therapy (and analysis). Due to their characteristic expression mechanism, whereby transcription and replication only occur in the cytoplasm, expression of the introduced gene is shown to be very high (Moriya, C. *et al*., FEBS Lett. 425(1): 105-111 (1998); WO00/70070).

In the present invention, "vector" refers to a carrier that transfers nucleic acids into cells. In the present invention, "(-)strand RNA virus vector" refers to a vector (carrier) that is derived from a (-)strand RNA virus, and transfers nucleic acids into cells. In the present invention, the (-)strand RNA virus vector may be an infective virus particle. Virus particle refers to a nucleic-acid-containing minute particle that is released from a cell by the action of viral proteins. Virus particles can take various forms, e.g. that of spheres or rods, depending on the viral species. They are significantly smaller than cells, generally in the range of about 10 nm to about 800 nm. Paramyxovirus viral particles are structured such that the above-mentioned RNP comprises the genomic RNA and viral proteins, and is enclosed by a lipid membrane (or envelope) derived from the cell membrane. In the case of viruses whose genome includes genes that encode mutant viral proteins comprising amino acid substitution(s) (e.g. mutant M, HM, P, or L protein as described in the Examples), the viral vector can be a complex consisting of the genomic RNA of a (-)strand RNA virus, and the viral proteins, i.e. ribonucleoprotein (RNP). RNP can be introduced into target cells, for example, in combination with a desired transfection reagent. Such RNP is, more specifically, a complex comprising the genomic RNA of a (-)strand RNA virus, N protein, P protein, and L protein. When RNP is introduced into cells, the viral proteins work to transcribe cistrons encoding viral proteins from the genomic RNA, so that the genome itself is replicated, and daughter RNPs are produced. Replication of the genomic RNA can be confirmed by detecting the increase in RNA copy number using RT-PCR, Northern hybridization, or such. Herein, "infectiveness" refers to the ability to introduce a gene from a vector into adhered cells, by maintaining the ability of a recombinant (-)strand RNA virus vector to adhere to cells. In a desirable embodiment, a (-)strand RNA virus vector is maintained such that it can express a foreign gene. A (-)strand RNA virus vector of the present invention does not comprise the replicative ability of a wild-type virus, because cell surface M protein aggregation has been reduced or eliminated, and particle formation suppressed. In the case of host cell infection by a viral vector, "replication ability" refers to the virus' ability to replicate in host cells, and to produce infective particles. Examples of host cells include LLC-MK2 and CV-1.

A (-)strand RNA virus vector contains the genomic RNA of a (-)strand RNA virus. "Genomic RNA" refers to RNA that comprises the ability to form (-)strand RNA viral proteins along with RNP, to use these proteins to express genes from the genome, and to then replicate these nucleic acids and form daughter RNPs. A (-)strand RNA virus comprises a negative strand RNA as its genome, and this kind of RNA encoded carried genes in the antisense mode. In general, (-)strand RNA viral genomes comprise viral genes in antisense series between the 3'-leader region and the 5'-trailer region. Between the open reading frames for each gene, a series of sequences is present: a transcription termination sequence (E sequence), intervening sequence (I sequence), and transcription initiation sequence (S sequence). Thus the RNA encoding each gene's open reading frame is transcribed as a separate cistron. Genomic RNAs included in the viruses of this invention encode (in antisense mode) nucleocapsid (N), phosphor (P) and large (L) proteins, and mutant proteins thereof. These proteins are necessary for the expression of genes encoded by the RNAs, and for autonomous replication of the RNA themselves. In a preferable embodiment, the RNA does not encode matrix protein (M), which is needed for the formation of viral particles, nor does it encode mutant M protein. The RNA may or may not encode spike proteins, which are needed for viral particle infection. In a preferable embodiment, the RNA comprises a mutation in at least one spike protein. Alternatively, the RNA does not encode at least one spike protein. Paramyxovirus spike proteins of include fusion protein (F protein) , which induces fusion of cell membranes; and hemagglutinin-neuramidase protein (HN protein) , which is needed for adhesion between proteins and cells.

"Recombinant" refers to a compound or composition produced via a recombinant polynucleotide. "Recombinant polynucleotide" refers to a polynucleotide that is not bound to its natural state. Specifically, recombinant polynucleotides include artificially rearranged polynucleotide chains, or artificially synthesized polynucleotides. Herein, a "recombinant" (-)strand RNA virus vector refers to a (-)strand RNA virus vector constructed by genetic engineering, or a (-)strand RNA virus vector obtained by amplifying this vector. Recombinant (-)strand RNA virus vectors can be generated, for example, by reconstituting recombinant (-)strand RNA virus cDNAs.

Genes encoding paramyxovirus viral proteins include, for example, the NP, P, M, F, HN and L genes. "NP, P, M, F, HN and L genes" refer to genes encoding nucleocapsid, phospho, matrix, fusion, hemagglutinin-neuraminidase and large proteins, respectively. Genes of viruses belonging to the subfamily Paramyxovirinae are represented in general as below: The NP gene is also generally described as the "N gene".

| | | | | | | | |
|---|---|---|---|---|---|---|---|
| Genus Respirovirus | NP | P/C/V | M | F | HN | - | L |
| Genus Rubulavirus | NP | P/V | M | F | HN | (SH) | L |
| Genus Morbillivirus | NP | P/C/V | M | F | H | - | L |

For example, database accession numbers for nucleotide sequences of Sendai virus genes classified into Respiroviruses of the family Paramyxoviridae are: M29343, M30202, M30203, M30204, M51331, M55565, M69046 and X17218 for the NP gene; M30202, M30203, M30204, M55565, M69046, X00583, X17007 and X17008 for the P gene; D11446, K02742, M30202, M30203, M30204, M69046, U31956, X00584 and X53056 for the M gene; D00152, D11446, D17334, D17335, M30202, M30203, M30204, M69046, X00152 and X02131 for the F gene; D26475, M12397, M30202, M30203, M30204, M69046, X00586, X02808 and X56131 for the HN gene; andD00053, M30202, M30203, M30204, M69040, X00587 and X58886 for the L gene.

The term "gene" used herein means a genetic substance, which includes nucleic acids such as RNA, DNA, etc. In general, a gene may or may not encode a protein. In the present invention, a nucleic acid encoding a protein is called a protein gene. For example, a gene may encode a functional RNA such as a ribozyme, antisense RNA, etc. A gene may have a naturally derived or artificially designed sequence. Herein, "DNA" includes single-stranded DNA and double-stranded DNA. The term "encoding a protein" refers to comprising an antisense or sense ORF, which encodes a protein amino acid sequence, such that a polynucleotide can be expressed under suitable conditions.

In the present invention, there is no particular limitation as to the (-)strand RNA virus vector to be tested or produced. A preferred (-)strand RNA virus vector includes, for example, a vector exhibiting replicative capability, and which can self-replicate under conditions that ensure persistent complementation of the reduction or elimination of cell-surface M protein aggregation. For example, the genome of natural paramyxoviruses generally contains a short leader region at the 3' end, followed by six genes encoding the N, P, M, F, HN and L proteins, and a short 5'-trailer region at the other end. A paramyxovirus vector capable of self-replication can be produced by designing a genome comprising a structure similar to this. For example, a viral genome by which M protein localization is reduced or eliminated due to a mutation or deficiency in any one of these genes, or a viral genome comprising other genes by which M protein localization is reduced or eliminated, is constructed. Virus reconstitution is then carried out by transcribing the genome under a condition ensuring complementation of the deficiency. A vector expressing a foreign gene can be prepared by inserting the foreign gene into the genome. In a (-)strand RNA virus vector of the present invention, the arrangement of the viral genes may be modified to differ from that of the wild-type virus.

In the present invention's viral vector in which particle formation ability has been reduced or eliminated, viral genes may be further deleted or mutagenized. For example, a viral vector comprising an M gene containing a temperature-sensitive mutation, or a viral vector with reduced particle formation ability due to M gene deletion, may contain mutations or deletions in the HN, F, and/or other viral genes. For example, and as described in the Examples, the present invention facilitates production of a vector comprising a temperature-sensitive M gene, and further comprising an HN, F and/or other gene carrying a temperature-sensitive mutation, or comprising a deletion of an F, HN and/or other gene. In addition, a vector comprising an M-gene deletion, and in which an F and/or HN gene has been deleted or carries mutations such as temperature-sensitive mutations, can be produced. When these vectors comprise a temperature-sensitive mutation, they are reconstituted at a permissive temperature. When a gene is deleted or deficient, reconstitution is carried out by supplying, in a trans-acting fashion, gene products comprising normal function. For example, genes encoding these gene products are chromosomally integrated into host cells, an expression vector coding the vector genome is introduced and expressed, the gene products are thus supplied to the host cells, and vector reconstitution can be carried out. The amino acid sequences of such gene products are not restricted to the virus-derived sequences themselves. If the nucleic acid-introducing activity of the viral vector to be produced is equivalent to or greater than that of the natural type, a mutation may be introduced into the sequence, or a homologous gene derived from another virus may be used instead of that sequence.

Furthermore, it is possible to prepare a vector that contains, as an envelope, a protein different from the envelope protein of the virus from which the vector genome is derived. For example, when reconstituting a viral vector, a virus comprising a desired envelope protein can be produced by expressing in cells an envelope protein different to that encoded by the viral genome on which the vector is based. Such a protein is not particularly restricted, and for example includes envelope proteins from other viruses, e.g. , G protein (VSV-G) from vesicular stomatitis virus (VSV). The (-)strand RNA virus vector of the present invention includes pseudo-type viral vectors comprising envelope proteins such as VSV-G protein, derived from viruses other than the virus from which the genome is derived.

For example, (-)strand RNA virus vectors of the present invention include those comprising, on the surface of their envelope, proteins such as adhesion factors, ligands, and receptors, capable of adhering to specific cells. These proteins can also include, for example, chimeric proteins that comprise such proteins in extracellular regions, or that comprise viral envelope-derived polypeptides in intracellular regions. In this way, vectors targeting specific tissues can be created. These proteins may be encoded in the viral genome, or supplied on viral vector reconstitution by the expression of genes other than those in the genome (e.g. other expression vectors or host chromosome genes).

Viral genes comprised by the (-)strand RNA virus vector of this invention may be modified from wild-type genes in order to, for example, reduce immunogenicity by vector-derived viral proteins, or to enhance RNA transcription and replication efficiency. Specifically, in paramyxoviral vectors for example, transcription or replication functions can be enhanced by modifying at least one of the replication factors: NP gene, P/C gene, and L gene. The structural protein HN comprises both hemagglutinin and neuraminidase activities. If, for example, the activity of the former can be reduced, the stability of the virus in blood can be enhanced. If, for example, the activity of the latter can be modified, infectivity can be regulated. Membrane fusion ability can be regulated by modifying the F protein, which is involved in membrane fusion. For example, by using analysis of the antigen-presenting epitopes and such of possible cell surface antigenic molecules, such as the F and HN proteins, a viral vector with weakened antigen-presenting ability against these proteins can be created.

A viral vector with a deficient accessory gene can be used as the (-)strand RNA virus vector of the present invention. For example, by knocking out the V gene, an SeV accessory gene, SeV pathogenicity to hosts such as mice is markedly decreased without damaging gene expression and replication in cultured cells (Kato, A. *et al*., 1997, J. Virol. 71: 7266-7272; Kato, A. *et al*., 1997, EMBO J. 16:578-587; Curran, J. *et al*., WO01/04272, EP1067179). This kind of attenuated vector is particularly preferred as a viral vector for *in vivo* or *ex vivo* gene transfer.

The genomic RNA of a viral vector of the present invention may include RNA encoding a foreign gene. Recombinant viral vectors comprising foreign genes can be obtained by inserting these foreign genes into the viral vector genome. Any gene whose expression is desired in target cells may be used as the foreign gene. The foreign gene may be a gene encoding a natural protein, or a gene modified by deletion, substitution, or insertion, as long as it encodes a protein functionally equivalent to the natural protein. Alternatively, it may encode a deletion-type natural protein, or an artificial protein, or the like. For example, if gene therapy is intended, a gene for treating the target disease is inserted into the viral vector DNA. If a foreign gene is inserted into the viral vector DNA, for example, into the Sendai virus vector DNA, it is preferable to insert a sequence comprising a nucleotide number of multiple six between the transcription termination sequence (E) and transcription initiation sequence (S), etc. (Journal of Virology, Vol. 67, No. 8, 1993, p.4822-4830). Foreign genes may be inserted before and/or after each viral gene (e.g. the NP, P, M, F, HN and L genes). The E-I-S sequence (transcription termination sequence-intervening sequence-transcription initiation sequence) or portion thereof is appropriately inserted before or after a foreign gene, and an E-I-S sequence unit is located between each gene so as to avoid interference with the expression of genes before and/or after the foreign gene. Alternatively, foreign genes can be inserted using IRES.

Expression level of the inserted gene can be regulated by the type of transcription initiation sequence added upstream of the gene (WO01/18223), as well as by the site of gene insertion and the nucleotide sequences before and after the gene. In the example of the Sendai virus, the closer the insertion site is to the 3'-end of the negative-strand RNA of the viral genome (in the gene arrangement of the wild type viral genome, the closer to the NP gene) , the greater the expression of the inserted gene. To achieve high level expression of an inserted gene, the gene is preferably inserted into an upstream region (the 3'-side in the minus-strand), such as upstream of the NP gene (the 3'-side in the negative-strand) or between the NP and P genes. Conversely, the closer insertion is to the 5'-end of the negative-strand RNA (in the gene arrangement of the wild type viral genome, the closer to the L gene), the greater the reduction in expression of the inserted gene. To suppress foreign gene expression to a low level, the foreign gene is inserted, for example, to the far most 5'-side of the negative-strand, that is, downstream of the L gene in the wild type viral genome (the 5'-side adjacent to the L gene in the negative-strand) or upstream of the L gene (the 3'-side adjacent to the L gene in the negative-strand). Thus, the insertion position of a foreign gene can be properly adjusted so as to obtain a desired level of gene expression, or so as to optimize the combination of the foreign gene and the viral protein-encoding genes before and after it. Take, for example, the case where toxicity may be caused by overexpression of a gene introduced by inoculation using a high titer viral vector. In such a case, an appropriate therapeutic effect can be achieved not only by limiting the titer of the virus to be inoculated, but also by, for example, reducing the expression level of individual viral vectors, by inserting the gene into the vector at a position as close as possible to the 5'-terminus of the negative-strand genome, or by replacing the transcription initiation sequence with a less efficient sequence.

High expression of a foreign gene is generally advantageous, so long as cytotoxicity does not occur. Thus it is preferable to ligate the foreign gene with a highly efficient transcription initiation sequence, and to insert the gene near the 3'-terminus of the negative-strand genome. Examples of preferable vectors include vectors where the foreign gene is located on the 3'-side of any of the viral protein genes in the negative-strand genome of the (-)strand RNA virus vector. For example, a vector in which the foreign gene is inserted upstream (at the 3'-side of the N gene-encoding sequence in the negative-strand) of the N gene is preferable. Alternatively, the foreign gene may be inserted immediately downstream of the N gene. A foreign gene may also be inserted into the genomic region from which a viral gene, such as the M and/or F genes, has been deleted.

To simplify foreign gene insertion, a cloning site may be designed at the insertion point of the genome-encoding vector DNA. For example, the cloning site can be a restriction enzyme recognition sequence. A cloning site may also be a so-called multi-cloning site, comprising multiple restriction enzyme recognition sequences. The (-)strand RNA viral vectors of this invention may also retain other foreign genes at insertion sites other than these.

In order to produce a (-)strand RNA virus vector, cDNA encoding the (-)strand RNA virus' genomic RNA is transcribed in mammalian cells, in the presence of viral proteins necessary for the reconstitution of RNP which comprises the (-)strand RNA virus' genomic RNA, i.e., in the presence of N, P, and L proteins. The viral RNP may be reconstituted by forming a negative strand genome (i.e., the antisense strand that is the same as the viral genome) or a positive strand (the sense strand encoding the viral proteins). For improved reconstitution efficiency, formation of the positive strand is preferable. The 3'-leader and 5'-trailer sequence at the RNA ends is preferably reflects the natural viral genome as accurately as possible. To accurately control the 5'-end of the transcription product, a T7 RNA polymerase recognition sequence may be used as a transcription initiation site to express the RNA polymerase in cells. The 3'-end of the transcription product can be controlled, for example, by encoding a self-cleaving ribozyme onto this 3'-end, ensuring it is accurately cut (Hasan, M. K. *et al*., J. Gen. Virol. 78: 2813-2820 (1997); Kato, A. *et al*., EMBO J. 16: 578-587 (1997); and Yu, D. *et al*., Genes Cells 2: 457-466 (1997)).

Recombinant Sendai virus vectors comprising a foreign gene can be constructed according to, for example, the descriptions in "Hasan, M. K. *et al*., J. Gen. Virol. 78: 2813-2820, 1997", "Kato, A. *et al*., 1997, EMBO J. 16: 578-587" and "Yu, D. *et al*., 1997, Genes Cells 2: 457-466". This method is outlined below:

To introduce a foreign gene, a DNA sample comprising the cDNA nucleotide sequence of the desired foreign gene is first prepared. The DNA sample is preferably identified as a single plasmid electrophoretically at a concentration of 25 ng/µl or more. The following example describes the use of the NotI site in the insertion of a foreign gene into DNA encoding viral genomic RNA: If the target cDNA nucleotide sequence comprises a NotI recognition site, this site should be removed beforehand using a technique such as site-specific mutagenesis to change the nucleotide sequence, without changing the amino acid sequence it codes. The desired gene fragment is amplified and recovered from this DNA sample using PCR. By attaching NotI sites to the 5'-regions of the two primers, both ends of the amplified fragment become NotI sites. The E-I-S sequence (or its part, depending on the insertion site) is arranged such that it can be included in the primer, so that E-I-S sequence units can be placed between the ORFs either side of the viral genes, and the ORF of the foreign gene (after it has been incorporated into the viral genome).

For example, to assure cleavage by NotI, the forward side synthetic DNA sequence is arranged as follows: Two or more nucleotides (preferably four nucleotides excluding sequences such as GCG and GCC that are derived from the NotI recognition site; more preferably ACTT) are randomly selected on its 5'-side, and a NotI recognition site "gcggccgc" is added to its 3'-side. In addition, a spacer sequence (nine random nucleotides, or nucleotides of nine plus a multiple of six) and an ORF (a sequence equivalent to about 25 nucleotides and comprising the initiation codon ATG of the desired cDNA) are also added to the 3'-side. About 25 nucleotides are preferably selected from the desired cDNA, such that G or C are the final nucleotides on the 3'-end of the forward side synthetic oligo DNA.

The reverse side synthetic DNA sequence is arranged as follows: Two or more random nucleotides (preferably four nucleotides excluding sequences such as GCG and GCC that originate in the NotI recognition site; more preferably ACTT) are selected from the 5'-side, a NotI recognition site "gcggccgc" is added to the 3'-side, and an oligo DNA insertion fragment is further added to the 3'-side in order to regulate length. The length of this oligo DNA is designed such that the number of nucleotides in the final PCR-amplified NotI fragment product, which comprises the E-I-S sequence, becomes a multiple of six (the so-called "rule of six"; Kolakofski, D. *et al*., J. Virol. 72: 891-899, 1998; Calain, P. and Roux, L., J. Virol. 67: 4822-4830, 1993; Calain, P. and Roux, L., J. Virol. 67: 4822-4830, 1993). A sequence complementary to the Sendai virus S sequence, preferably 5'-CTTTCACCCT-3' (SEQ ID NO: 1), a sequence complementary to the I sequence, preferably 5'-AAG-3', and a sequence complementary to the E sequence, preferably 5'-TTTTTCTTACTACGG-3' (SEQ ID NO: 2), are further added to the 3'-side of the inserted fragment. The 3'-end of the reverse side synthetic oligo DNA is formed by the addition of a complementary sequence, equivalent to about 25 nucleotides counted in reverse from the termination codon of the desired cDNA, and whose length is selected such that G or C becomes the final nucleotide.

PCR can be carried out according to the usual method with, for example, ExTaq polymerase (Takara Shuzo). PCR is preferably performed using Vent polymerase (NEB), and more preferably Pfu polymerase (Toyobo). Desired fragments thus amplified are digested with NotI, and then inserted into the NotI site of the plasmid vector pBluescript. The nucleotide sequences of PCR products thus obtained are confirmed using a sequencer to select a plasmid comprising the right sequence. The inserted fragment is excised from the plasmid using NotI, and cloned to the NotI site of the plasmid carrying the genomic cDNA. Alternatively, recombinant Sendai virus cDNA can be obtained by directly inserting the fragment into the NotI site, without the mediation of the plasmid vector.

For example, a recombinant Sendai virus genome cDNA can be constructed according to the method described in the references (Yu, D. *et al*., Genes Cells 2: 457-466, 1997; Hasan, M. K. *et al*., J. Gen. Virol. 78: 2813-2820, 1997). For example, an 18-bp spacer sequence comprising a NotI restriction site (5'-(G)-CGGCCGCAGATCTTCACG-3') (SEQ ID NO: 3) is inserted into a cloned Sendai virus genome cDNA (pSeV(+)) between the leader sequence and the ORF encoding N protein, and thus a plasmid pSeV18⁺b(+) containing a self-cleaving ribozyme site derived from the antigenomic strand of delta-hepatitis virus is obtained (Hasan, M. K. *et al*., 1997, J. General Virology 78: 2813-2820).

For example, in the case of M gene deletion, or introduction of a temperature-sensitive mutation to the M gene, the (-)strand virus' full genome cDNA is digested by a restriction enzyme, and the M gene-comprising fragments are collected and cloned into an appropriate plasmid. M gene mutagenesis or construction of an M gene-deficient site is carried out using this plasmid. The introduction of a mutation can be carried out, for example, using a QuikChange™ Site-Directed Mutagenesis Kit (Stratagene, La Jolla, CA) according to the method described in the kit directions. For example, M gene deficiency or deletion can be carried out using a combined PCR-ligation method, whereby deletion of all or part of the M gene ORF, and ligation with an appropriate spacer sequence, can be achieved. After obtaining an M gene-mutated or -deleted sequence of interest, fragments comprising the sequence are recovered, and the M gene region in the original full length cDNA is substituted by this sequence. Thus, viral genome cDNA or the like, comprising a temperature-sensitive mutant M gene, can be prepared. Using similar methods, mutation can be introduced into, for example, F and/or HN genes.

A (-)strand RNA virus vector in which particle formation ability has been reduced or eliminated can be produced by transcribing the DNA encoding the (-)strand RNA virus vector genome, and reconstituting the vector in cells under a condition ensuring the persistent complementation of the reduction or elimination of M protein localization. The present invention provides a DNA encoding the (-)strand RNA virus vector genome, to be used in the method of the present invention for producing recombinant (-)strand RNA virus vectors in which particle formation ability has been reduced or eliminated. Further, the present invention relates to the use of vector genome-encoding DNA for the present invention's method for producing recombinant (-)strand RNA virus vectors in which particle formation ability has been reduced or eliminated. Viral reconstitution from the viral vector DNA can be carried out using a known method (WO97/16539; WO97/16538; Durbin, A. P. *et al*., 1997, Virology 235: 323-332; Whelan, S. P. *et al*., 1995, Proc. Natl. Acad. Sci. USA 92: 8388-8392; Schnell. M. J. *et al*., 1994, EMBO J. 13: 4195-4203; Radecke, F. *et al*., 1995, EMBO J. 14: 5773-5784; Lawson, N. D. *et al*., Proc. Natl. Acad. Sci. USA 92: 4477-4481; Garcin, D. *et al*., 1995, EMBO J. 14: 6087-6094; Kato, A. *et al*., 1996, Genes Cells 1: 569-579; Baron, M. D. and Barrett, T., 1997, J. Virol. 71: 1265-1271; Bridgen, A. and Elliott, R. M., 1996, Proc. Natl. Acad. Sci. USA 93: 15400-15404). Using these methods, (-)strand RNA viruses, or RNP as viral components, can be reconstituted from their DNA, including viruses such as parainfluenza virus, vesicular stomatitis virus, rabies virus, measles virus, rinderpest virus, Sendai virus, etc.

Specifically, a recombinant (-)strand RNA virus vector can be produced by (a) transcribing a vector DNA encoding the negative strand RNA derived from a (-)strand RNA virus, or the complementary strand (positive strand) thereof, into cells expressing NP, P, and L proteins (helper cells), and (b) culturing these cells, or cells into which the viral vector obtained from these cells or its RNP constituent has been introduced, under a condition ensuring persistent complementation of the reduction or elimination of subcellular M protein localization (for example, the reduction or elimination of cell-surface M protein aggregation), and then recovering viral particles from this culture supernatant. The RNA transcribed from the vector DNA forms an RNP complex with NP, L, and P proteins, and further viral particles, coated with the capsid containing the envelope protein, are formed in step (b). In the present invention, the culture in step (b) is preferably carried out at a low temperature, specifically, at 35°C or lower, more preferably at 34°C or lower, further preferably at 33°C or lower, most preferably at 32°C or lower.

When a temperature-sensitive mutant protein is used, culturing the above cells "under a condition ensuring persistent complementation of the reduction or elimination of subcellular M protein localization" means, specifically, culturing these cells at a permissive temperature. When producing a virus carrying a gene deficiency (or deletion) mutation, this means, for example, culturing under a condition where the wild-type gene, or a gene comprising a function equivalent thereto, is persistently expressed, and more specifically, for example, culturing under a condition where such a gene(s) has been integrated in the chromosome of helper cells and is expressed.

The DNA encoding the viral genome (vector DNA) which is to be expressed in the helper cells encodes the genome minus strand (negative strand RNA) or its complementary strand (positive strand RNA). For example, the DNA encoding the negative strand RNA or its complementary strand is placed downstream of a T7 promoter, and then transcribed into RNA using T7 RNA polymerase. A desired promoter that does not comprise the T7-polymerase recognition sequence can also be used. Alternatively, *in-vitro* transcribed RNA may be transfected into the helper cells. The strand to be transcribed in cells may be the positive or negative strand of the viral genome, but to increase reconstitution efficiency, the positive strand is preferably transcribed.

Methods for transferring vector DNA into cells include the following: 1) the method of preparing DNA precipitates that can be taken up by objective cells; 2) the method of preparing a positively charged DNA-comprising complex which has low cytotoxicity and can be taken up by target cells; and 3) the method of using electric pulses to instantaneously open holes in target cell membranes so that DNA molecules can pass through.

In the above method 2), a variety of transfection reagents can be utilized, examples being DOTMA (Boehringer), Superfect (QIAGEN #301305) , DOTAP, DOPE, DOSPER (Boehringer #1811169), etc. An example of method 1) is a transfection method using calcium phosphate, in which DNA that enters cells is incorporated into phagosomes, but is also incorporated into the nuclei in sufficient amounts (Graham, F. L. and Van Der Eb, J., 1973, Virology 52: 456; Wigler, M. and Silverstein, S., 1977, Cell 11: 223). Chen and Okayama have investigated the optimization of the transfer technique, reporting that optimal precipitates can be obtained under conditions where 1) cells are incubated with co-precipitates in an atmosphere of 2% to 4% CO₂ at 35°C for 15 to 24 hours; 2) circular DNA with a higher activity than linear DNA is used; and 3) DNA concentration in the precipitate mixture is 20 to 30 µg/ml (Chen, C. and Okayama, H., 1987, Mol. Cell. Biol. 7: 2745). Method 2) is suitable for transient transfection. In an older known method, a DEAE-dextran (Sigma #D-9885, M.W. 5 x 10⁵) mixture is prepared in a desired DNA concentration ratio, and transfection is performed. Since many complexes are decomposed inside endosomes, chloroquine may be added to enhance results (Calos, M. P., 1983, Proc. Natl. Acad. Sci. USA 80: 3015). Method 3) is referred to as electroporation, and is more versatile than methods 1) and 2) because it doesn't involve cell selectivity. Method 3) is said to be efficient when conditions are optimal for pulse electric current duration, pulse shape, electric field potency (the gap between electrodes, voltage), buffer conductivity, DNA concentration, and cell density.

Of the above three categories, method 2) is easily operable, and facilitates examination of many test samples using a large numbers of cells. Transfection reagents are therefore suitable for cases where DNA is introduced into cells for vector reconstitution. Preferably, Superfect Transfection Reagent (QIAGEN, Cat. No. 301305) or DOSPER Liposomal Transfection Reagent (Roche, Cat. No. 1811169) is used, but the transfection reagents are not limited thereto.

Specifically, the reconstitution of viral vectors from cDNA can be performed, for example, as follows:

Simian kidney-derived LLC-MK2 cells are cultured to 70% to 80% confluency in 24-well to 6-well plastic culture plates, or 100 mm diameter culture dishes and such, using a minimum essential medium (MEM) containing 10% fetal calf serum (FCS) and antibiotics (100 units/ml penicillin G and 100 µg/ml streptomycin). These cells are then infected, for example, at 2 PFU/cell with recombinant vaccinia virus vTF7-3 expressing T7 polymerase. This virus has been inactivated by UV irradiation treatment for 20 minutes in the presence of 1 µg/ml psoralen (Fuerst, T. R. *et al*., Proc. Natl. Acad. Sci. USA 83: 8122-8126, 1986; Kato, A. *et al*., Genes Cells 1: 569-579, 1996). The amount of psoralen added and the UV irradiation time can be appropriately adjusted. One hour after infection, the lipofection method or the like is used to transfect cells with 2 µg to 60 µg, more preferably 3 µg to 30 µg, of the above-described DNA, which encodes the genomic RNA of the recombinant Sendai virus in which particle formation ability has been reduced or eliminated. Such methods use Superfect (QIAGEN) , and plasmids which express the trans-acting viral proteins'required for the production of viral RNP (0.5 µg to 24 µg of pGEM-N, 0.5 µg to 12 µg of pGEM-P and 0.5 µg to 24 µg of pGEM-L, more preferably, for example, 1 µg of pGEM-N, 0.5 µg of pGEM-P and 1 µg of pGEM-L) (Kato, A. *et al*., Genes Cells 1: 569-579, 1996). The ratio of expression vectors encoding N, P, and L is preferably 2:1:2. The amount of plasmid is appropriately adjusted, for example, to 1 µg to 4 µg of pGEM-N, 0.5 µg to 2 µg of pGEM-P, and 1 µg to 4 µg of pGEM-L. If genes necessary for particle formation are co-transfected at the time, the formed viral particles re-infect helper cells, and the virus can be further amplified. The transfected cells are cultured in a serum-free MEM containing 100 µg/ml each of rifampicin (Sigma) and cytosine arabinoside (AraC) if desired, more preferably containing only 40 µg/ml of cytosine arabinoside (AraC) (Sigma). Reagent concentrations are optimised for minimum vaccinia virus-caused cytotoxicity, and maximum recovery rate of the virus (Kato, A. *et al*., 1996, Genes Cells 1, 569-579). After transfection, cells are cultured for about 48 hours to about 72 hours, recovered, and then disrupted by three repeated freezing and thawing cycles. LLC-MK2 cells are re-transfected with the disrupted cells, and then cultured under a condition where the reduction or elimination of subcellular M protein localization is persistently complemented. In this process, viral particles are formed, and the virus is amplified. Alternatively, the culture supernatant can be recovered and added to the culture medium of cells being cultured for viral production. The cells are cultured for three to seven days under a condition where the reduction or elimination of subcellular M protein localization is persistently complemented, and the culture solution is then collected.

RNP may be introduced to cells as a complex formed together with, for example, lipofectamine and a polycationic liposome. Specifically, a variety of transfection reagents can be utilized. Examples of these are DOTMA (Roche), Superfect (QIAGEN #301305), DOTAP, DOPE, DOSPER (Roche #1811169), etc. Chloroquine may be added to prevent RNP decomposition in endosomes (Calos, M. P., 1983, Proc. Natl. Acad. Sci. USA 80: 3015).

When reconstituting a viral vector deficient in the gene encoding the envelope protein, LLC-MK2 cells expressing the envelope protein may be used for transfection, or co-transfected with an envelope-expression plasmid. Alternatively, the viral vector can be amplified by overlaying the transfected cells onto LLC-MK2 cells expressing the envelope protein, and culturing these cells under a condition where the reduction or elimination of subcellular M protein localization is persistently complemented (see WO00/70055 and WO00/70070). Alternatively, the above-mentioned cell lysate obtained by freeze-thawing may be inoculated through the allantoic membrane into 10-day old embryonated chicken eggs, which are maintained under a condition where the reduction or elimination of subcellular M protein localization is persistently complemented, and the allantoic fluids are recovered after approximately three days. Viral titers in culture supernatants or allantoic fluids can be determined by assaying hemagglutination activity (HA) HA can be determined by the "endo-point dilution method" (Kato, A. *et al*., 1996, Genes Cells 1: 569-579; Yonemitsu, Y. & Kaneda, Y. , Hemaggulutinating virus of Japan-liposome-mediated gene delivery to vascular cells. Ed. by Baker AH. Molecular Biology of Vascular Diseases. Method in Molecular Medicine: Humana Press: pp. 295-306, 1999). The allantoic fluid samples obtained are suitably diluted to remove potentially contaminated T7 polymerase expressing-vaccinia virus (for example, diluted 10⁶ times). The viruses can be amplified again in chicken eggs under a condition where the reduction or elimination of subcellular M protein localization is persistently complemented. Re-amplification can be repeated, for example, three times or more. The viral stock obtained can be stored at -80°C.

Potency of the recovered virus can be determined, for example, by measuring Cell-Infected Units (CIU) or hemagglutination activity (HA) (WO00/70070; Kato, A. *et al*., Genes Cells 1: 569-579 (1996); Yonemitsu, Y. and Kaneda, Y., "Hemaggulutinating virus of Japan-liposome-mediated gene delivery to vascular cells.", Ed. by Baker, A. H., Molecular Biology of Vascular Diseases. Methods in Molecular Medicine. , Humana Press. , pp. 295-306 (1999)). In the case of vectors labeled with a marker gene, such as the green fluorescent protein (GFP) gene, the virus titer is quantified by directly counting infected cells using the marker as an indicator (e.g., as GFP-CIU). Titers thus determined can be considered equivalent to CIU (WO00/70070).

Host cells used for reconstitution are not restricted as long as the viral vector can be reconstituted. For example, in the reconstitution of the Sendai virus vector and such, monkey kidney-derived LLCMK2 cells and CV-1 cells, cultured cells such as hamster kidney-derived BHK cells, human-derived cells, and so on, can be used. By expressing a suitable envelope protein in these cells, infective virions comprising this protein in the envelope can be obtained. Large amounts of a viral vector can be obtained by infecting the viral vector obtained from the above host into embryonated chicken eggs, to amplify this vector under a condition where the reduction or elimination of subcellular M protein localization is persistently complemented. The method of producing viral vectors using chicken eggs has already been developed ("Shinkei-kagaku Kenkyu-no Saisentan Protocol III, Bunshi Shinkei Saibou Seirigaku (Leading edge techniques protocol III in neuroscience research, Molecular, Cellular Neurophysiology)", edited by Nakanishi, et al., KOSEISHA, Osaka, 1993, pp. 153-172). Specifically, for example, fertilized eggs are moved to an incubator, and the embryo is grown under culture for nine to twelve days at 37°C to 38°C. The viral vector is then inoculated into the allantoic membrane cavity, the egg is incubated for a few days to proliferate the viral vector, and the allantoic fluid containing the virus is then collected. Conditions such as culture duration change according to the recombinant virus amplified. Separation and purification of the viral vector from the allantoic fluid is done according to the usual methods ("Protocols of Virology" by Masato Tashiro, edited by Nagai and Ishihama, Medical View, pp.68-73, (1995)).

Specifically, for example, when intending to reconstitute a recombinant (-)strand RNA virus vector comprising a temperature-sensitive mutant M gene with which subcellular M protein localization is reduced or eliminated, the virus can be produced through the steps of (a) transcribing a vector DNA encoding the negative strand RNA derived from the (-)strand RNA virus, or the complementary strand thereof (positive strand) , in cells expressing NP, P, and L proteins (helper cells) , and (b) culturing these cells, or cells in which the viral vector obtained from these cells or the RNP constituent thereof has been introduced, at a permissive or lower temperature, and recovering viral particles from the culture supernatant. In the present invention, particularly, it is preferable to carry out the culture of step (b) at a permissive or lower temperature. That is, 35°C or lower, more preferably 34°C or lower, further preferably 33°C or lower, most preferably 32°C or lower.

A (-)strand RNA virus vector, in which M protein has been deleted, can be constructed and prepared as follows:

### <1> Construction of an M-deleted (-)strand RNA viral genome cDNA and M-expression plasmid

The full-length genome cDNA of a (-)strand RNA virus is digested with restriction enzymes, and fragments containing the M gene are recovered and cloned into pUC18. The M gene deletion site is built in this plasmid. The M gene is deleted by the combined use of PCR and a ligation method. The M gene ORF is removed, and the remainder is ligated together with an appropriate spacer sequence. Thus, M-deleted genomic cDNA is constructed. DNAs encoding the genome regions upstream and downstream of the M gene are amplified using PCR, and these fragments are then ligated to produce a plasmid containing the full-length M-deleted (-)strand RNA viral genome cDNA. A foreign gene can be inserted, for example, into a restriction site within the M-deleted site.

### <2> Preparation of helper cells expressing the (-)strand RNA virus M protein in an inducible fashion

To prepare a vector in which it is possible to express the (-)strand RNA virus M protein in an inducible fashion, for example, inducible promoters or expression regulating systems using recombination (such as Cre/loxP) are used. A Cre/loxP inducible expression plasmid directing expression of the (-)strand RNA viral M gene is constructed by amplifying the (-)strand RNA virus M gene using PCR, and for example, inserting it at a unique SwaI site in plasmid pCALNdlw, which has been designed to inducibly express gene products' using Cre DNA recombinase (Arai, T. *et al*., J. Virology 72, 1998, p1115-1121).

In order to recover infectious viral particles from an M-deficient genome, a helper cell line capable of persistently expressing M protein is established. For example, the monkey kidney-derived cell line LLC-MK2 or the like can be used for such cells. LLC-MK2 cells are cultured at 37°C in MEM containing 10% heat-treated immobilized fetal bovine serum (FBS) , 50 units/ml sodium penicillin G, and 50 µg/ml streptomycin, under an atmosphere of 5% CO₂. The above-mentioned plasmid, which has been designed to inducibly express the M gene products with Cre DNA recombinase, is introduced into LLC-MK2 cells using a calcium-phosphate method (mammalian transfection kit (Stratagene)) according to known protocols.

For example, 10 µg of M-expression plasmid is transfected into LLC-MK2 cells grown to be 40% confluent in a 10-cm plate. These cells are then incubated in an incubator at 37°C, in 10 ml of MEM containing 10% FBS and under 5% CO₂. After 24 hours of incubation, the cells are harvested and suspended in 10 ml of medium. The suspension is then plated onto five dishes of 10-cm diameter: 5 ml of the suspension are added to one dish, 2 ml to two dishes, and 0.2 ml to two dishes. The cells in each dish are cultured with 10 ml of MEM containing 10% FBS and 1200 µg/ml G418 (GIBCO-BRL) for 14 days; the medium is changed every two days. Thus, cell lines in which the gene has been stably introduced are selected. The G418-resistant cells grown in the medium are harvested using cloning rings. Cells of each clone harvested are further cultured to confluence in a 10-cm plate.

High level expression of M protein in helper cells is important in recovering a high titer virus. For this purpose, for example, the above selection of M-expressing cells is preferably carried out twice or more. For example, an M-expressing plasmid comprising a drug-resistance marker gene is transfected, and cells comprising the M gene are selected using the drug. Following this, an M-expressing plasmid comprising a marker gene resistant to a different drug is transfected into the same cells, and cells are selected using this second drug-resistance marker. Cells selected using the second marker are likely to express M protein at a higher level than cells selected after the first transfection. Thus, M-helper cells constructed through twice-repeated transfections can be suitably applied. Since the M-helper cells can simultaneously express the F gene, production of infective viral particles deficient in both F and M genes is possible (see Examples). In this case also, transfection of the F-gene-expressing plasmids more than twice is preferable in raising the level of F protein expression induction.

M protein induction expression is achieved by incubating cells to confluence in a 6-cm dish, and then, for example, infecting these cells with adenovirus AxCANCre at MOI=∼3, according to the method of Saito *et al*. (Saito *et al*., Nucl. Acids Res. 23: 3816-3821 (1995); Arai, T. *et al*., J. Virol. 72, 1115-1121 (1998)).

### <3> Reconstitution and amplification of an M-deleted virus

To produce recombinant M-deficient (-)strand RNA virus particles using cells that express wild-type M protein or an equivalent protein (M helper cells), the M-deficient (-)strand RNA virus RNP may be introduced into or produced in these cells. RNP can be introduced into M helper cells, for example, by the transfection of RNP-containing cell lysate into M helper cells, or by cell fusion induced by the co-cultivation of RNP-producing cells and M helper cells. It can also be achieved by transcribing genomic RNA into M helper cells and conducting *de novo* RNP synthesis the presence of N, P, and L proteins. A recombinant viral vector can be reconstituted and produced from a (-)strand RNA viral genome cDNA in which the M gene has been deleted. Specifically, this can be achieved by (a) transcribing a vector DNA encoding the M gene-deleted negative strand RNA (derived from the (-)strand RNA virus or the complementary strand thereof (positive strand) ) in cells expressing NP, P, and L proteins (helper cells) , and (b) culturing these cells, or cells in which the viral vector obtained from these cells or the RNP constituent thereof has been introduced, under a condition ensuring expression of the chromosomally integrated M gene in these or the transfected cells, and then recovering viral particles from the culture supernatant. In the present invention, it is particularly preferable to carry out the culture of step (b) at a permissive or lower temperature. Such a temperature can be 35°C or less, more preferably 34°C or less, further preferably 33°C or less, most preferably 32°C or less. In the production of a vector using a temperature-sensitive mutant M protein, the process of producing viral particles is necessarily carried out at less than the permissive temperature. However, surprisingly, the present inventors found that in the present method, efficient particle production was possible when the process of viral particle formation was carried out at low temperatures, even when using the wild-type M protein. In step (a), the expression of NP, P, and L proteins can be achieved, for example, by transfecting the expression plasmids encoding these proteins into cells. Further, in step (a) , M protein may be expressed in the helper cells (in the case of an F and/or HN gene-deficient virus, these genes may also be expressed) (see the Examples). Alternatively, the cells to be used in step (b) , in which the M gene has been chromosomally integrated, can also be used in step (a). RNP produced in step (a) can be introduced into the cells in step (b), for example, by disrupting the cells of step (a) by freeze-thawing, and then introducing the lysate into the cells of step (b) using known transfection reagents.

Specifically, for example, a plasmid encoding the above M-deleted (-)strand RNA viral genome is transfected into LLC-MK2 cells as follows: When inducing the transcription of genomic RNA using T7 RNA polymerase, LLC-MK2 cells are plated in a 100-mm Petri dish at 5x10⁶ cells/dish, and cultured for 24 hours. The cells are then infected at room temperature for one hour with T7 RNA polymerase-expressing recombinant vaccinia virus (PLWUV-VacT7) (MOI=2), which has been treated with psoralen and long-wavelength ultraviolet light (365 nm) for 20 minutes (MOI=2∼3, and preferably MOI=2 can also be used) (Fuerst, T.R. *et al*., Proc. Natl. Acad. Sci. USA 83, 8122-8126 (1986)). The vaccinia virus is irradiated with ultraviolet light using, for example, a UV Stratalinker 2400 equipped with five 15 watt bulbs (catalogue No. 400676 (100V); Stratagene, La Jolla, CA, USA). The cells are washed three times. Plasmids expressing the genomic RNA, and plasmids directing the expression of NP, P, and L proteins (optionally, of M protein and others) are suspended in OptiMEM (GIBCO) , and SuperFect transfection reagent (1 µg DNA/5 µl SuperFect, QIAGEN) is added and mixed. The mixture is allowed to stand at room temperature for ten minutes, and then added to 3 ml of OptiMEM containing 3% FBS. Alternatively, plasmids expressing the genomic RNA and plasmids expressing the N, P, L, F, and HN proteins respectively, may be used to transfect cells using appropriate lipofection reagents. The ratio of plasmids can be, for example, 6:2:1:2:2:2, but it is not limited thereto. After culturing for three to five hours, the cells are washed twice with serum-free MEM, and then cultured for 24 to 70 hours in MEM containing 40 µg/ml cytosine-β-D-arabinofuranoside (AraC, Sigma) , and 7.5 µg/ml trypsin (GIBCO). Herein, the cells may be overlaid with cells that express M protein continuously (M helper cells), at a density of about 8.5 x 10⁶ cells/dish, and then cultured for a further two days at 37°C in MEM containing 40 µg/ml AraC and 7.5 µg/ml trypsin. The cultured cells are collected and the precipitate is suspended in OptiMEM at 10⁷ cells/ml. After mixing with the lipofection reagent DOSPER (Roche) by freezing and thawing three times (at 10⁶ cells/25 µl), the mixture is allowed to stand at room temperature for 15 minutes, and is then transfected to the M-expressing helper cells that were cloned as described above (at 10⁶ cells/well on a 12-well-plate). The transfected cells are cultured in serum-free MEM (containing 40 µg/ml AraC and 7.5 µg/ml trypsin), preferably at a low temperature, and the supernatant is recovered. Transfection without the addition of a transfection reagent, such as Lippofection reagent DOSPER, is also possible in this process. Similarly, viral vectors with deficient F and/or HN proteins can be produced by deleting the F and/or HN genes from the genome, and then inducing co-expression of F and/or HN proteins in helper cells.

According to the present invention, a viral vector can be released in to the external fluid of virus-producing cells, for example, at a titer of 1x10⁵ CIU/ml or more, preferably 1x10⁶ CIU/ml or more, more preferably 5x10⁶ CIU/ml or more, more preferably 1x10⁷ CIU/ml or more, more preferably 5x10⁷ CIU/ml or more, more preferably 1x10⁸ CIU/ml or more, and more preferably 5x10⁸ CIU/ml or more. The virus titer can be measured by the methods described in the specification and other literature (Kiyotani, K. *et al*., Virology 177(1): 65-74 (1990); WO00/70070).

One preferred embodiment of a method for reconstituting a recombinant viral vector from the M-deleted (-)strand RNA viral genome cDNA is as follows: Namely, the method comprises the steps of (a) transcribing a vector DNA encoding the negative strand RNA in which the M gene has been deleted (derived from the (-)strand RNA virus, or the complementary strand thereof (positive strand)) in cells expressing the viral proteins necessary for the formation of infective viral particles (i.e., NP, NP, P, L, M, F, and HN proteins) (helper cells); (b) co-culturing these cells with cells expressing chromosomally integrated M gene (M helper cells); (c) preparing a cell extract from this culture; (d) introducing the extract into the cells expressing the chromosomally integrated M gene (M helper cells) and culturing these cells; and (e) recovering viral particles from the culture supernatant. Step (d) is preferably carried out under the low temperature conditions described above. The obtained viral particles can be amplified by re-infection of helper cells (preferably at low temperatures). Specifically, the virus can be reconstituted according to the description in the Examples.

When preparing a vector with deficient viral genes, for example, two or more vector types, each of which has a different deficient viral gene in it's viral genome, are introduced into the same cells. Each deficient viral protein is expressed and supplied by the other vector, this mutual complementation results in the formation of infective viral particles, and the viral vector can be amplified in the replication cycle. Namely, when two or more types of vector of the present invention are inoculated in combination to complement viral proteins, mixed viral gene-deficient viral vectors can be produced on a large scale and at a low cost. As these viruses lack viral genes, their genome is smaller than that of an intact virus, and they can thus comprise larger foreign genes. In addition, co-infectivity is difficult to maintain in these viruses, which are non-propagative due to viral gene deficiency, and are diluted outside of cells. Such vectors are thus sterile, which is advantageous from the viewpoint of controlling environmental release.

A recovered (-)strand RNA virus can be purified so as to be substantially pure. Purification can be performed by known purification and separation methods including filtration, centrifugation, column chromatographic purification, and such, or by combination thereof. "Substantially pure" used herein means that the virus, as a component, is the main proportion of the sample in which the virus exists. Typically, substantially pure viral vectors can be detected by confirming that the ratio of virus-derived protein to total protein in the sample (except protein added as a carrier or stabilizer) is 10% or more, preferably 20% or more, more preferably 50% or more, more preferably 70% or more, more preferably 80% or more, and even more preferably 90% or more. Specifically, a (-)strand RNA virus can be purified, for example, by a method in which cellulose sulfate ester or crosslinked polysaccharide sulfate ester is used (Examined Published Japanese Patent Application (JP-B) No. Sho 62-30752; JP-B Sho 62-33879; JP-B Sho 62-30753), a method in which adsorption to fucose sulfate-containing polysaccharide and/or a decomposition product thereof is used (WO97/32010), etc.

When a viral vector is prepared using a therapeutic gene as the foreign gene, gene therapy can be carried out by administering that viral vector. In applying viral vectors produced by this invention to gene therapy, it is possible to express a foreign gene expected to comprise treatment effects, or an endogenous gene which is in insufficient supply in the patient's body. This can be achieved by either direct (*in vivo*) or indirect (*ex vivo*) administration of the complex. There is no particular limitation as to the type of foreign gene, and they may include nucleic acids that encode proteins, and nucleic acids that do not encode proteins, such as an antisense or ribozyme nucleic acids.

A (-)strand RNA virus vector produced by the method of the present invention can be formulated into a composition, as required, by combining it with a desired pharmaceutically acceptable carrier or solvent. A "pharmaceutically acceptable carrier or solvent" refers to a material that can be administered along with the vector, and that does not significantly inhibit gene transfer of that vector. For example, a vector can be formulated into a composition by appropriately diluting it with physiological saline, phosphate-buffered physiological saline (PBS), or so on. When the (-)strand RNA virus vector is propagated in chicken eggs or so on, the composition may contain allantoic fluid. Further, a composition comprising the vector may contain carriers or solvents such as deinonized water and 5% dextrose solution. In addition to these, the composition can contain vegetable oil, suspending agent, detergent, stabilizer, biocide, etc. Further, preservatives and other additives can be added to the composition. Compositions containing the (-)strand RNA virus vector are useful as reagents and pharmaceuticals.

Vector dosage depends on the type of disease, the patient's weight, age, sex and symptoms, the purpose of administration, the dosage form of the composition to be administered, the method of administration, type of gene to be introduced, etc. However, those skilled in the art can determine the dosage properly. The administration dose of a (-)strand RNA virus vector is preferably within about 10⁵ to 10¹¹ CIU/ml, more preferably within about 10⁷ to 10⁹ CIU/ml, most preferably within about 1x10⁸ to 5x10⁸ CIU/ml. It is preferable to administer the vector mixed with pharmaceutically acceptable carriers. The preferred dose for each administration to a human individual is 2x 10⁹ to 2x 10¹⁰ CIU. Administration can be carried out one or more times within the limits of clinically acceptable side effects. The frequency of daily administration can be similarly determined. When administering the viral vector to animals other than humans, for example, the dose to be administered can be determined by converting the above dose based on the weight ratio, or the volume ratio of the administration target sites (for example, an average value) between the target animals and humans. A composition containing the (-)strand RNA virus vector can be administered to all mammalian species including humans, monkeys, mice, rats, rabbits, sheep, cattle, dogs, etc.

### Brief Description of the Drawings

Fig. 1 shows a schematic representation of the construction of an F-deleted SeV genome cDNA in which a temperature-sensitive mutation has been introduced into the M gene.
Fig. 2 shows the structures of viral genes constructed to suppress secondary particle release based on temperature-sensitive mutations introduced into the M gene, and viral genes constructed or used to test and compare the effects of these introduced mutations.
Fig. 3 shows microscopic images representing GFP expression in cells (LLC-MK2/F7/A) persistently expressing F protein, which were cultured at 32°C and 37°C for six days after infection with SeV18+/ΔF-GFP or SeV18+/MtsHNtsΔF-GFP.
Fig. 4 shows a picture representing the result of semi-quantitative determination, over time and using Western-blotting, of F protein expression levels in cells (LLC-MK2/F7/A) persistently expressing SeV-F protein, which were cultured in trypsin-free, serum-free MEM at 32°C or 37°C.
Fig. 5 shows microscopic images representing GFP expression in LLC-MK2 cells which were cultured at 32°C, 37°C or 38°C for three days after infection with SeV18+GFP, SeV18+/ΔF-GFP or SeV18+/MtsHNtsΔF-GFP at MOI=3.
Fig. 6 shows hemagglutination activity (HA activity) in the culture supernatant, which was sampled over time (fresh medium was introduced at the same time) , of LLC-MK2 cells cultured at 32°C, 37°C or 38°C after infection with SeV18+GFP, SeV18+/ΔF-GFP or SeV18+/MtsHNtsΔF-GFP at MOI= 3.
Fig. 7 shows pictures representing the ratio of M protein level in cells to that in virus-like particles (VLPs). This ratio was determined by Western-blotting using an anti-M antibody. The culture supernatant and cells were recovered from a LLC-MK2 cell culture incubated at 37°C for two days after infection with SeV18+GFP, SeV18+/ΔF-GFP or SeV18+/MtsHNtsΔF-GFP at MOI=3. Each lane contained the equivalent of 1/10 of the content of one well from a 6-well plate culture.
Fig. 8 shows SEAP activity in the culture supernatant of LLC-MK2 cells cultured for 12, 18, 24, 50, or 120 hours after infection with SeV18+SEAP/ΔF-GFP or SeV18+SEAP/MtsHNtsΔF-GFP at MOI=3.
Fig. 9 shows HA activity in the culture supernatant of LLC-MK2 cells cultured for 24, 50, or 120 hours after infection with SeV18+SEAP/ΔF-GFP or SeV18+SEAP/MtsHNtsΔF-GFP at MOI=3.
Fig. 10 shows a picture representing the quantity of VLPs determined by Western-blotting using an anti-M antibody. LLC-MK2 cells were cultured for five days after infection with SeV18+SEAP/ΔF-GFP or SeV18+SEAP/MtsHNtsΔF-GFP at MOI=3. The culture supernatant was centrifuged to recover the viruses. Each lane contained the equivalent of 1/10 of the content of one well from a 6-well plate culture.
Fig. 11 shows cytotoxicity estimates based on the quantity of LDH released into the cell culture medium. LLC-MK2, BEAS-2B or CV-1 cells were infected with SeV18+GFP, SeV18+/ΔF-GFP or SeV18+/MtsHNtsΔF-GFP at MOI=0.01, 0.03, 0.1, 0.3, 1, 3, or 10. Cells were cultured in a serum-free or 10% FBS-containing medium, and the cytotoxicity assay was carried out three or six days after infection, respectively.
Fig. 12 shows pictures representing the subcellular localization of M protein in LLC-MK2 cells cultured at 32°C, 37°C or 38°C for two days after infection with SeV18+GFP, SeV18+/ΔF-GFP or SeV18+/MtsHNtsΔ-F-GFP at MOI=1, which was observed by immunostaining using an anti-M antibody.
Fig. 13 shows stereo three-dimensional images for the subcellular localization of M and HN proteins observed under a confocal laser microscope. A-10 cells were infected with SeV18+SEAP/ΔF-GFP or SeV18+SEAP/MtsHNtsΔF-GFP at MOI=1, and then cultured at 32°C or 37°C for one day. These images were obtained by immunostaining using an anti-M antibody and anti-HN antibody.
Fig. 14 shows stereo three-dimensional images for the subcellular localization of M and HN proteins observed under a confocal laser microscope. A-10 cells were infected with SeV18+SEAP/ΔF-GFP or SeV18+SEAP/MtsHNtsΔF-GFP at MOI=1, and then cultured at 32°C or 37°C for two days. These images were obtained by immunostaining using an anti-M antibody and anti-HN antibody.

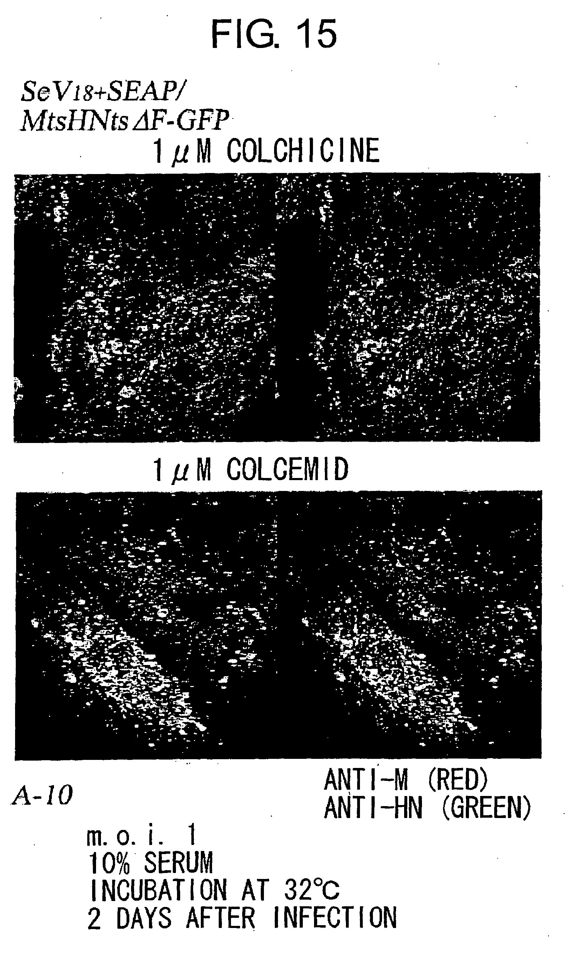

Fig. 15 shows pictures representing the effect of microtubule depolymerization reagent on the subcellular localization of M and HN proteins. A-10 cells were infected with SeV18+SEAP/MtsHNtsΔF-GFP at MOI=1, and a microtubule depolymerization reagent, colchicine or colcemid, was immediately added to these cells at a final concentration of 1 µM. The cells were cultured at 32°C. After two days, the cells were immunostained with an anti-M antibody and anti-HN antibody and then observed under a confocal laser microscope. These photographs show stereo three-dimensional images of the subcellular localization of M and HN proteins.

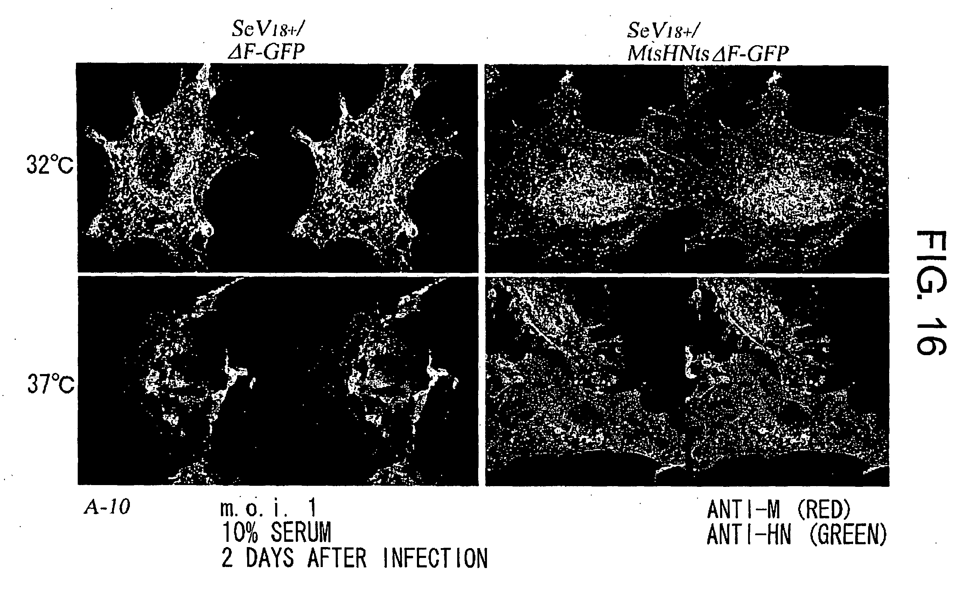

Fig. 16 shows pictures representing the effect of microtubule depolymerization reagent on the subcellular localization of M and HN proteins. A-10 cells were infected with SeV18+/ΔF-GFP or SeV18+/MtsHNtsΔF-GFP at MOI=1, and a microtubule depolymerization reagent, colchicine, was immediately added to the cells at a final concentration of 1 µM. The cells were cultured at 32°C or 37°C. After two days, these cells were immunostained with anti-M antibody and anti-HN antibody, and then observed under a confocal laser microscope. These photographs show stereo three-dimensional images for the subcellular localization of M and HN proteins.
Fig. 17 shows the construction scheme of the genome cDNA of the F-deficient SeV comprising P and L gene mutations.
Fig. 18 shows the result of the secondary release of viral particles from cells infected by the F-deficient SeV comprising P and L gene mutations. "dF" represents SeV18+/ΔF-GFP. "P86" represents SeV18+/P86Lmut ^{·} ΔF-GFP. "P511" represents SeV18+/P511Lmut ^{·}ΔF-GFP.
Fig. 19 shows the cytotoxicity results for the F-deficient SeV comprising P and L gene mutations. "P86" represents SeV18+/P86Lmut ^{·} ΔF-GFP. "P511" represents SeV18+/P511Lmut ^{·} ΔF-GFP. "DF" represents SeV18+/ΔF-GFP.
Fig. 20 shows the change in the number of cells expressing the introduced gene (GFP) in the CV-1 cells that were infected by the F-deficient SeV comprising P and L gene mutations. "P86" represents SeV18+/P86Lmut ^{·}ΔF-GFP. "P511" represents SeV18+/P511Lmut ^{·}ΔF-GFP. "dF" represents SeV18+/ΔF-GFP.
Fig. 21 shows photographs representing expression of the introduced gene (GFP) in the CV-1 cells that were infected by the F-deficient SeV comprising P and L gene mutations. "P86" represents SeV18+/P86Lmut ^{·}ΔF-GFP. "P511" represents SeV18+/P511Lmut ^{·}ΔF-GFP. "ΔF" represents SeV18+/ΔF-GFP.
Fig. 22 shows the constitutive expression of the introduced gene (SEAP) in cells infected with F-deficient SeV comprising P and L gene mutations. "NC" represents the negative control into which no vector was introduced. "dF" represents SeV18+SEAP/ΔF-GFP. "p86" represents SeV18+SEAP/P86Lmut ΔF-GFP. "P511" represents SeV18+SEAP/P511Lmut ^{·}ΔF-GFP.
Fig. 23 shows the result of the secondary release of viral particles from the cells that were infected by the F-deficient SeV (SEAP gene-comprising type) comprising P and L gene mutations. "dF" represents SeV18+SEAP/ΔF-GFP. "p86" represents SeV18+SEAP/P86Lmut ·ΔF-GFP. "P511" represents SeV18+SEAP/P511Lmut ·ΔF-GFP.
Fig. 24 shows cytotoxicity results for F-deficient SeV (SEAP gene-comprising type) comprising P and L gene mutations. "dF+SEAP" represents SeV18+SEAP/ΔF-GFP. "p86+SEAP" represents SeV18+SEAP/P86Lmut ^{·} ΔF-GFP. "P511+SEAP" represents SeV18+SEAP/P511Lmut ^{·}ΔF-GFP.
Fig. 25 shows the genomic structure of the F-deficient SeV comprising temperature-sensitive mutations in the M and HN genes and mutations in the P and L genes.
Fig. 26 shows the construction scheme for the genome cDNA of the F-deficient SeV comprising temperature-sensitive mutations in the M and HN genes and mutations in the P and L genes.
Fig. 27 shows the result of the secondary release of viral particles from cells infected with F-deficient SeV comprising temperature-sensitive mutations in the M and HN genes and mutations in the P and L genes. "dF" represents SeV18+/ΔF-GFP. "ts" represents SeV18+/MtsHNts ΔF-GFP. "ts+86" represents SeV18+/MtsHNts P86Lmut ^{·}ΔF-GFP. "ts+511" represents SeV18+/MtsHts P511Lmut ^{·}ΔF-GFP.
Fig. 28 shows cytotoxicity results for F-deficient SeV comprising temperature-sensitive mutations in the M and HN genes and mutations in the P and L genes. "dF" represents SeV18+/ ΔF-GFP. "ts" represents SeV18+/MtsHNts ΔF-GFP. "ts+86" represents SeV18+/MtsHNts P86Lmut ^{·}ΔF-GFP. "ts+511" represents SeV18+/MtsHts P511Lmut ^{·}ΔF-GFP.
Fig. 29 shows the results of time-course expression for a foreign gene in the F-deficient SeV (foreign gene-comprising type) comprising temperature-sensitive mutations in the M and HN genes and mutations in the P and L genes. "dF" represents SeV18+SEAP/ ΔF-GFP. "ts+86" represents SeV18+SEAP/MtsHNts P86Lmut ^{·}ΔF-GFP. "ts+511" represents SeV18+SEAP/MtsHNts P511Lmut ^{·}ΔF-GFP.
Fig. 30 shows the results of cytotoxicity time-courses for the F-deficient SeV (foreign gene-comprising type) comprising temperature-sensitive mutations in the M and HN genes and mutations in the P and L genes. "dF" represents SeV18+SEAP/ΔF-GFP. "dF/ts+P86L" represents SeV18+SEAP/MtsHNts P86Lmut ^{·} ΔF-GFP. "dF/ts+P511L" represents SeV18+SEAP/MtsHNts P511Lmut ^{·}ΔF-GFP.
Fig. 31 shows a schematic representation of the construction of an M-deleted SeV genome cDNA comprising the EGFP gene.
Fig. 32 shows a schematic representation of the construction of'an F- and M-deleted SeV genome cDNA.
Fig. 33 shows the structures of the constructed F- and/or M-deleted SeV genes.
Fig. 34 shows a schematic representation of the construction of an M gene-expressing plasmid comprising the hygromycin-resistance gene.
Fig. 35 shows pictures representing a semi-quantitative comparison, by Western-blotting, of the expression levels of M and F proteins in cloned cells inducibly expressing the cloned M protein (and F protein); following infection with a recombinant adenovirus (AcCANCre) that expresses Cre DNA recombinase.
Fig. 36 shows pictures representing the viral reconstitution of M-deleted SeV (SeV18+/ΔM-GFP) with helper cell (LLC-MK2/F7/M) clones #18 and #62.
Fig. 37 shows the viral productivity of SeV18+/ΔM-GFP (CIU and HAU time courses).
Fig. 38 shows pictures and an illustration representing the result of RT-PCR confirming gene structure in SeV18+/ΔM-GFP virions.
Fig. 39 shows pictures representing the result of a comparison of SeV18+/ΔM-GFP with SeV18+GFP and SeV18+/ΔF-GFP, where, after infection of LLC-MK2 cells, Western-blotting was carried out on the viral proteins from these cells and cell cultures to confirm the viral structure of SeV18+/ΔM-GFP from a protein viewpoint.
Fig. 40 shows pictures representing a quantitative comparison of virus-derived proteins in the culture supernatant of LLC-MK2 cells infected with SeV18+/ΔM-GFP and SeV18+/ΔF-GFP (a series of dilutions were prepared and assayed using Western-blotting). Anti-SeV antibody was used.
Fig. 41 shows HA activity in the culture supernatant, collected over time, of LLC-MK2 cells infected with SeV18+/ΔM-GFP or SeV18+/ΔF-GFP at MOI=3.
Fig. 42 shows fluorescence microscopic images obtained five days after LLC-MK2 cells were infected with SeV18+/ΔM-GFP or SeV18+/ΔF-GFP at MOI=3.
Fig. 43 shows fluorescence microscopic images of LLC-MK2 cells prepared as follows: LLC-MK2 cells were infected with SeV18+/ΔM-GFP or SeV18+/ΔF-GFP at MOI=3, and then five days after infection the culture supernatant was recovered and transfected into LLC-MK2 cells using a cationic liposome (Dosper). Microscopic observation was carried out after two days.
Fig. 44 shows pictures representing the viral reconstitution of F- and M-deleted SeV (SeV18+/ΔMΔF-GFP).
Fig. 45 shows fluorescence microscopic images obtained three and five days after cells expressing both M and F (LLC-MK2/F7/M62/A) were infected with SeV18+/ΔM-GFP or SeV18+/ΔF-GFP.
Fig. 46 shows the construction scheme of M or G gene expression-inducing vectors comprising the zeocin-selective marker.
Fig. 47 shows photographs representing the expression of M and F proteins in helper cells expressing M and F.
Fig. 48 shows photographs representing the GFP expression in cells infected by M/F double-deficient SeV comprising the GFP gene.
Fig. 49 shows the result of virus production by cells infected by M/F double-deficient SeV comprising the GFP gene.
Fig. 50 shows photographs indicating the result of confirmation of the M/F double-deficient SeV genomic structure using RT-PCR. "dF" represents SeV18+/ ΔF-GFP. "dM" represents SeV18+/ ΔM-GFP. "dMdF" represents SeV18+/ ΔMΔF-GFP.
Fig. 51 shows photographs representing the result of confirmation of the absence of M and F protein expression in cells infected by M/F double-deficient SeV.
Fig. 52 shows the results of analysis of the presence and absence of the secondary release of viral particles from cells infected by M/F double-deficient SeV, analyzed by measuring HA activity.
Fig. 53 shows photographs representing the results of analysis of the presence and absence of the secondary release of viral particles from cells infected by M/F double-deficient SeV, analyzed using culture supernatant fluid transfection.
Fig. 54 shows photographs representing the infectivity of M/F double-deficient SeV and M-deficient SeV in nerve cells of the cerebral cortex.
Fig. 55 shows photographs representing the expression of induced genes after the *in vivo* application of M/F double-deficient SeV and M-deficient SeV into Mongolian gerbil brains.
Fig. 56 shows the results of infectivity-dependent cytotoxicity for M/F double-deficient SeV and M-deficient SeV. "Positive control" represents SeV with full replicative activity (Sev18+GFP). "dF" represents SeV18+/ΔF-GFP. "dM" represents SeV18+/ΔM-GFP. "dMdF" represents SeV18+/ ΔMΔF-GFP.

### Best Mode for Carrying Out the Invention

The present invention is illustrated in detail below with reference to Examples, but is not to be construed as being limited thereto. All references cited herein are incorporated by reference.

### [Example 1] Construction of an F-deleted SeV genome cDNA in which temperature-sensitive mutations have been introduced

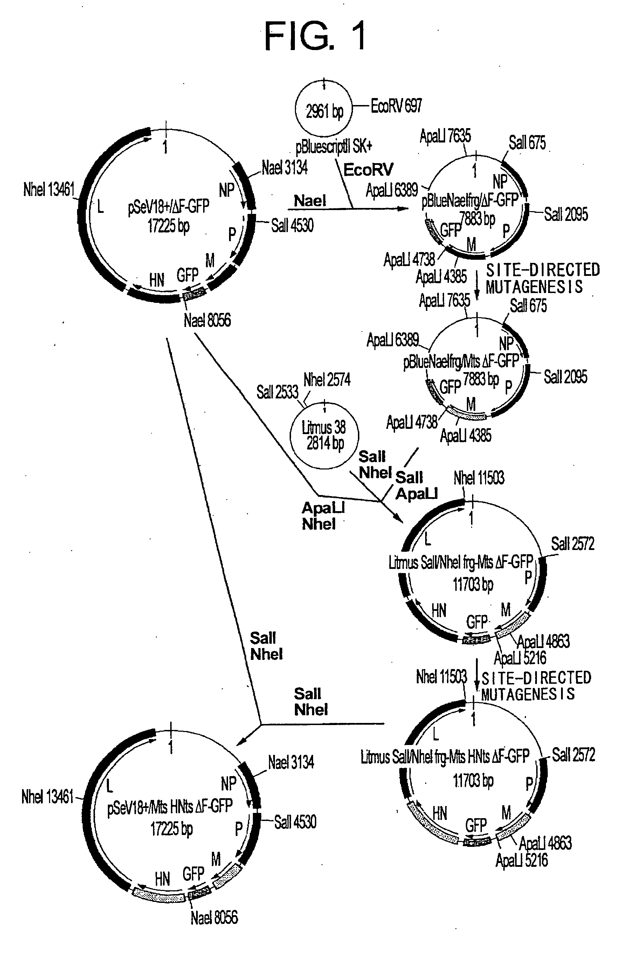

Fig. 1 shows a scheme that represents the construction of a F-deficient Sev genome cDNA introduced with temperature-sensitive mutations, described as follows: An F-deleted full-length Sendai viral genome cDNA containing the EGFP gene at the F deletion site (pSeV18+/ΔF-GFP: Li, H.-O. *et al*., J. Virology 74, 6564-6569 (2000) ; WO00/70070) was digested with NaeI. The M gene-containing fragment (4922 bp) was separated using agarose electrophoresis. After cutting the band of interest out, the DNA was recovered by QIAEXII Gel Extraction System (QIAGEN, Bothell, WA) and subcloned into pBluescript II (Stratagene, La Jolla, CA) at the EcoRV site (pBlueNaeIfrg-ΔFGFP construction). Introduction of temperature-sensitive mutations into the M gene of pBlueNaeIfrg-ΔFGFP was achieved using a QuikChange™ Site-Directed Mutagenesis Kit (Stratagene, La Jolla, CA) , according to kit method. The three types of mutation introduced into the M gene were G69E, T116A and A183S, based on the sequence of the Cl.151 strain reported by Kondo *et al*. (Kondo, T. *et al*., J. Biol. Chem. 268: 21924-21930 (1993)). The sequences of the synthetic oligonucleotides used to introduce the mutations were: G69E (5'-gaaacaaacaaccaatctagagagcgtatctgacttgac-3'/SEQ ID NO: 4, 5'-gtcaagtcagatacgctctctagattggttgtttgtttc-3'/SEQ ID NO: 5), T116A (5'-attacggtgaggagggctgttcgagcaggag-3'/SEQ ID NO: 6, 5'-ctcctgctcgaacagccctcctcaccgtaat-3'/SEQ ID NO: 7). and A183S (5'-ggggcaatcaccatatccaagatcccaaagacc-3'/SEQ ID NO: 8, 5'-ggtctttgggatcttggatatggtgattgcccc-3'/SEQ ID NO: 9).

The plasmid pBlueNaeIfrg-ΔFGFP, whose M gene contains the three mutations, was digested with SalI and then partially digested with ApaLI. The fragment containing the entire M gene was then recovered (2644 bp). pSeV18+/ΔF-GFP was digested with ApaLI/NheI, and the HN gene-containing fragment (6287 bp) was recovered. The two fragments were subcloned into Litmus38 (New England Biolabs, Beverly, MA) at the SalI/NheI site (LitmusSalI/NheIfrg-MtsΔFGFP construction). Temperature-sensitive mutations were introduced into the LitmusSalI/NheIfrg-MtsΔFGFP HN gene in the same way as for the introduction of mutations into the M gene, by using a QuikChange™ Site-Directed Mutagenesis Kit according to kit method. The three mutations introduced into the HN gene were A262T, G264R and K461G, based on the sequence of ts271 strain reported by Thompson *et al*. (Thompson, S.D. *et al*., Virology 160: 1-8 (1987)). The sequences of the synthetic oligonucleotides used to introduce the mutations were: A262T/G264R (5'-catgctctgtggtgacaacccggactaggggttatca-3'/SEQ ID NO: 10, 5'-tgataacccctagtccgggttgtcaccacagagcatg-3'/SEQ ID NO: 11), and K461G (5'-cttgtctagaccaggaaatgaagagtgcaattggtacaata-3'/SEQ ID NO: 12, 5'-tattgtaccaattgcactcttcatttcctggtctagacaag-3'/SEQ ID NO: 13). The mutations were introduced into the M and HN genes in separate vectors, but it is also possible to introduce all of the mutations into both M and HN genes by using a plasmid (LitmusSalI/NheIfrg-ΔFGFP) obtained by subcloning, at the SalI/NheI site of Litmus38, a fragment containing the M and HN genes (8931 bp), provided by digesting pSeV18+/ΔF-GFP with SalI/NheI. Successive introduction of mutations resulted in the introduction of six temperature-sensitive mutations in total; three mutations on the M gene, and three mutations on the HN gene (LitmusSalI/NheIfrg-MtsHNtsΔFGFP construction).

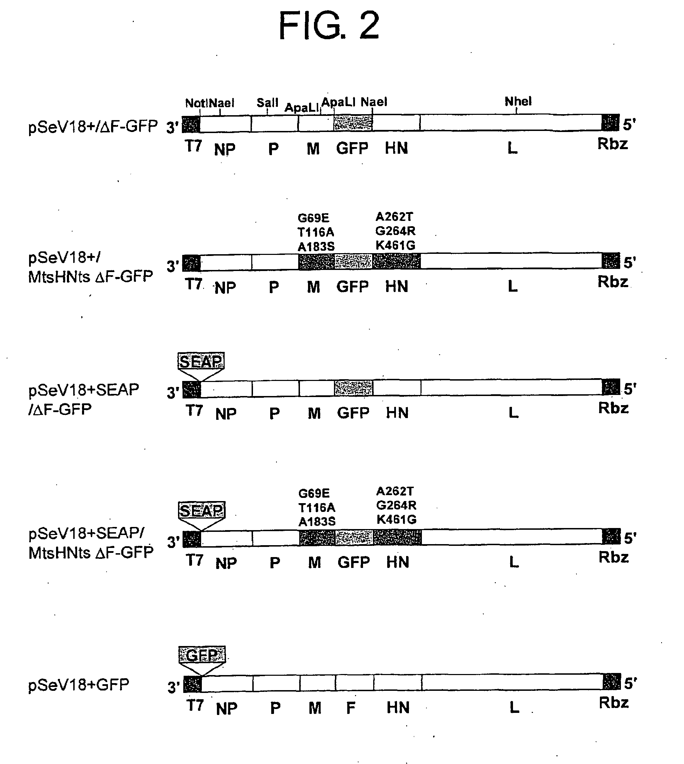

LitmusSalI/NheIfrg-MtsHNtsΔFGFP was digested with SalI/NheI and a 8931 bp fragment was recovered. Another fragment (8294 bp), lacking the M and HN genes and such, was recovered on digestion of pSeV18+/ΔF-GFP with SalI/NheI. Both fragments were ligated together to construct the F-deleted full-length Sendai virus genome cDNA (pSeV18+/MtsHNtsΔF-GFP) comprising the six temperature-sensitive mutations in the M and HN genes, and the EGFP gene at the site of the F deletion (Fig. 2).

Further, to quantify the expression level of genes in the plasmid, a cDNA containing the secretory alkaline phosphatase (SEAP) gene was also constructed. Specifically, NotI was used to cut out an SEAP fragment (1638 bp), comprising the termination signal-intervening sequence-initiation signal downstream of the SEAP gene (WO00/70070). This fragment was recovered and purified following electrophoresis. The fragment was then inserted into pSeV18+/ΔF-GFP and pSeV18+/MtsHNtsΔF-GFP at their respective NotI sites. The resulting plasmids were named pSeV18+SEAP/ΔF-GFP and pSeV18+SEAP/MtsHNtsΔF-GFP, respectively (Fig. 2).

### [Example 2] Reconstitution and amplification of a virus in which temperature-sensitive mutations had been introduced

Viral reconstitution was performed according to the report by Li *et al*. (Li, H.-O. *et al*., J. Virology 74. 6564-6569 (2000); WO00/70070). Since F was deleted in the virus, F protein helper cells were utilized, prepared using an inducible Cre/loxP expression system. The system uses a pCALNdLw plasmid, designed for Cre DNA recombinase-mediated inducible gene product expression (Arai, T. *et al*., J. Virol. 72: 1115-1121 (1988)). In this system, the inserted gene is expressed in a transformant carrying this plasmid, by using the method of Saito *et al*. to infect the transformant with a recombinant adenovirus (AxCANCre) expressing Cre DNA recombinase (Saito, I. *et al*., Nucl. Acid. Res. 23, 3816-3821 (1995), Arai, T. *et al*., J. Virol. 72, 1115-1121 (1998)). In the case of the SeV-F protein, the transformed cells comprising the F gene are herein referred to as LLC-MK2/F7, and cells persistently expressing the F protein after induction by AxCANCre are herein referred to as LLC-MK2/F7/A.

Reconstitution of the virus comprising the temperature-sensitive mutations was carried out as follows: LLC-MK2 cells were plated on to a 100-mm dish at 5x 10⁶ cells/dish, and then cultured for 24 hours. T7 polymerase-expressing recombinant vaccinia virus, which had been treated with psoralen and long-wavelength ultraviolet light (365 nm) for 20 minutes (PLWUV-VacT7: Fuerst, T.R. *et al*., Proc. Natl. Acad. Sci. USA 83, 8122-8126 (1986)), was infected (MOI=2) to these cells at room temperature for one hour. The cells were washed with serum-free MEM. Plasmids, pSeV18+/MtsHNtsΔF-GFP, pGEM/NP, pGEM/P, pGEM/L and pGEM/F-HN (Kato, A. *et al*., Genes Cells 1, 569-579 (1996)), were suspended in Opti-MEM (Gibco-BRL, Rockville, MD) at amounts of 12 µg, 4 µg, 2 µg, 4 µg and 4 µg/dish, respectively. SuperFect transfection reagent (Qiagen, Bothell, WA) corresponding to 1 µg DNA/5 µl was added and mixed. The resulting mixture was allowed to stand at room temperature for 15 minutes, and then added to 3 ml of Opti-MEM containing 3% FBS. This mixture was added to the cells. After being cultured for five hours, the cells were washed twice with serum-free MEM, and cultured in MEM containing 40 µg/ml cytosine β-D-arabinofuranoside (AraC: Sigma, St. Louis, MO) and 7.5 µg/ml trypsin (Gibco-BRL, Rockville, MD). After 24 hours of culture, cells persistently expressing F protein (LLC-MK2/F7/A: Li, H.-O. *et al*., J. Virology 74. 6564-6569 (2000) , WO00/70070) were overlaid at 8.5x10⁶ cells/dish. These cells were further cultured in MEM containing 40 µg/mL AraC and 7.5 µg/mL trypsin at 37°C for two days (P0). The cells were harvested and the pellet was suspended in 2 ml Opti-MEM per dish. Freeze-and-thaw treatment was repeated three times, and the lysate was directly transfected into LLC-MK2/F7/A. The cells were cultured in serum-free MEM containing 40 µg/mL AraC and 7.5 µg/mL trypsin at 32°C (P1). After five to seven days, part of the culture supernatant was infected into freshly prepared LLC-MK2/F7/A, and the cells were cultured in the same serum-free MEM containing 40 µg/mL AraC and 7.5 µg/mL trypsin at 32°C (P2). After three to five days, freshly prepared LLC-MK2/F7/A were infected again, and the cells were cultured in serum-free MEM containing only 7.5 µg/mL trypsin at 32°C for three to five days (P3). BSA was added to the recovered culture supernatant at a final concentration of 1%, and the mixture was stored at -80°C. The viral solution stored was thawed and used in subsequent experiments.

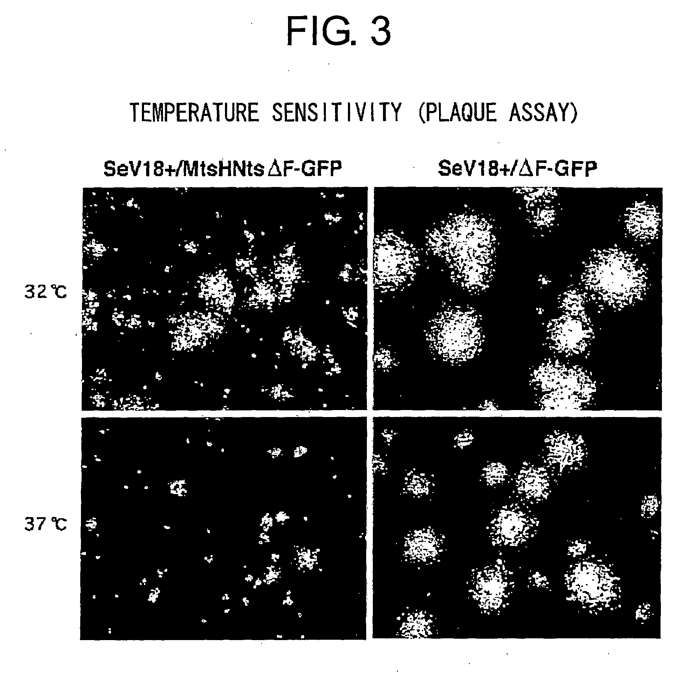

The titers of viral solutions prepared by this method were as follows: SeV18+/ΔF-GFP, 3x 10⁸; SeV18+/MtsHNtsΔF-GFP, 7x 10⁷; SeV18+SEAP/ΔF-GFP, 1.8x 10⁸; SeV18+SEAP/MtsHNtsΔF-GFP, 8.9x 10⁷ GFP-CIU/mL (GFP-CIU has been defined in WO00/70070). In determining SeV18+/ΔF-GFP and SeV18+/MtsHNtsΔF-GFP titers, the post-infection spread of plaques of cells persistently expressing F protein (LLC-MK2/F7/A) was observed at 32°C and 37°C. Fig. 3 shows photographs of patterns observed six days after infection. SeV18+/MtsHNtsΔF-GFP plaques spread to some extent at 32°C, but were greatly reduced at 37°C. This suggests that virion formation is reduced at 37°C.

### [Example 3] Effect of culture temperature (32°C) on viral reconstitution

In the experimental reconstitution of viruses in which temperature-sensitive mutations had been introduced (Example 2) , P1 and all subsequent cultures were carried out at 32°C. This temperature was used because the reference virus, used for assessing the introduction of temperature-sensitive mutations, grows well at 32°C (Kondo, T. *et al*., J. Biol. Chem. 268: 21924-21930 (1993), Thompson, S.D. *et al*., Virology 160: 1-8 (1987)). Close examination of the experimental conditions revealed that, for SeV reconstitution (and for other viruses in addition to those in which temperature-sensitive mutations had been introduced), reconstitution efficiency was improved by carrying out P1 and subsequent cultures at 32°C, giving a high possibility of recovering viruses that were previously difficult to obtain.

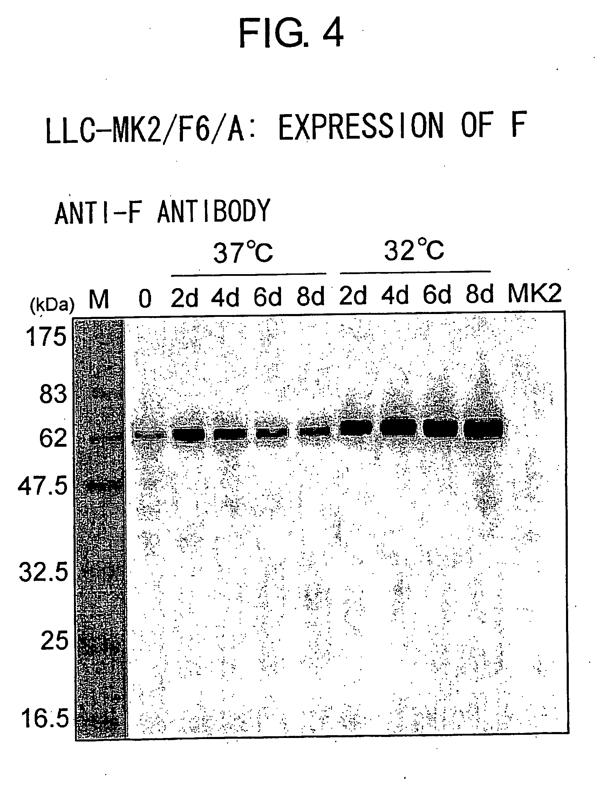

There are though to be two reasons for enhanced reconstitution efficiency at 32°C. The first point is that, when cultured at 32°C as opposed to 37°C, cytotoxicity due to AraC, which is supplemented to inhibit vaccinia virus amplification, is thought to be suppressed. Under conditions for viral reconstitution, culturing LLC-MK2/F7/A cells at 37°C, in serum-free MEM containing 40 µg/ml of AraC and 7.5 µg/ml of trypsin, caused cell damage after three to four days, including an increase in detached cells. However, cultures at 32°C could be sufficiently continued for seven to ten days with cells still intact. When reconstituting SeV with inefficient transcription and/or replication, or with inefficient formation of infectious virions, success is thought to be a direct reflection of culture duration. The second point is that F protein expression is maintained in LLC-MK2/F7/A cells when the cells are cultured at 32°C. After culturing LLC-MK2/F7/A cells that continuously express F protein to confluency on 6-well culture plates in MEM containing 10% FBS and at 37°C, the medium was replaced with a serum-free MEM containing 7.5 µg/ml of trypsin, and the cells were further cultured at 32°C or 37°C. Cells were recovered over time using a cell scraper, and Western-blotting using an anti-F protein antibody (mouse monoclonal) was used to semi-quantitatively analyze intra-cellular F protein. F protein expression was maintained for two days at 37°C, and then decreased. However, at 32°C expression was maintained for at least eight days (Fig. 4). These results confirm the validity of viral reconstitution at 32°C (after P1 stage).

The above-described Western-blotting was carried out using the following method: Cells recovered from one well of a 6-well plate were stored at -80°C, then thawed in 100 µl of 1x diluted sample buffer for SDS-PAGE (Red Loading Buffer Pack; New England Biolabs, Beverly, MA). Samples were then heated at 98°C for ten minutes, centrifuged, and a 10-µl aliquot of the supernatant was loaded on to SDS-PAGE gel (multigel 10/20; Daiichi Pure Chemicals Co., Ltd., Tokyo, Japan). After electrophoresis at 15 mA for 2.5 hours, proteins were transferred on to a PVDF membrane (Immobilon PVDF transfer membrane; Millipore, Bedford, MA) using semi-dry method at 100 mA for one hour. The transfer membrane was immersed in a blocking solution (Block Ace; Snow Brand Milk Products Co., Ltd., Sapporo, Japan) at 4°C for one hour or more, soaked in a primary antibody solution containing 10% Block Ace supplemented with 1/1000 volume of the anti-F protein antibody, and then allowed to stand at 4°C overnight. After washing three times with TBS containing 0.05% Tween 20 (TBST) , and a further three times with TBS, the membrane was immersed in a secondary antibody solution containing 10% Block Ace supplemented with 1/5000 volume of the anti-mouse IgG + IgM antibody bound with HRP (Goat F(ab')2 Anti-Mouse IgG + IgM, HRP; BioSource Int., Camarillo, CA). Samples were then stirred at room temperature for one hour. The membrane was washed three times with TBST, and three times with TBS, and proteins on the membrane were then detected using the chemiluminescence method (ECL western blotting detection reagents; Amersham Pharmacia biotech, Uppsala, Sweden).

### [Example 4] Quantification of secondarily released particles from temperature sensitive mutation-introduced viruses (HA assay, Western-Blotting)

Levels of secondarily released particles were compared, together with SeV18+/ΔF-GFP and SeV18+/MtsHNtsΔF-GFP, using the autonomously replicating type SeV, that comprises all of the viral proteins and the GFP fragment (780 bp) which comprises the termination signal-intervening sequence-initiation signal downstream of the GFP gene at the NotI site (SeV18+GFP: Fig. 2).

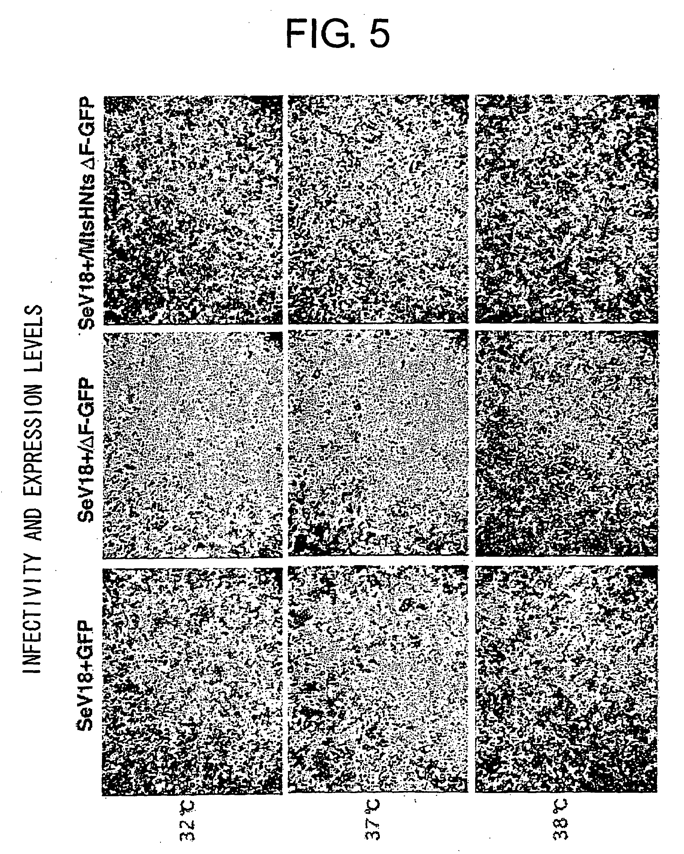

LLC-MK2 cells were grown to confluency on 6-well plates. To these cells were added 3x10⁷ CIU/ml of each virus solution at 100 µl per well (MOI=3), and the cells were infected for one hour. After washing the cells with MEM, serum-free MEM (1 ml) was added to each well, and the cells were cultured at 32°C, 37°C and 38°C, respectively. Sampling was carried out every day, and immediately after sampling, 1 ml of fresh serum-free MEM was added to the remaining cells. Culturing and sampling were performed over time. Three days after infection, observation of GFP expression under a fluorescence microscope indicated that infection levels were almost equal for the three types of virus for all temperature conditions (32°C, 37°C and 38°C), and that GFP expression was similar (Fig. 5).

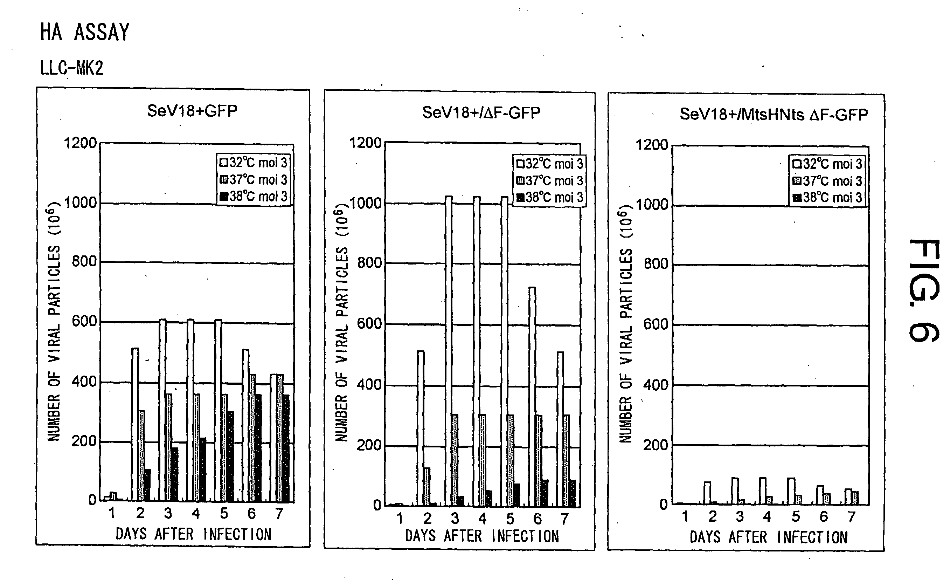

Secondarily released particles were quantified using an assay of hemagglutination activity (HA activity), performed according to the method of Kato *et al*. (Kato, A., *et al*., Genes Cell 1, 569-579 (1996)). Specifically, round-bottomed 96 well-plates were used for the serial dilution of the viral solution with PBS. Serial two-fold 50 µl dilutions were carried out in each well. 50 µl of preserved chicken blood (Cosmo Bio, Tokyo, Japan) , diluted to 1% with PBS, was added to 50 µl of the viral solution, and the mixture was allowed to stand at 4°C for one hour. Erythrocyte agglutination was then examined. The highest virus dilution rate among the agglutinated samples was judged to be the HA activity. In addition, one hemagglutination unit (HAU) was calculated to be 1x10⁶ viruses, and expressed as a number of viruses (Fig. 6). The secondarily released particles of SeV18+/MtsHNtsΔF-GFP remarkably decreased, and at 37°C, was judged to be about 1/10 the level of SeV18+/ΔF-GFP. SeV18+/MtsHNtsΔF-GFP viral particle formation was also reduced at 32°C, and although only a few particles were produced, a certain degree of production was still thought possible.

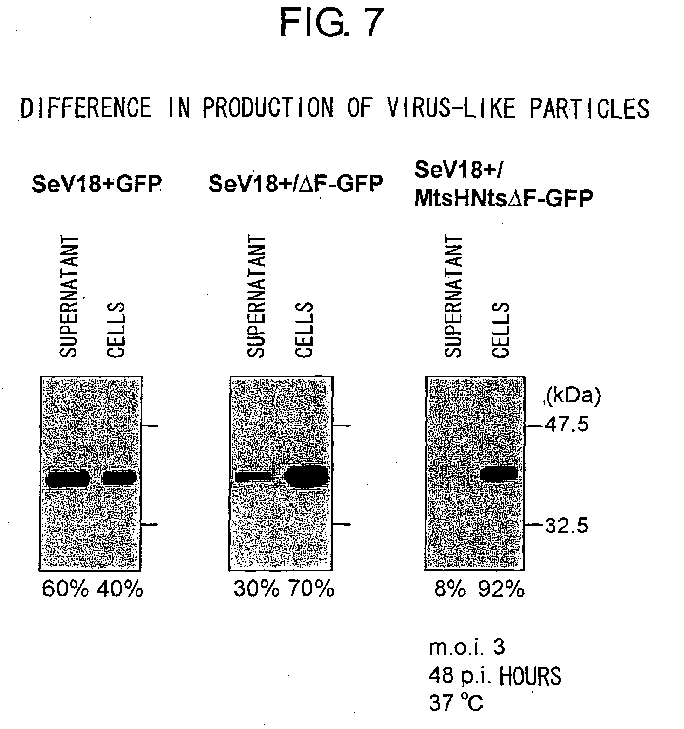

Western-Blotting was used to quantify secondarily released particles. In a manner similar to that described above, LLC-MK2 cells were infected at MOI=3 with the virus, and the culture supernatant and cells were recovered two days after infection. The culture supernatant was centrifuged at 48,000 g for 45 minutes to recover the viral proteins. After SDS-PAGE, Western-Blotting was performed to detect these proteins using an anti-M protein antibody. This anti-M protein antibody is a newly prepared polyclonal antibody, prepared from the serum of rabbits immunized with a mixture of three synthetic peptides: corresponding to amino acids 1-13 (MADIYRFPKFSYE+Cys/SEQ ID NO: 14), 23-35 (LRTGPDKKAIPH+Cys/SEQ ID NO: 15), and 336-348 (Cys+NVVAKNIGRIRKL/SEQ ID NO: 16) of the SeV M protein. Western-Blotting was performed according to the method described in Example 3, in which the primary antibody, anti-M protein antibody', was used at a 1/4000 dilution, and the secondary antibody, anti-rabbit IgG antibody bound with HRP (Anti-rabbit IgG (Goat) H+L conj.; ICN P., Aurola, OH), was used at a 1/5000 dilution. In the case of SeV18+/MtsHNtsΔF-GFP infected cells, M proteins were widely expressed to a similar degree, but viral proteins were reduced (Fig. 7). Western-blotting also confirmed a decrease in secondarily released viral particles.

### [Example 5] The expression level of genes comprised by the virus in which the temperature-sensitive mutations have been introduced (SEAP assay)

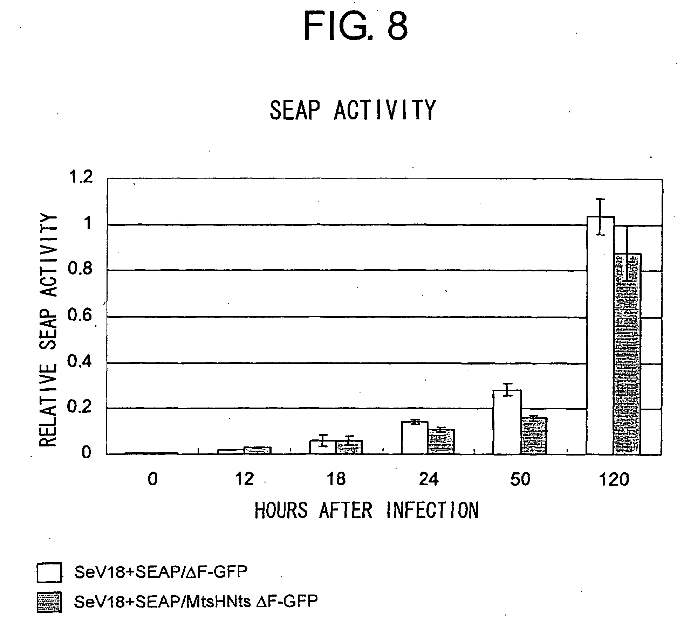

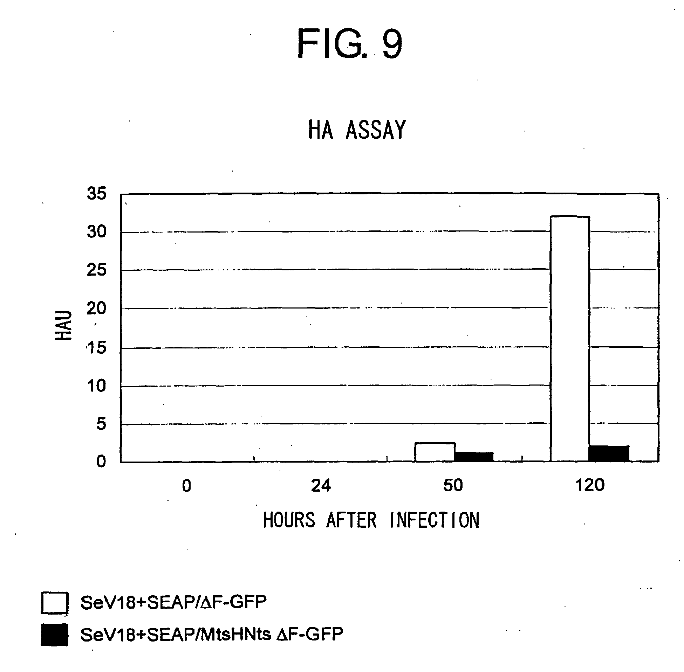

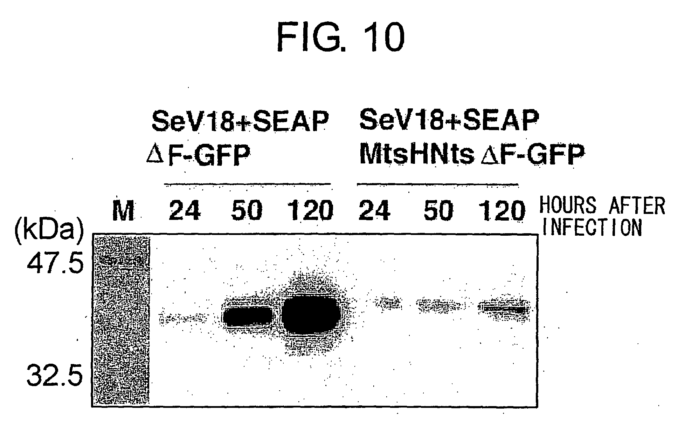

SeV18+/MtsHNtsΔF-GFP secondary particle release was reduced. However, such a modification would be meaningless in a gene expression vector if accompanied with a simultaneous decrease in comprised gene expression. Thus, gene expression level was evaluated. LLC-MK2 cells were infected with SeV18+SEAP/ΔF-GFP or SeV18+SEAP/MtsHNtsΔF-GFP at MOI=3, and culture supernatant was collected over time (12, 18, 24, 50 and 120 hours after infection). SEAP activity in the supernatant was assayed using a Reporter Assay Kit-SEAP (TOYOBO, Osaka, Japan) according to kit method. SEAP activity was comparable for both types (Fig. 8). The same samples were also assayed for hemagglutination activity (HA activity). The HA activity of SeV18+SEAP/MtsHNtsΔF-GFP was reduced to about one tenth (Fig. 9). Viral proteins were harvested from viruses in the samples by centrifugation at 48,000 g for 45 minutes, and then semi-quantitatively analyzed by Western-Blotting using an anti-M antibody. The level of viral protein in the supernatant was also reduced (Fig. 10). These findings indicate that the introduction of temperature-sensitive mutations reduced the level of secondary particle release to about 1/10, with virtually no reduction in the expression of comprised genes.

### [Example 6] Cytotoxicity of viruses in which temperature-sensitive mutations have been introduced (LDH assay)

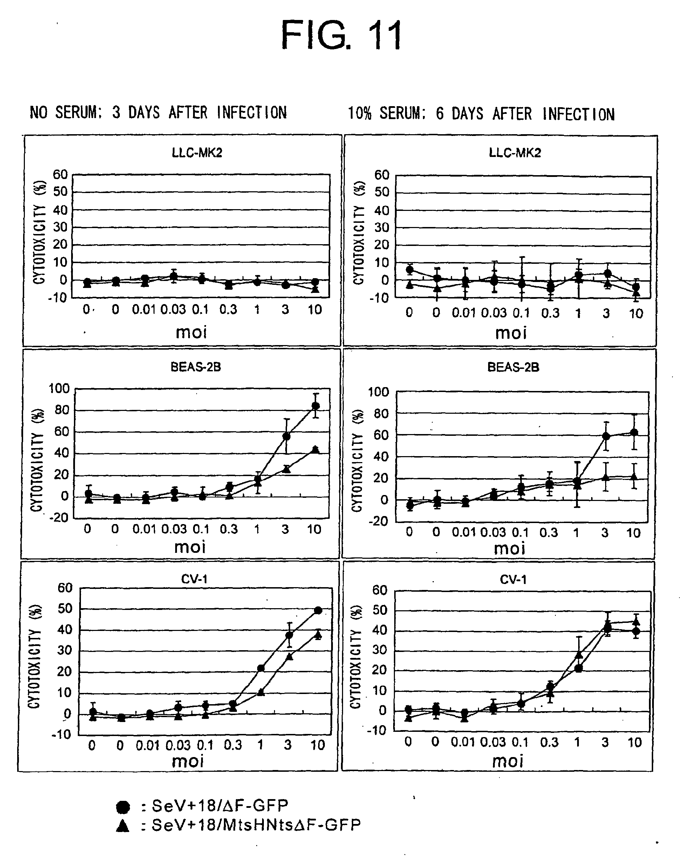

SeV infection is often cytotoxic. The influence of introduced mutations was thus examined from this respect. LLC-MK2, BEAS-2B and CV-1 cells were each plated on a 96-well plate at 2.5x10⁴ cells/well (100 µL/well), and then cultured. LLC-MK2 and CV-1 were cultured in MEM containing 10% FBS, and BEAS-2B was cultured in a 1:1 mixed medium of D-MEM and RPMI (Gibco-BRL, Rockville, MD) containing 10% FBS. After 24 hours of culture, virus infection was carried out by adding 5 µL/well of a solution of SeV18+/ΔF-GFP or SeV18+/MtsHNtsΔF-GFP diluted with MEM containing 1% BSA. After six hours, the medium containing the viral solution was removed, and replaced with the corresponding fresh medium, with or without 10% FBS. The culture supernatant was sampled three days after infection when FBS-free medium was used, or six days after infection when medium containing FBS was used. Cytotoxicity was analyzed using a Cytotoxicity Detection Kit (Roche, Basel, Switzerland) according to kit instructions. Neither of the viral vectors was cytotoxic in LLC-MK2. Further, SeV18+/MtsHNtsΔF-GFP cytotoxicity was assessed as being comparable to or lower than that of SeV18+/ΔF-GFP in CV-1 and BEAS-2B (Fig. 11). Thus, it was concluded that cytotoxicity was not induced by suppressing secondary particle release by the introduction of temperature-sensitive mutations.

### [Example 7] Study of the mechanism of secondary particle release suppression

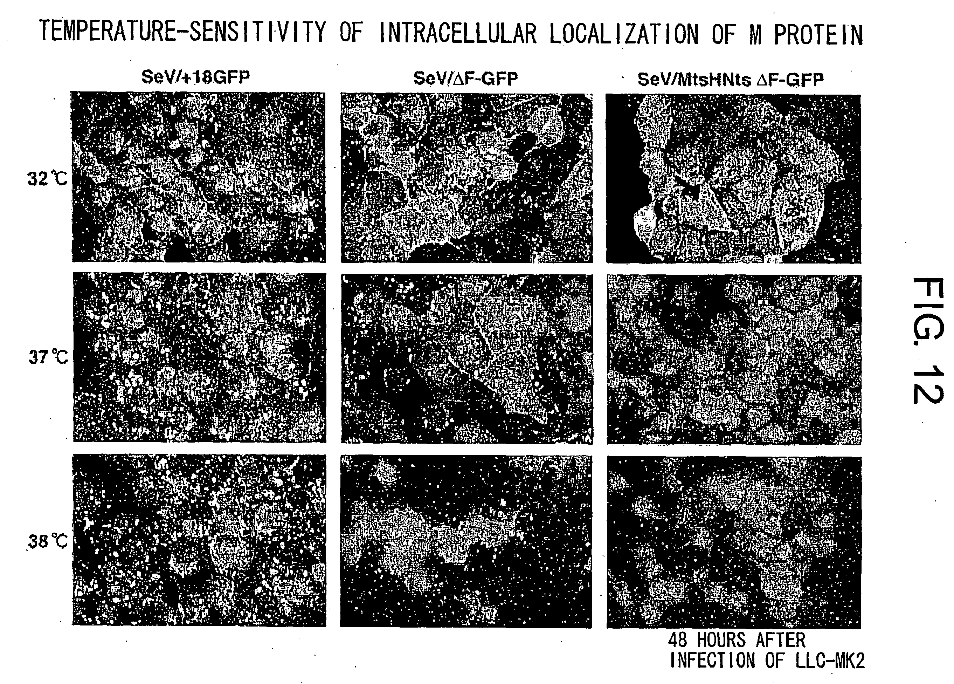

In order to elucidate part of the mechanism underlying the suppression of secondary particle release by the introduction of temperature-sensitive mutations, the subcellular localization of M protein was examined. LLC-MK2 cells were infected with each type of SeV (SeV18+GFP, SeV18+/ΔF-GFP, SeV18+/MtsHNtsΔF-GFP), and cultured at 32°C, 37°C or 38°C for two days. The cells were immunostained by using an anti-M antibody. Immunostaining was performed as follows: The cultured cells were washed once with PBS, methanol cooled to -20°C was added, and the cells were fixed at 4°C for 15 minutes. After washing the cells three times with PBS, blocking was carried out at room temperature for one hour using PBS solution containing 2% goat serum and 0.1% Triton. After washing with PBS a further three times, the cells were reacted with a primary antibody solution (10 µg/mL anti-M antibody) containing 2% goat serum at 37°C for 30 minutes. After washing three times with PBS, the cells were reacted with a secondary antibody solution (10 µg/mL Alexa Fluor 488 goat anti-rabbit IgG (H+L) conjugate: Molecular Probes, Eugene, OR) containing 2% goat serum at 37°C for 15 minutes. Finally, after a further three washes with PBS, the cells were observed under a fluorescence microscope. In the case of the self-replicating SeV18+GFP comprising both F and HN proteins, condensed M protein was detectable on cell surfaces at all of the temperatures tested (Fig. 12). Such M protein condensation has been previously reported (Yoshida, T. *et al*., Virology 71: 143-161 (1976)), and is assumed to reflect the site of virion formation. Specifically, in the case of SeV18+GFP, cell-surface M protein localization appeared to be normal at all temperatures, suggesting that a sufficient amount of virions were formed. On the other hand, in the case of SeV18+/ΔF-GFP, M protein condensation was greatly reduced at 38°C. M protein is believed to localize on cell surfaces, binding to both F and HN protein cytoplasmic tails (Sanderson, C.M. *et al*., J. Virology 68: 69-76 (1994), Ali, A. *et al*., Virology 276: 289-303 (2000)). Because one of these two proteins, namely the F protein, is deleted in SeV18+/ΔF-GFP, F protein deficiency is assumed to have an impact on M protein localization. This impact was expected to be stronger for SeV18+/MtsHNtsΔF-GFP, and it was also expected that, even at 37°C, M protein localization would be disturbed and the number of particles in the secondary release would be reduced.

### [Example 8] Study of the suppression mechanism of secondary particle release (2)

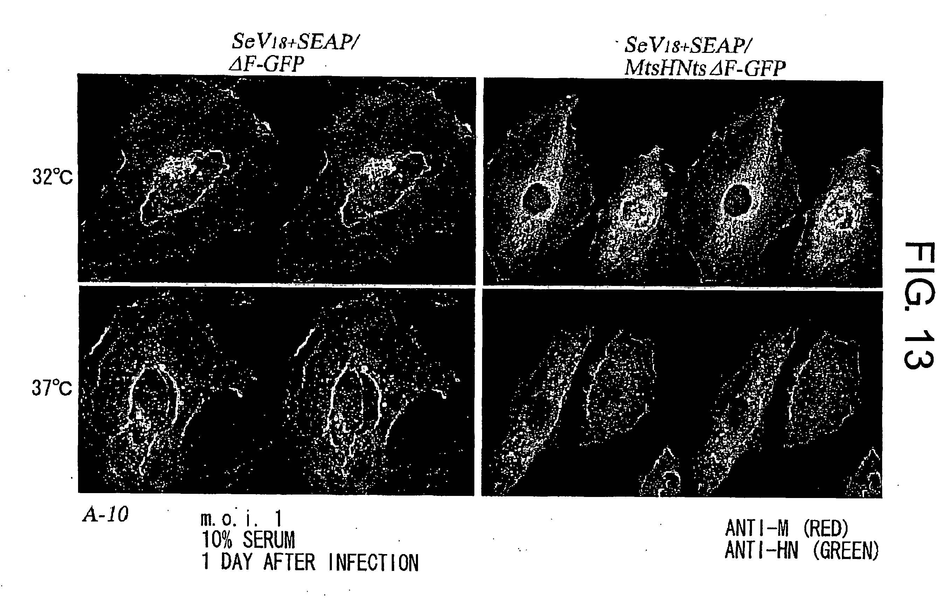

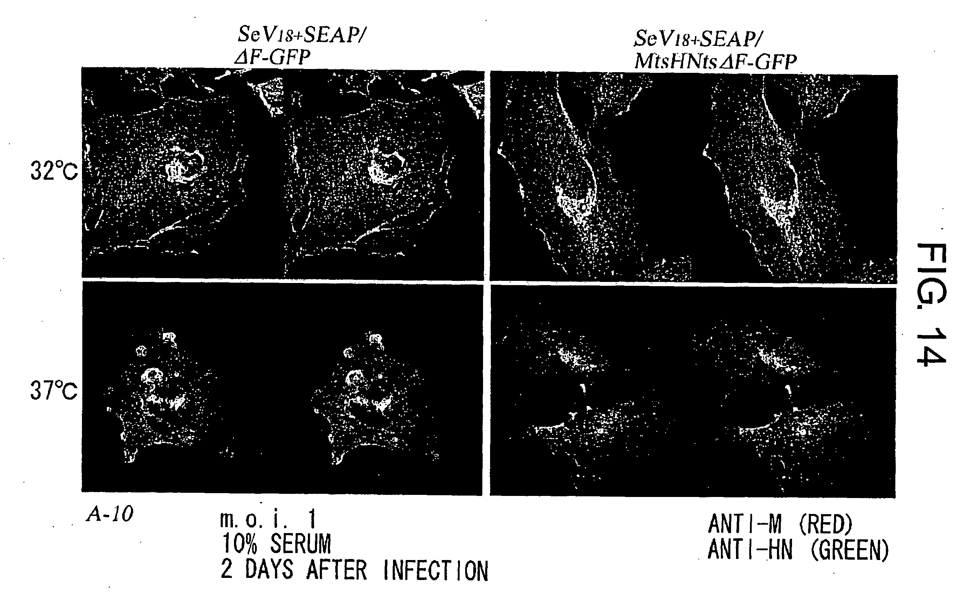

In order to study the SeV protein's subcellular localization in more detail, analyses were carried out using a confocal laser microscope (MRC1024; Bio-Rad Laboratories Inc., Hercules, CA). A-10 cells (rat myoblasts) were infected with each of SeV18+SEAP/ΔF-GFP and SeV18+SEAP/MtsHNtsΔF-GFP (MOI=1), and then cultured in MEM containing 10% serum at 32°C or 37°C. One or two days later, the cells were immunostained using anti-M antibody and anti-HN antibody. Immunostaining was performed as follows: The infected culture cells were washed once with PBS. Methanol cooled to -20°C was added to the cells, and the cells were fixed at 4°C for 15 minutes. The cells were washed three times with PBS, and blocking was then carried out for one hour at room temperature, using PBS solution containing 2% goat serum, 1% BSA and 0.1% Triton. The cells were reacted with M primary antibody solution (10 µg/mL anti-M antibody) containing 2% goat serum at 37°C for 30 minutes. The cells were then reacted with HN primary antibody solution (1 µg/mL anti-HN antibody (IL4-1)) at 37°C for 30 minutes. After washing three times with PBS, the cells were reacted with a secondary antibody solution (10 µg/mL Alexa Fluor 568 goat anti-rabbit IgG(H+L) conjugate and 10 µg/mL Alexa Fluor 488 goat anti-mouse IgG(H+L) conjugate: Molecular Probes, Eugene, OR) containing 2% goat serum at 37 °C for 15 minutes. The cells were washed three times with PBS and The nuclei were stained with TO_PRO3 (Molecular Probes, Eugene, OR) diluted 4000 times. The cells were allowed to stand at room temperature for 15 minutes. Finally, to prevent quenching, a Slow Fade Antifade Kit solution (Molecular Probes, Eugene, OR) was substituted for the liquid, and the cells were observed under a confocal laser microscope. Fig. 13 shows the results one day after infection. Red represents M protein localization; green, HN protein localization; and yellow, co-localization of the two. Far red has been subjected to color conversion, and thus blue represents the nucleus. In the case of SeV18+SEAP/ΔF-GFP, each protein's localization pattern did not differ largely between 32°C and 37°C, and cell-surface localization of M protein and HN protein was observed. On the other hand, localization of each protein for SeV18+SEAP/MtsHNtsΔF-GFP was different at both temperatures from that for SeV18+SEAP/ΔF-GFP, and hardly any M protein was localized on the cell surface. At 37°C in particular, the M protein and HN protein were almost completely separated, such that the M protein was localized at sites presumed to be close to the central body of microtubules (i.e., near the Golgi body). A similar result was obtained for cells cultured two days after infection. Particularly in SeV18+SEAP/MtsHNtsΔF-GFP-infected cells, subcellular M protein localization did not change between one day and two days after infection (Fig. 14) , and protein transport appeared to have stopped. This result also showed that the reduced secondary particle release of viruses in which temperature-sensitive mutations had been introduced was caused by a deficiency in localization of the M protein, which is expected to play a central role in particle formation.

When the cells were cultured at 32°C after infection with SeV18+SEAP/MtsHNtsΔF-GFP, the M protein stained in a morphology similar to that of a microtubule (Fig. 13). To show the involvement of microtubules, a reagent that enhances microtubule depolymerization was added, and changes in M protein (and HN protein) localization were then studied. A-10 cells were infected with SeV18+SEAP/MtsHNtsΔF-GFP at MOI=1, and a depolymerization reagent, colchicine (Nakarai Tesque, Kyoto, Japan) or colcemid (Nakarai Tesque, Kyoto, Japan), was immediately added at a final concentration of 1 mM. The cells were then cultured at 32°C. Two days after infection, the subcellular localizations of the M and HN proteins were observed by the same method as described above. In the absence of the depolymerization reagent, M protein distribution was. similar in morphology to a microtubule (Fig. 13). However, addition of the depolymerization reagent resulted in disruption of this structure, and M protein was detected as a large fibrous structure (Fig. 15). This structure may be an aggregate of M protein by itself, or M protein bound to the residues of depolymerized microtubules. In either case, as seen in Fig. 13, it was plausibly judged that M protein was localized in microtubules in cells cultured at 32°C after infection with SeV18+SEAP/MtsHNtsΔF-GFP.

In order to clarify whether or not the above-mentioned localization of M protein in microtubules was characteristic of temperature-sensitive viruses, the post-infection influence of the microtubule depolymerization reagent (colchicine) on changes to M protein(and HN protein) localization was evaluated for both viruses SeV18+/ΔF-GFP and SeV18+/MtsHNtsΔF-GFP. A-10 cells were infected with SeV18+/ΔF-GFP or SeV18+/MtsHNtsΔF-GFP at MOI=1, and the depolymerization reagent colchicine was immediately added at a final concentration of 1 µM. The cells were cultured at 32°C or 37°C. Two days after infection, the subcellular localization of M protein (and HN protein) was observed using the same method as described above. The results are shown in Fig. 16. Infected cells exhibited similar features for both viruses. Specifically, when the cells were cultured at 32°C after infection, M protein was observed as a large fibrous structure, similar to that in Fig. 15. M protein's coexistance with microtubules was also suggested for SeV18+/ΔF-GFP. In particular, in cells infected with SeV18+/MtsHNtsΔF-GFP and cultured at 37°C, M protein was observed to be localized in areas supposed to be near the Golgi body.

Based on the above results, the following can be inferred: M protein is synthesized near the Golgi body; it is transported around the cell along microtubules (for example, bound to a motor protein such as kinesin) , mainly bound to the cytoplasmic tails of F and HN proteins (Sanderson, C.M. *et al*., J. Virology 68: 69-76 (1994) ; Ali, A. *et al*., Virology 276: 289-303 (2000)); and the M protein is localized on the cell surface, followed by particle formation. In viruses comprising a temperature-sensitive mutation, everything up to the point of intracellular transport along microtubules may be normal at 32°C. However, translocation from microtubules to the cell surface may be hindered, resulting in localization along microtubules. At 37°C it can be assumed that even intracellular transport along microtubules may be hindered, and thus, localization in the vicinity of the Golgi body is observed. M protein synthesis is supposed to take place near the Golgi body. However, it is possible that M protein aggregation is observed at these sites, and that the area of synthesis itself is elsewhere. However, it has been reported that tubulin, a microtubule component, activates and is involved in SeV transcription and replication (Moyer, S.A. *et al*., Proc. Natl. Acad. Sci. U.S.A. 83: 5405-5409 (1986); and Ogino, T. *et al*., J. Biol. Chem. 274: 35999-36008 (1999)). Moreover, as the Golgi body is located near the central body, where tubulin is predicted to exist in abundance, the Golgi body can be synthesized close to the microtubule central body (i.e., near the Golgi body). In addition, although the SeV mutant strain, F1-R, comprises a mutation in its M gene, it modifies microtubules after infecting cells, and this modification may enable particle formation independent of F1-R strain cell polarity (Tashiro, M. *et al*., J. Virol. 67, 5902-5910 (1993)). In other words, the results obtained in the present Example may also be interpreted by assuming the intracellular transport of M protein along tubulin. In this supposed mechanism, introduction of temperature-sensitive mutations to the M and HN genes may result in deficient subcellular M protein localization, resulting in a reduction in secondary particle release.

### [Example 9] Construction of genome cDNA of F-deficient SeV comprising introduced mutations in the P and L genes

To suppress secondary particle release (and reduce cytotoxicity) by further introduction of mutations to the F-deficient SeV vector, modifications were made based on a gene structure identified in a SeV with maintained infectivity (Bossow, *S. et al*., Negative Strand Viruses. p. 157 (2000)). The actual design was carried out in the following two patterns: Each pattern was identified by the analysis of the above SeV with maintained infectivity. Mutations were introduced at one site in the P gene (different for the two patterns) and at two sites in the L gene (the same for both patterns). Specifically, the two patterns were P (E86K) and L(N1197S/K1795E); and P (L511F) and L(N1197S/K1795E). The mutant strains identified as comprising these mutations have been reported as having decreased transcription activity (1/4 to 1/8 of a control) and decreased replication activity (1/2 to 1/3 of a control), and virus release is also reported to be reduced (to about 1%) (Bossow, S. *et al*., Negative Strand Viruses. p. 157 (2000)).

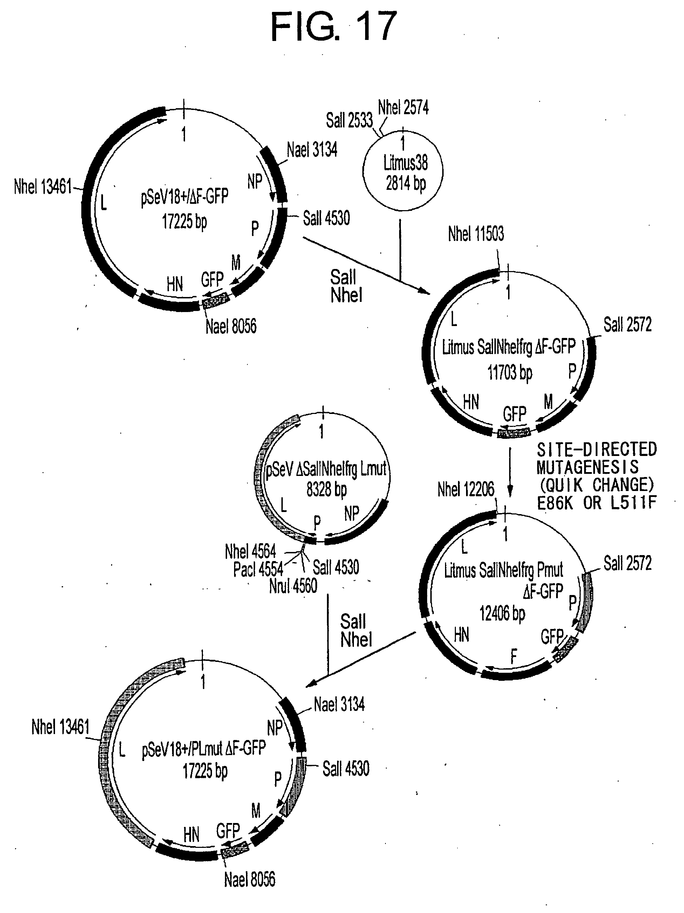

The scheme of mutation introduction is shown in Fig. 17. SalI and NheI were used to digest the full-length genome cDNA of the F-deficient Sendai virus comprising the GFP gene at the F-deficient site (pSeV18+/ΔF-GFP; Li, H.-O. *et al*., J. Virology 74: 6564-6569 (2000) ; WO00/70070). The NP gene-comprising fragment (8294 bp) was recovered, and a multicloning site was introduced using synthetic oligo DNAs (pSeV/ΔSalINheIfrg-MCS construction). The synthetic oligonucleotide sequences used to introduce the multicloning site were 5'-tcgacaccaggtatttaaattaattaatcgcgag-3' (SEQ ID NO: 17) and 5'-ctagctcgcgattaattaatttaaatacctggtg-3' (SEQ ID NO: 18). Mutations were introduced to the L gene using the constructed pSeV/ΔSalINheIfrg-MCS. Introduction was carried out according to mutagenesis kit instructions (QuikChange™ Site-Directed Mutagenesis Kit (Stratagene, La Jolla, CA)). The synthetic oligonucleotide sequences used to introduce mutation N1197S in the L gene were 5'-gttctatcttcctgacTCtatagacctggacacgcttac-3' (SEQ ID NO: 19) and 5'-gtaagcgtgtccaggtctataGAgtcaggaagatagaac-3' (SEQ ID NO: 20). The synthetic oligonucleotide sequences used to introduce mutation K1795E were 5'-ctacctattgagccccttagttgacGaAgataaagataggcta-3' (SEQ ID NO: 21) and 5'-tagcctatctttatcTtCgtcaactaaggggctcaataggtag-3' (SEQ ID NO: 22). The introduction of a mutation to the P gene was carried out using LitmusSalI/NheIfrg ΔF-GFP, prepared by ligating the P gene-comprising fragment (8931 bp), obtained by the digestion of pSeV18+/ ΔF-GFP by SalI/NheI, to the same Litmus98 site. The synthetic oligonucleotide sequences used to introduce mutation E86K in the P gene were 5'-caagataatcgatcaggtAaAgagagtagagtctctgggag-3' (SEQ ID NO: 23) and 5'-ctcccagagactctactctcTtTacctgatcgattatcttg-3' (SEQ ID NO: 24). The synthetic oligonucleotide sequences used to introduce mutation L511F were 5'-ctcaaacgcatcacgtctcTtTccctccaaagagaagc-3' (SEQ ID NO: 25) and 5'-gcttctctttggagggAaAgagacgtgatgcgtttgag-3' (SEQ ID NO: 26). After the introduction of these mutations, the following fragments were ligated: the 8294 bp fragment obtained by digesting the plasmids comprising a single P gene mutation (LitmusSalI/NheIfrg ΔF-GFP) with SalI/NheI; and the L gene-comprising fragment (8931 bp) obtained by digesting the plasmids comprising two L gene mutations (pSeV/ΔSalINheIfrg-MCS) with SalI/NheI. Then, pSeV18+/P86Lmut ^{·} ΔF-GFP (which comprises mutation E86K in the P gene and N1197S/K1795E in the L gene) and pSeV18+/P511Lmut ^{·}ΔF-GFP (which comprises mutation L511Fin the P gene and N1197S/K1795E in the L gene) were constructed. These are collectively called pSeV18+/PLmut ^{·}ΔF-GFP.

Furthermore, to quantify the expression level of the comprised genes, the present inventors constructed a cDNA comprising the secretory alkaline phosphatase (SEAP) gene. Specifically, NotI was used to cut out an SEAP fragment (1638 bp) that comprises a termination signal-intervening sequence-initiation signal downstream of the SEAP gene (WO00/70070). The fragment was incorporated into pSeV18+/P86Lmut ^{·}ΔF-GFP and pSeV18+/P511Lmut ^{·}ΔF-GFP at the NotI site on the 18th nucleotide, creating pSeV18+SEAP/P86Lmut ^{·}ΔF-GFP and pSeV18+SEAP/P511Lmut ^{·}ΔF-GFP, respectively.

### [Example 10] Re-constitution and amplification of the F-deficient SeV that incorporates the SeV sequence for continuous infectivity in P/L.

Re-constitution of the virus was carried out according to the method described by Li *et al*. (Li, H.-O. *et al*., J. Virology 74: 6564-6569 (2000), WO00/70070). Specifically, the same procedure as that described in Example 2 of this specification was carried out. Viral solution titers prepared in this method were 4.0x10⁸ GFP-CIU/ml for SeV18+/P86Lmut ^{·}ΔF-GFP; 2.8x10⁸ GFP-CIU/ml for SeV18+/P511Lmut^{·} ΔF-GFP; 3.7x10⁸ GFP-CIU/ml for SeV18+SEAP/P86Lmut ^{·}ΔF-GFP; and 2.0x10⁸ GFP-CIU/ml for SeV18+SEAP/P511Lmut ^{·}ΔF-GFP. (GFP-CIU is defined in WO00/70070.)

### [Example 11] Quantification of secondarily released particles from the F-deficient SeV that incorporates the SeV sequence for continuous infectivity in P/L

Secondarily released particles were quantified by measuring HA activity using culture supernatant from infected cells. For purposes of comparison, SeV18+/ΔF-GFP secondarily released particles were also measured at the same time. The details of this experiment are described in Example 4 above. Briefly, 100 µl of 1x10⁷ CIU/ml (MOI=1) or 5x10⁷ CIU/ml (MOI=5) of each viral solution was added to each well of LLC-MK2 cells grown confluently on 6-well plates. Cells were then infected for one hour. The cells were washed with MEM, 1 ml of serum-free MEM was added to each well, and the cells were then cultured at 37°C. Sampling was carried out every day. Immediately after sampling, 1 ml of fresh serum-free MEM was added to each sample. Culturing and sampling were conducted at certain time intervals.

HA activity was measured according to the method of Kato *et al*. (Kato, A. *et al*., Genes Cell 1, 569-579 (1996)). Thus, PBS was used to make serial two-fold 50 µl dilutions of the viral solution, for each well of a round-bottomed 96 well-plate. 50 µl of this solution was combined with 50 µl of preserved chicken blood (Cosmo Bio Co. Ltd., Tokyo, Japan) diluted to 1% with PBS, and then allowed to stand at 4°C for one hour. Erythrocyte agglutination was examined, and HA activity was judged to be the highest dilution rate achieving hemagglutination among the agglutinated samples.

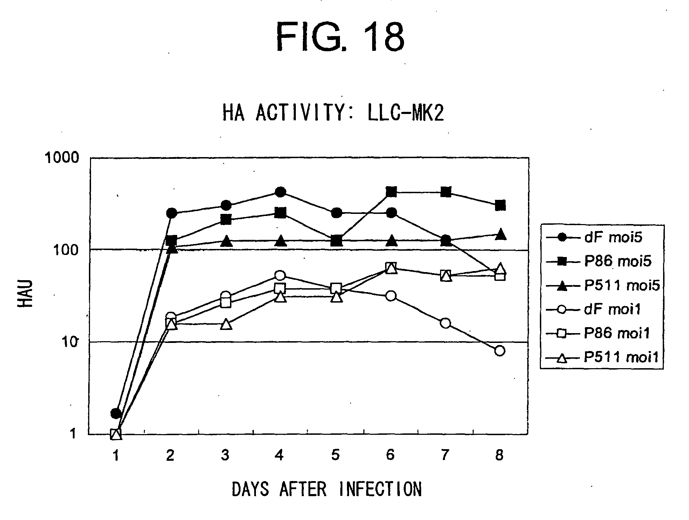

In both infections with MOI=1 and MOI=5, the levels of secondarily released particles were slightly reduced in cells infected with a vector that incorporates the SeV sequence for continuous infectivity in P and L (Fig. 18). Thus, transducing the mutation into P and L is considered to result in a slight reduction in the formation of secondarily released particles. However, the degree of inhibition was not very significant. Characteristically, the level of secondarily released particles was gradually reduced in the SeV18+/ΔF-GFP-infected cells at a later stage of the infection, while such a reduction was virtually absent in the SeV18+/P86Lmut· ΔF-GFP- and SeV18+/P511Lmut·ΔF-GFP-infected cells. These findings show that transducing the mutation into P and L reduces cytotoxicity, allowing the SeV vector to be transcribed and replicated even late in infection, thereby maintaining the formation of secondarily released particles.

### [Example 12] Cytotoxicity of the F-deficient SeV that incorporates the SeV sequence for continuous infectivity in P/L

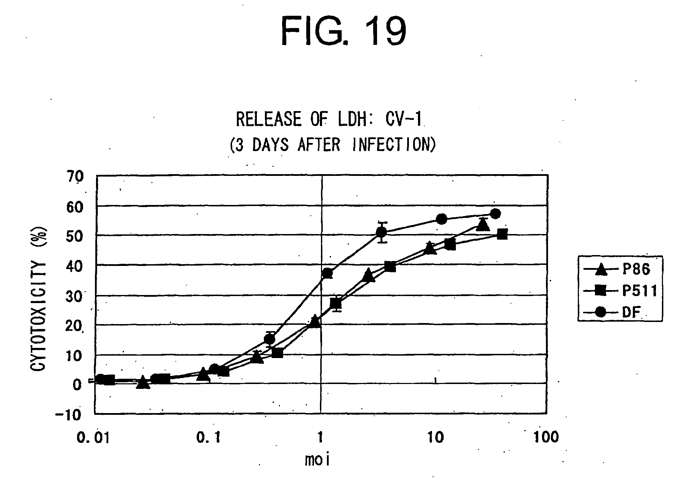

Notable SeV infection-dependent cytotoxicity can be observed in CV-1 cells, and was evaluated by utilizing these cells. F-deficient SeV without transduced mutations in P/L (SeV18+/ΔF-GFP) was employed as a control. The experimental method is detailed in Example 6. Briefly, CV-1 cells were inoculated into a 96-well plate at a density of 2.5x10⁴ cells/well (100 µL/well), and then cultured. MEM supplemented with 10% FBS was used for the culture. After culturing for 24 hours, an SeV18+/ΔF-GFP, SeV18+/P86Lmut ^{·} ΔF-GFP or SeV18+/P511Lmut^{·}ΔF-GFP solution diluted with a 1% BSA-supplemented MEM was added in a volume of 5 µL/well to affect infection. Six hours later, the viral solution-comprising medium was removed, and replaced with FBS-free MEM medium. Three days after infection, the culture supernatant was sampled, and subjected to quantification using a Cytotoxicity Detection Kit (Roche, Basel, Switzerland) according to kit instructions. SeV18+/P86Lmut^{·}ΔF-GFP and SeV18+/P511Lmut·ΔF-GFP each exhibited a marked reduction in cytotoxicity compared to SeV18+/ΔF-GFP (Fig. 19).

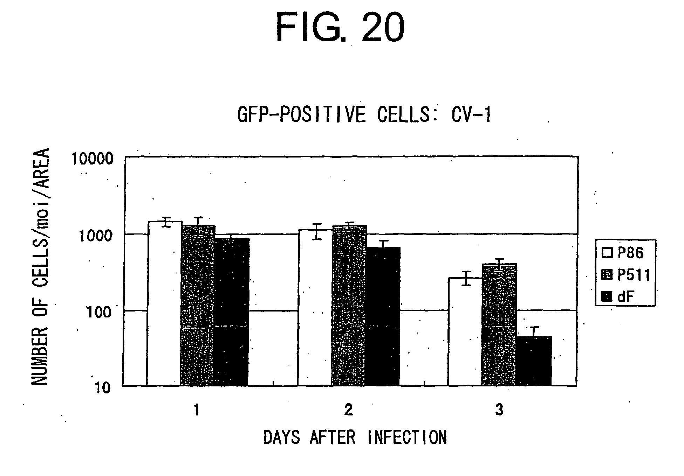

In the same experiment, GFP-positive cells were counted at certain time intervals. The number of positive cells was maintained in the CV-1 cells infected with each of the two vectors incorporating the SeV sequence for continuous infectivity in P and L (Fig. 20). CV-1 cells were susceptible to SeV infection-dependent cytotoxicity, and the infected cells readily peeled off. Cells infected with a vector which incorporated the SeV sequence for continuous infectivity in P and L, were satisfactorily maintained, strongly supporting the suggestion that these vectors reduce cytotoxicity.

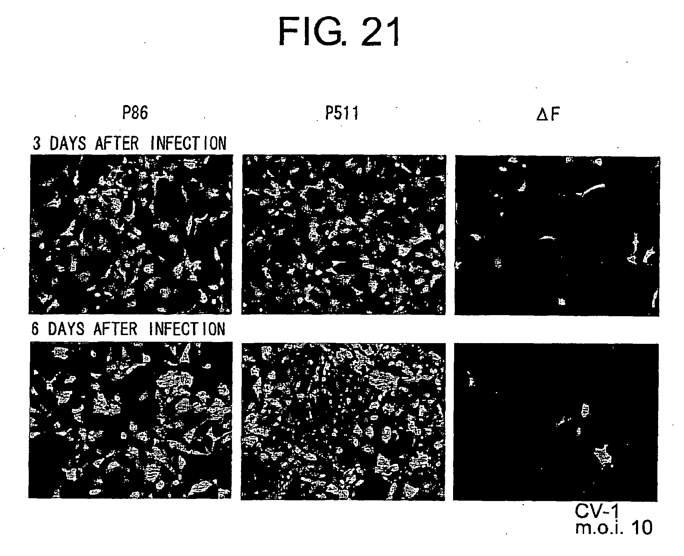

Fig. 21 shows fluorescence microscope photographs of the same infected (MOI=10) CV-1 cells, three and six days after infection. The number of cells infected with a vector incorporating the SeV sequence for continuous infectivity in P and L was. large, and satisfactory conditions could also be verified visually. The proliferation of SeV18+/P511Lmut^{·}ΔF-GFP-infected CV-1 cells was especially evident. These findings demonstrate that transducing the SeV sequence for continuous infectivity into P and L can potentially reduce SeV infection-dependent cytotoxicity.

### [Example 13] Quantification of the expression of genes carried on the F-deficient SeV incorporating the SeV sequence for continuous infectivity in P/L

The ability of SeV18+/P86Lmut ^{·} ΔF-GFP and SeV18+/P511Lmut · ΔF-GFP to reduce secondarily released particles and to reduce cytotoxicity may be attributable to reductions in transcription and replication in the initially identified mutant (reported reductions of 1/4 to 1/8, and 1/2 to 1/3 respectively: Bossow, S. *et al*., Negative Strand Viruses 2000, p.157). Thus, reduced transcription and replication may, at the same time, lead to reduced expression of a comprised gene. Such a reduction, if large, may result in the loss of an advantageous property of the SeV vector. Accordingly, the SeV vector incorporating the SeV sequence for continuous infectivity in P/L was also transduced with an SEAP gene at the +18 position, and the SEAP expression level in infected cells was measured over time.

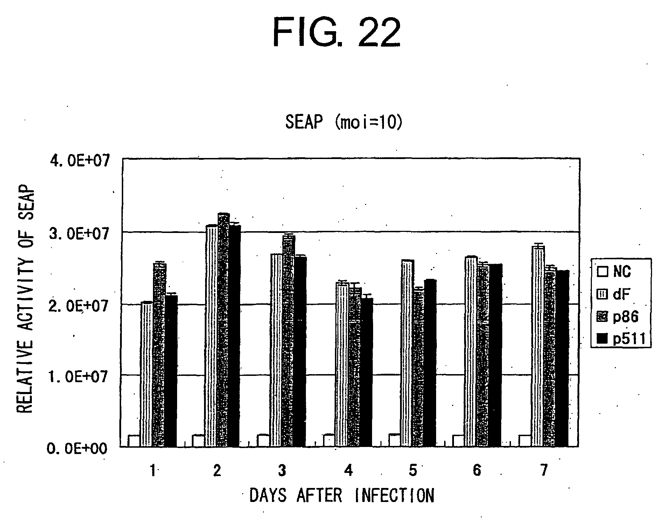

The experimental method is detailed in Example 5. Briefly, LLC-MK2 cells were infected with an SeV18+SEAP/ΔF-GFP, SeV18+SEAP/P86Lmut ^{·} ΔF-GFP or SeV18+SEAP/P511Lmut ^{·} ΔF-GFP at MOI=10, and the culture supernatant was sequentially sampled (every 24 hours) . SEAP activity was measured using a Reporter Assay Kit-SEAP (Toyobo Co., Ltd., Osaka, Japan) according to kit instructions. SEAP activity levels in all vectors were virtually the same (Fig. 22). Thus, there was virtually no reduction in expression level, even when using a vector incorporating the SeV sequence for continuous infectivity in P/L.

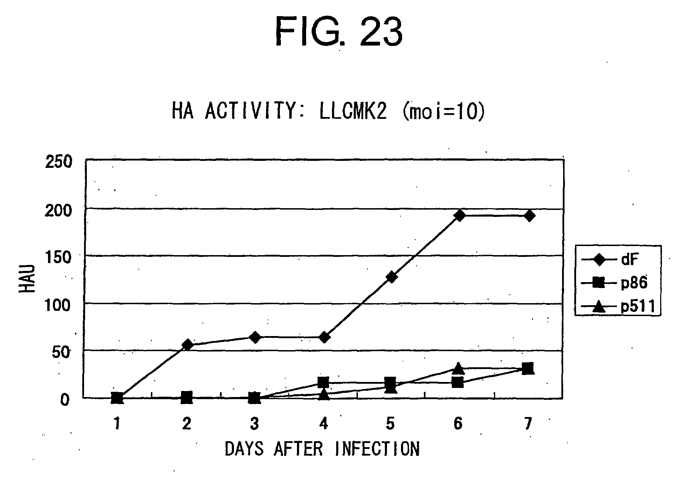

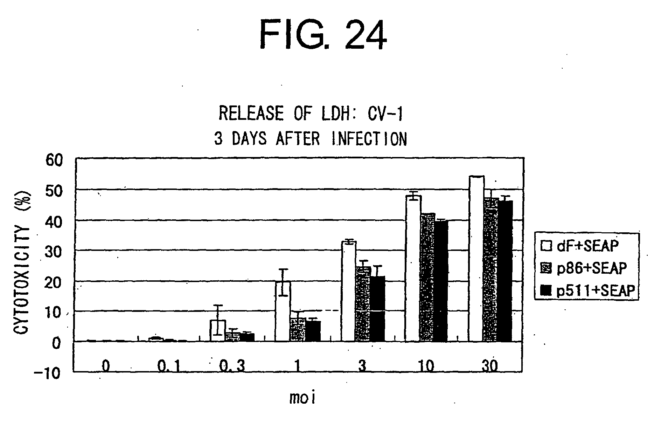

Each vector comprising an SEAP gene was also subjected to quantification of secondarily released particles from infected cells, and, as an index of cytotoxicity, quantification of LDH in the comprised cell culture supernatant. The effect of the installed SEAP gene was strongly reflected in the level of secondarily released particles. Specifically, this level was reduced to about 1/10 in both vectors incorporating the SeV sequence for continuous infectivity in P/L (Fig. 23). Transducing mutations into P/L was judged to reflect better results. Reduction in cytotoxicity was similarly observed in both transduction of the SeV sequence for continuous infectivity into P/L, and in vectors without the SEAP gene (Fig. 24).

### [Example 14] Construction of an F-deficient SeV genome cDNA comprising both a temperature sensitive mutation and a P/L mutation

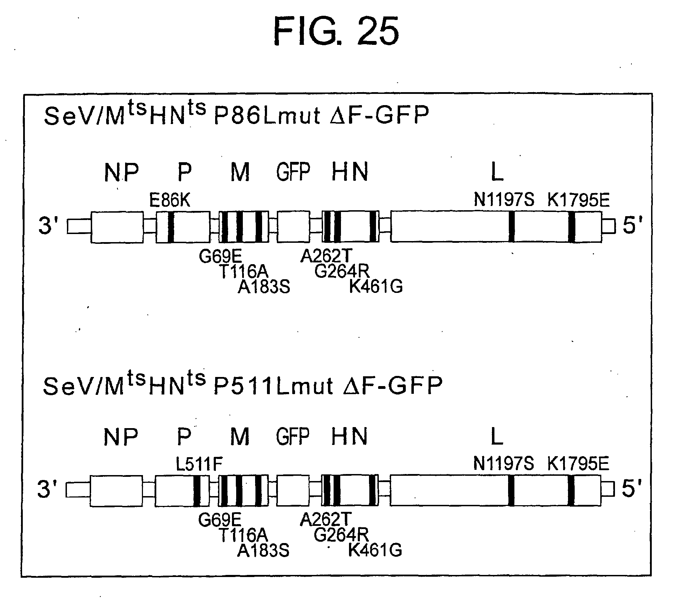

By combining the transduction of a temperature sensitive mutation into the M protein and HN protein, with the transduction of an SeV-derived continuous infectivity sequence into the P protein and L protein, the combined effect of reduction of secondarily released particles (and of cytotoxicity) may be greater than for each mutation acting alone. Accordingly, two F-deficientSeV vectors (Fig. 25: SeV18+/MtsHNtsP86Lmut^{·}ΔF-GFP, SeV18+/MtsHNtsP511Lmut^{·}ΔF-GFP) were constructed. These vectors comprised nine mutations in total, consisting of six temperature sensitive mutations (M: G69E, T116A and A183S; and HN: A262T, G264R and K461G) and three SeV-derived continuous infectivity mutations (P: E86K or L511F; and L: N1197S and K1795E).

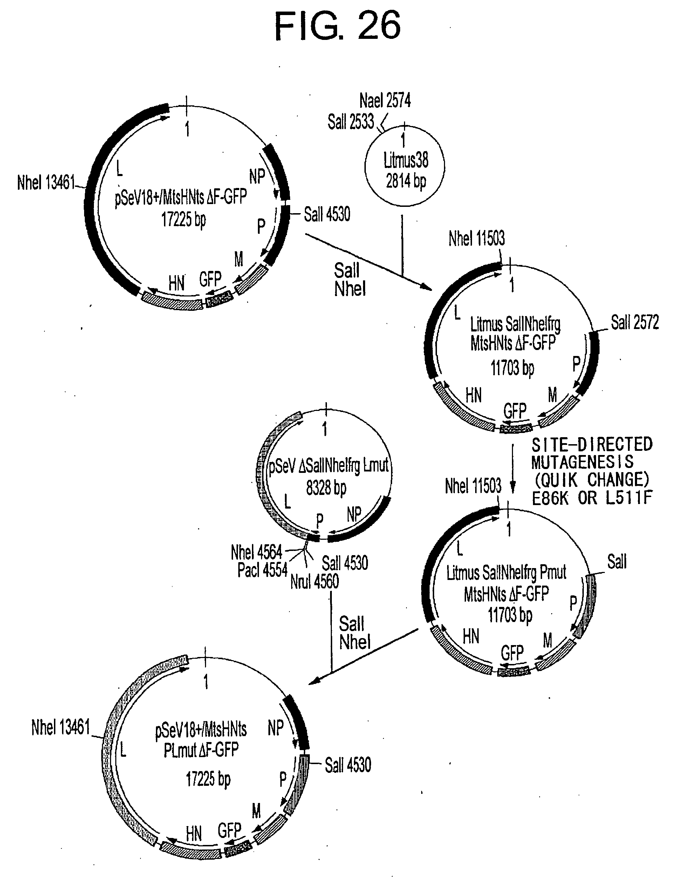

The mutation transduction scheme is shown in Fig. 26. F-deficient Sendai virus vector genome cDNA comprising temperature sensitive mutation (pSeV18+/MtsHNts ΔF-GFP: see, Example 1) was digested with SalI and NheI, and ligated to the P gene-comprising fragment (8931 bp) at the same site as Litmus38. This yielded LitmusSalI/NheIfrg MtsHNts ^{·} ΔF-GFP, which was then employed. According to the method described in Example 1, the transduction of E86K mutation into the P gene employed the synthetic oligonucleotides comprising the sequences: 5'-caagataatcgatcaggtAaAgagagtagagtctctgggag-3'/SEQ ID No: 23; and 5'-ctcccagagactctactctcTtTacctgatcgattatcttg-3'/SEQ ID No: 24. The L511F mutation transduction employed the synthetic oligonucleotides comprising the sequences: 5'-ctcaaacgcatcacgtctcTtTccctccaaagagaagc-3'/SEQ ID No: 25; and 5'-gcttctctttggagggAaAgagacgtgatgcgtttgag-3'/SEQ ID No: 26. After transducing these mutations, the plasmids (LitmusSalI/NheIfrg MtsHNts ΔF-GFP), each comprising one mutation in the P gene, were digested with SalI/NheI. The resultant 8294 bp fragment was ligated to the L gene-carrying fragment (8931 bp), which was recovered by SalI/NheI digestion of the plasmid comprising two mutations in the L gene (pSeV/ΔSalINheIfrg-MCS), constructed in Example 1. Finally, pSeV18+/MtsHNtsP86Lmut^{·}ΔF-GFP (comprising the temperature sensitive mutation, and the mutations P(E86K) and L(N1197S/K1795E)) and pSeV18+/MtsHNtsP511Lmut ^{·} ΔF-GFP (comprising the temperature sensitive mutation and the mutations P(L511F) and L(N1197S/K1795E)) (commonly designated as pSeV18+/MtsHNtsPLmut ΔF-GFP) were constructed.

A cDNA comprising the SEAP gene was constructed to measure the expression level of comprised genes. Specifically, NotI was used to cut out an 1638 bp SEAP fragment comprising a termination signal-intervening sequence-initiation signal downstream of the SEAP gene (WO00/70070). This fragment was integrated into the NotI site at the +18 position of pSeV18+/MtsHNts P86Lmut ^{·} ΔF-GFP and pSeV18+/MtsHNts P511Lmut ^{·} ΔF-GFP, which were then designated as pSeV18+SEAP/MtsHNts P86Lmut ^{·} ΔF-GFP and pSeV18+SEAP/MtsHNts P511Lmut^{·}ΔF-GFP, respectively.

### [Example 15] Reconstruction and amplification of F-deficient SeV comprising both temperature resistant mutation and P/L mutation

The virus was reconstructed in accordance with the method reported by Li *et al*. (Li, H.-O. *et al*., J. Virology 74, 6564-6569 (2000) , WO00/70070), the details of which are found in Example 2 of this specification. The titers of the respective viral solutions prepared by this method were as follows: 8.6x10⁸ GFP-CIU/mL for SeV18+/MtsHNts P86Lmut^{·}ΔF-GFP; 4.2x10⁸ GFP-CIU/mL for SeV18+/MtsHNts P511Lmut^{·}ΔF-GFP; 1.7x10⁸ GFP-CIU/mL for SeV18+SEAP/MtsHNts P86Lmut^{·} ΔF-GFP; and 1.7x10⁸ GFP-CIU/mL for SeV18+SEAP/MtsHNts P511Lmut ^{·} ΔF-GFP (GFP-CIU is defined as described in WO00/70070).

### [Example 16] Quantification of secondarily released particles from F-deficient SeV comprising both temperature resistant mutation and P/L mutation

Secondarily released particles were quantified by measuring HA activity using the culture supernatant of infected cells. Measurments for SeV18+/ΔF-GFP were also conducted at the same time. Experimental method is detailed in Example 4 and Example 11 above. Briefly, 1x10⁷ CIU/ml or 3x 10⁷ CIU/ml of each viral solution was added (100 µl/well) to LLC-MK2 cells grown to confluency on 6-well plates (each MOI=1 or MOI=3). Cells were then infected for one hour. The cells were washed with MEM and combined with 1 ml of serum-free MEM per well, and then cultured at 37°C. Sampling was carried out every day, and immediately after sampling, 1 ml of fresh serum-free MEM was added to the sample. Culturing and sampling were conducted over time.

HA activity was measured according to the method of Kato *et al*. (Kato, A., *et al*., Genes Cell 1, 569-579 (1996)). Thus, the viral solution was serially diluted with PBS, making serial two-fold 50 µl dilutions for each well of a 96-well round-bottomed plate. 50 µl of this solution was combined with 50 µl of preserved chicken blood diluted to 1% with a PBS (Cosmo Bio Co. Ltd. , Tokyo, Japan) , and allowed to stand at 4°C for one hour. Erythrocyte agglutination was examined, and, among agglutinated samples, HA activity was judged to be the highest dilution rate to achieve hemagglutination.

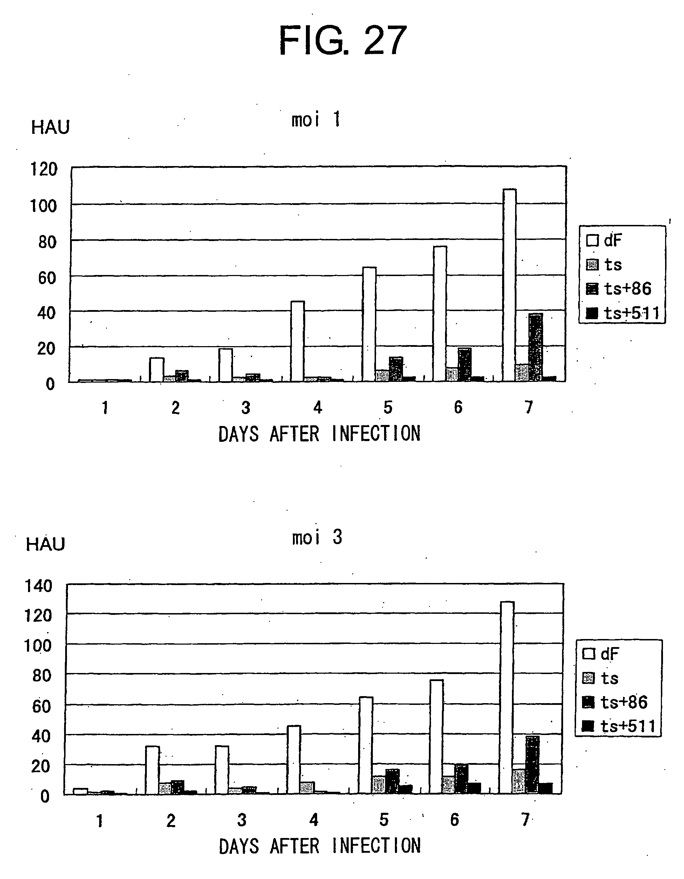

When compared with cells infected with a non-mutant F-deficient SeV (SeV18+/ΔF-GFP), for both MOI=1 and MOI=3, secondarily released particle levels were reduced in cells infected with the F-deficient SeV vector comprising both the temperature resistant mutation and the P/L mutation (Fig. 27). Secondarily released particles were especially reduced in SeV18+/MtsHNts P511Lmut^{·}ΔF-GFP compared to SeV18+/MtsHNts ΔF-GFP without P/L mutation, revealing an additive effect attributable to P/L mutation transduction. However, SeV18+/MtsHNts P86Lmut ^{·} ΔF-GFP exhibited increased secondarily released particle levels compared to SeV18+/MtsHNts ΔF-GFP, thus exhibiting no additive effect. From the point of view of reduction in secondarily released particles, SeV18+/MtsHNts P511Lmut^{·}ΔF-GFP was judged to be outstanding.

### [Example 17] Cytotoxicity of F-deficient SeV comprising both temperature resistant mutation and P/L mutation

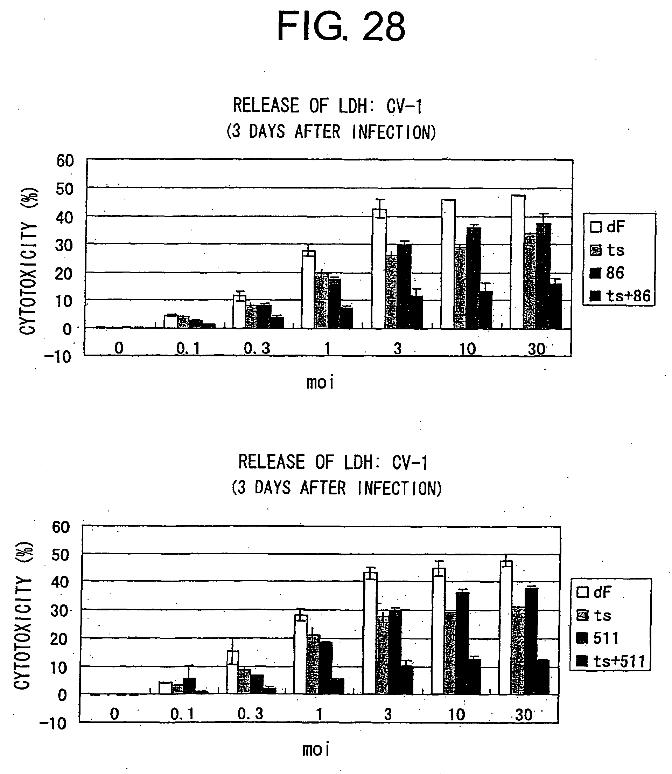

Cytotoxicity was evaluated by utilizing CV-1 cells. The controls employed were an F-deficient SeV comprising no mutation transduced into P/L (SeV18+/ΔF-GFP); and two types of SeV, one comprising only a temperature sensitive mutation (SeV18+/MtsHNts ΔF-GFP), and the other comprising only a P/L mutation (SeV18+/P511Lmut^{·}ΔF-GFP, SeV18+/P86Lmut^{·}ΔF-GFP). The experimental method is detailed in Example 6 and Example 12. Briefly, CV-1 cells were inoculated into a 96-well plate at a density of 2.5x 10⁴ cells/well (100 µL/well), and then cultured. The culture used MEM supplemented with 10% FBS. After 24 hours of culture, an SeV18+/ΔF-GFP, SeV18+/MtsHNts P86Lmut^{·}ΔF-GFP or SeV18+/MtsHNts P511Lmut^{·}ΔF-GFP solution diluted with 1% BSA-supplemented MEM was added in a volume of 5 µL/well to affect infection. Six hours later, medium comprising the virus solution was removed, and replaced with FBS-free MEM medium. Three days after infection, the culture supernatant was sampled, and subjected to quantification using a Cytotoxicity Detection Kit (Roche, Basel, Switzerland) according to kit instructions. Transduction of the temperature sensitive mutation (in SeV18+/MtsHNts ^{·} ΔF-GFP) reduced cytotoxicity to some extent, and transduction of the P/L mutation (in SeV18+/P86Lmut^{·}ΔF-GFP and SeV18+/P511Lmut^{·}ΔF-GFP) also resulted in a similar reduction in cytotoxicity. The combination of both (in SeV18+/MtsHNts P86Lmut^{·}ΔF-GFP and SeV18+/MtsHNts P511Lmut^{·} ΔF-GFP) resulted in an additive effect, leading to a marked reduction in cytotoxicity (Fig. 28).

### [Example 18] Quantification of expression of the genes comprised in F-deficient SeV comprising both temperature resistant mutation and P/L mutation

Both SeV18+/MtsHNts P86Lmut ^{·} ΔF-GFP and SeV18+/MtsHNts P511Lmut^{·}ΔF-GFP were also transduced with the SEAP gene at the +18 position, and SEAP expression level in infected cells was measured over time.

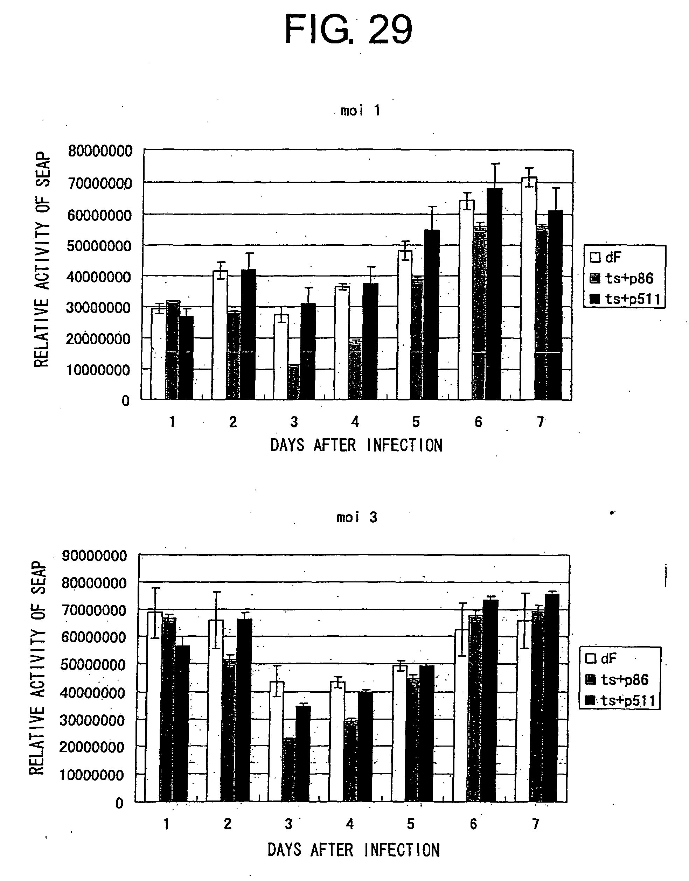

The experimental method is detailed in Example 5 and Example 13. Briefly, the LLC-MK2 cells were infected with SeV18+SEAP/ΔF-GFP, SeV18+SEAP/MtsHNts P86Lmut^{·}ΔF-GFP or SeV18+SEAP/MtsHNts P511Lmut· ΔF-GFP at MOI=1 or MOI=3, and the culture supernatant was sampled over time (every 24 hours). SEAP activity was examined using a Reporter Assay Kit-SEAP (Toyobo Co. Ltd., Osaka, Japan) according to kit instructions. While a slight reduction in the expression level during the early stages of infection was noted, SEAP activity was almost similar for all vectors (Fig. 29). Thus, it was judged that there was almost no reduction in expression level, even when using the vector comprising both temperature resistant mutation and P/L mutation.

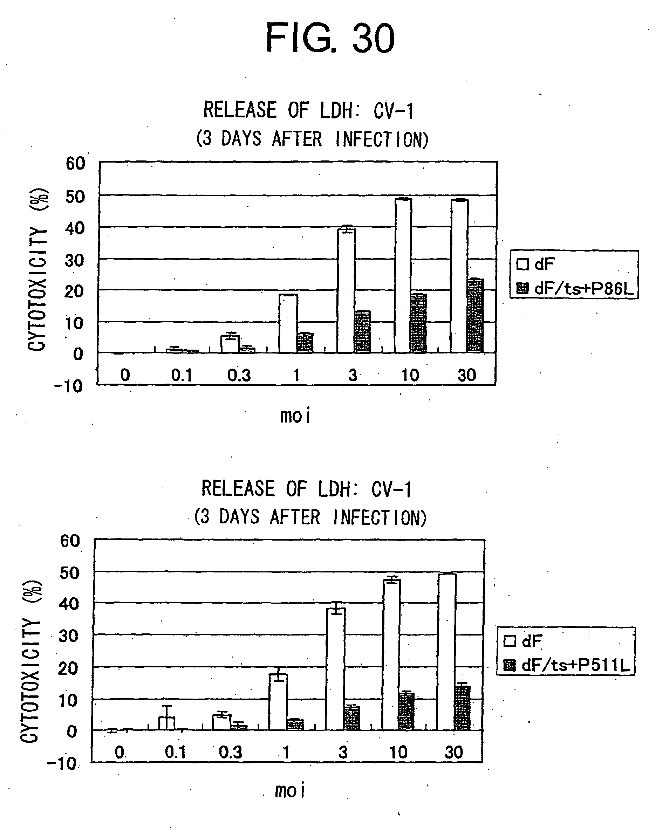

As an index of cytotoxicity, each vector comprising the SEAP gene was also subjected to LDH quantification in the infected cell culture supernatant. Similar to vectors without the SEAP gene, the transduction of both the temperature resistant mutation and the P/L mutation resulted in a marked reduction in cytotoxicity (Fig. 30).

### [Example 19] Construction of the genomic cDNA of M gene-deficient SeV comprising the EGFP gene

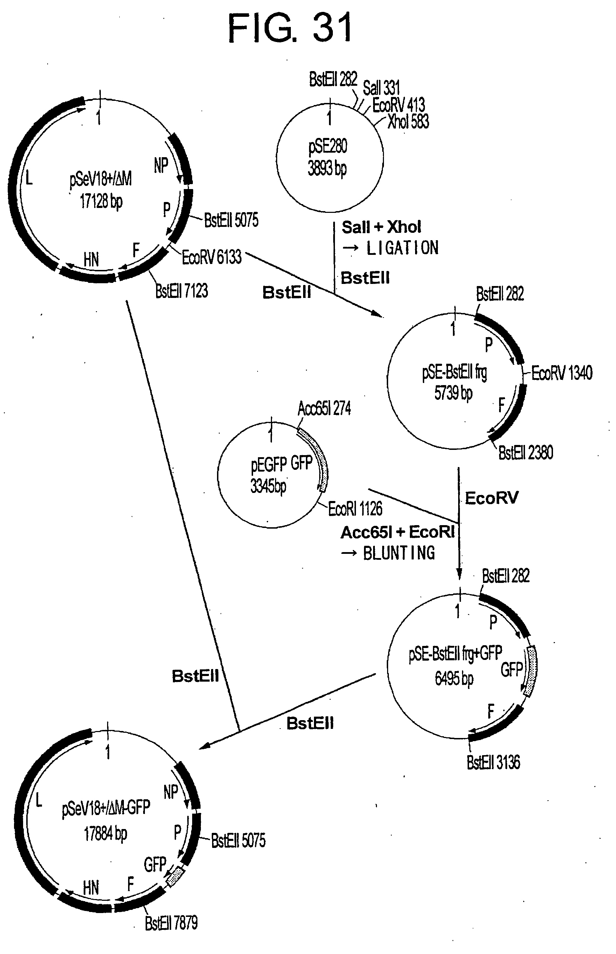

This construction used the full-length genomic cDNA of M-deficient SeV, which is M gene-deficient (pSeV18+/ΔM: WO00/09700). The construction scheme is shown in Fig. 31. The BstEII fragment (2098 bp) comprising the M-deficient site of pSeV18+/ΔM was subcloned to the BstEII site of pSE280 (pSE-BstEIIfrg construction). The EcoRV recognition site at this pSE280 site had been deleted by previous digestion with SalI/XhoI followed by ligation (Invitrogen, Groningen, Netherlands). pEGFP comprising the GFP gene (TOYOBO, Osaka, Japan) was digested using Acc65I and EcoRI, and the 5'-end of the digest was blunted by filling in using a DNA blunting Kit (Takara, Kyoto, Japan). The blunted fragment was then subcloned into the pSE-BstEIIfrg, which had been digested with EcoRV and treated with BAP (TOYOBO, Osaka, Japan). This BstEII fragment, comprising the EGFP gene, was returned to the original pSeV18+/ΔM to construct the M gene-deficient SeV genomic cDNA (pSeV18+/ΔM-GFP), comprising the EGFP gene at the M-deficient site.

### [Example 20] Construction of SeV genomic cDNA deficient in the M and F genes

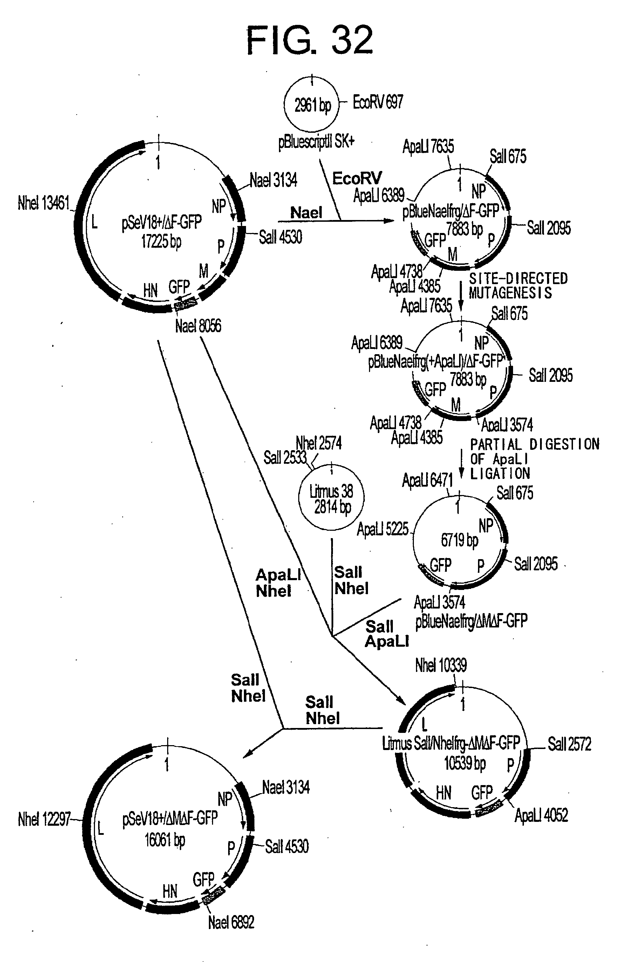

The construction scheme described below is shown in Fig. 32. The M gene was deleted using pBlueNaeIfrg-ΔFGFP, which was constructed by subcloning an NaeI fragment (4922 bp) of the F-deficient Sendai virus full-length genomic cDNA comprising the EGFP gene at the F gene-deficient site (pSeV18+/ΔF-GFP: Li, H.-O. *et al*., J. Virology 74, 6564-6569 (2000), WO00/70070), to the EcoRV site of pBluescript II (Stratagene, La Jolla, CA). Deletion was designed so as to excise the M gene using the ApaLI site directly behind it. That is, the ApaLI recognition site was inserted right behind the P gene, so that the fragment to be excised became 6n. Mutagenesis was performed using the QuikChange™ Site-Directed Mutagenesis Kit (Stratagene, La Jolla, CA) according to kit method. The synthetic oligonucleotide sequences used for the mutagenesis were 5'-agagtcactgaccaactagatcgtgcacgaggcatcctaccatcctca-3'/ SEQ ID NO: 27 and 5'-tgaggatggtaggatgcctcgtgcacgatctagttggtcagtgactct-3'/SEQ ID NO: 28. After mutagenesis, the resulting mutant cDNA was partially digested using ApaLI (at 37°C for five minutes) , recovered using a QIAquick PCR Purification Kit (QIAGEN, Bothell, WA) , and then ligated as it was. The DNA was again recovered using the QIAquick PCR Purification Kit, digested with BsmI and StuI, and used to transform DH5α to prepare the M gene-deficient (and F gene-deficient) DNA (pBlueNaeIfrg-ΔMΔFGFP).

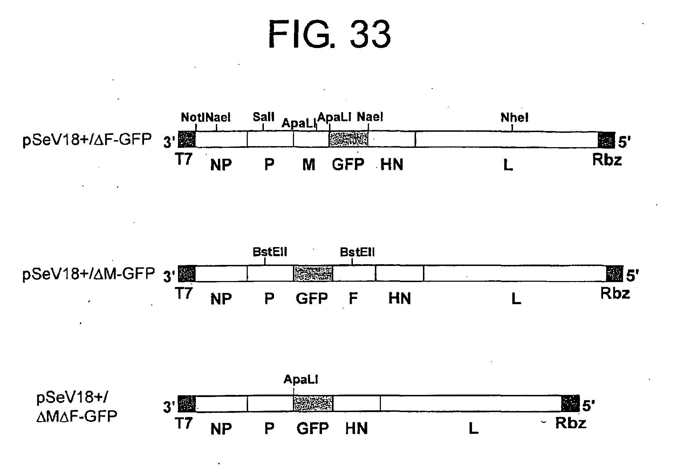

pBlueNaeIfrg-ΔMΔFGFP deficient in the M gene (and F gene) was digested with SalI and ApaLI to recover the 1480 bp fragment comprising the M gene-deficient site. pSeV18+/ΔF-GFP was digested with ApaLI/NheI to recover the HN gene-comprising fragment (6287 bp) , and these two fragments were subcloned into the SalI/NheI site of Litmus 38 (New England Biolabs, Beverly, MA) (LitmusSalI/NheIfrg-ΔMΔFGFP construction). The 7767 bp fragment recovered by digesting LitmusSalI/NheIfrg-ΔMΔFGFP with SalI/NheI was ligated to another fragment (8294 bp) obtained by digesting pSeV18+/ΔF-GFP with SalI/NheI, that did not comprise genes such as the M and HN genes. In this way an M- and F-deficient Sendai virus full-length genome cDNA comprising the EGFP gene at the deficient site (pSeV18+/ΔMΔF-GFP) was constructed. Structures of the M-deficient (and M- and F-deficient) viruses thus constructed are shown in Fig. 33.

### [Example 21] Preparation of helper cells expressing SeV-F and SeV-M proteins

To prepare helper cells expressing M protein (and F protein), the Cre/loxP expression induction system was used. This system uses a plasmid, pCALNdLw, which is designed to induce the expression of gene products using Cre DNA recombinase (Arai, T. *et al*., J. Virol. 72: 1115-1121 (1988)). This system was also employed for the preparation of helper cells (LLC-MK2/F7 cells) for the F protein (Li, H.-O. *et al*., J. Virology 74, 6564-6569 (2000), WO00/70070).

### <1> Construction of M gene-expressing plasmids

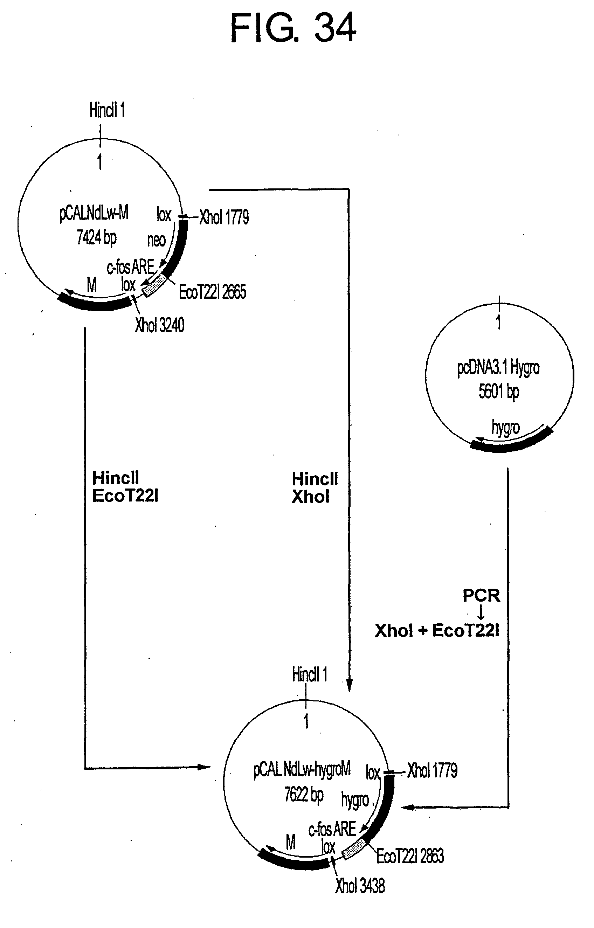

To prepare helper cells which induce the simultaneous expression of F and M proteins, the above-described LLC-MK2/F7 cells were used to transfer the M gene to these cells using the above-mentioned system. Since the pCALNdLw/F used in the transfer of the F gene contained the neomycin resistance gene, it was essential to insert a different drug resistance gene to enable use of the same cells. Therefore, according to the scheme described in Fig. 34, the neomycin resistance gene of the M gene-comprising plasmid (pCALNdLw/M: the M gene was inserted at the SwaI site of pCALNdLw) was replaced with the hygromycin resistance gene. That is, after pCALNdLw/M was digested with HincII and EcoT22I, an M gene-comprising fragment (4737 bp) was isolated by electrophoresis on agarose, and the corresponding band was excised and recovered using the QIAEXII Gel Extraction System. At the same time, pCALNdLw/M was digested with XhoI to recover a fragment that did not comprise the neomycin resistance gene (5941 bp), and then further digested with HincII to recover a 1779 bp fragment. The hygromycin resistance gene was prepared by performing PCR using pcDNA3.1hygro(+) (Invitrogen, Groningen, Netherlands) as the template and the following pair of primers: hygro-5' (5'-tctcgagtcgctcggtacgatgaaaaagcctgaactcaccgcgacgtctgtcgag-3'/SEQ ID NO: 29) and hygro-3' (5'-aatgcatgatcagtaaattacaatgaacatcgaaccccagagtcccgcctattcctttgc cctcggacgagtgctggggcgtc-3')/SEQ ID NO: 30). The PCR product was recovered using the QIAquick PCR Purification Kit, and then digested using XhoI and EcoT22I. pCALNdLw-hygroM was constructed by ligating these three fragments.

### <2> Cloning of helper cells which induce the expression of SeV-M (and SeV-F) protein(s)

Transfection was performed using the Superfect Transfection Reagent by the method described in the Reagent's protocol. LLC-MK2/F7 cells were plated on 60 mm diameter Petri dishes at 5x10⁵ cells/dish, and then cultured in D-MEM containing 10% FBS for 24 hours. pCALNdLw-hygroM (5 µg) was diluted in D-MEM containing neither FBS nor antibiotics (150 µl in total). This mixture was stirred, 30 µl of the Superfect Transfection Reagent was added, and the mixture was stirred again. After standing at room temperature for ten minutes, D-MEM containing 10% FBS (1 ml) was added. The transfection mixture thus prepared was stirred, and added to LLC-MK2/F7 cells which had been washed once with PBS. After three hours of culture in an incubator at 37°C and in 5% CO₂ atmosphere, the transfection mixture was removed, and the cells were washed three times with PBS. D-MEM containing 10% FBS (5 ml) was added to the cells, which were then cultured for 24 hours. After culture, the cells were detached using trypsin, plated onto a 96-well plate at a dilution of about 5 cells/well, and cultured in D-MEM containing 10% FBS supplemented with 150 µg/ml hygromycin (Gibco-BRL, Rockville, MD) for about two weeks. Clones propagated from a single cell were cultured to expand to a 6-well plate culture. A total of 130 clones were thus prepared, and were analyzed as detailed below.

### <3> Analysis of helper cell clones which induce the expression of SeV-M (and SeV-F) protein(s)

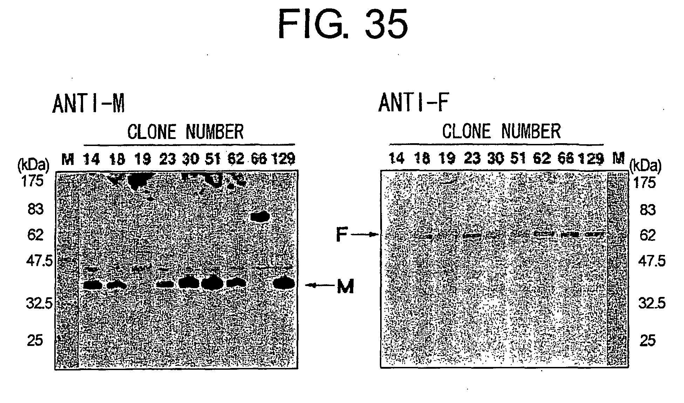

Western-blotting was used to semi-quantitatively analyze M protein expression in the 130 clones obtained as detailed above. Each clone was plated onto a 6-well plate, and, when in a state of near confluence, infected at MOI=5 with a recombinant adenovirus expressing Cre DNA recombinase (AxCANCre) diluted in MEM containing 5% FBS, according to the method of Saito *et al*. (Saito, I. *et al*., Nucl. Acid. Res. 23, 3816-3821 (1995); Arai, T. *et al*., J. Virol. 72, 1115-1121 (1998)). After culturing at 32°C for two days, the culture supernatant was removed. The cells were washed once with PBS, and recovered by detachment using a scraper. SDS-PAGE was performed by applying 1/10 of the cells thus recovered per lane, and then Western-Blotting was carried out using anti-M protein antibody, according to the method described in Examples 3 and 4. Of the 130 clones, those showing relatively high M protein expression levels were also analyzed by Western-blotting using the anti-F protein antibody (f236: Segawa, H. *et al*., J. Biochem. 123, 1064-1072 (1998)). Both results are described in Fig. 35.

### [Example 22] Reconstitution of M gene-deficient SeV virus

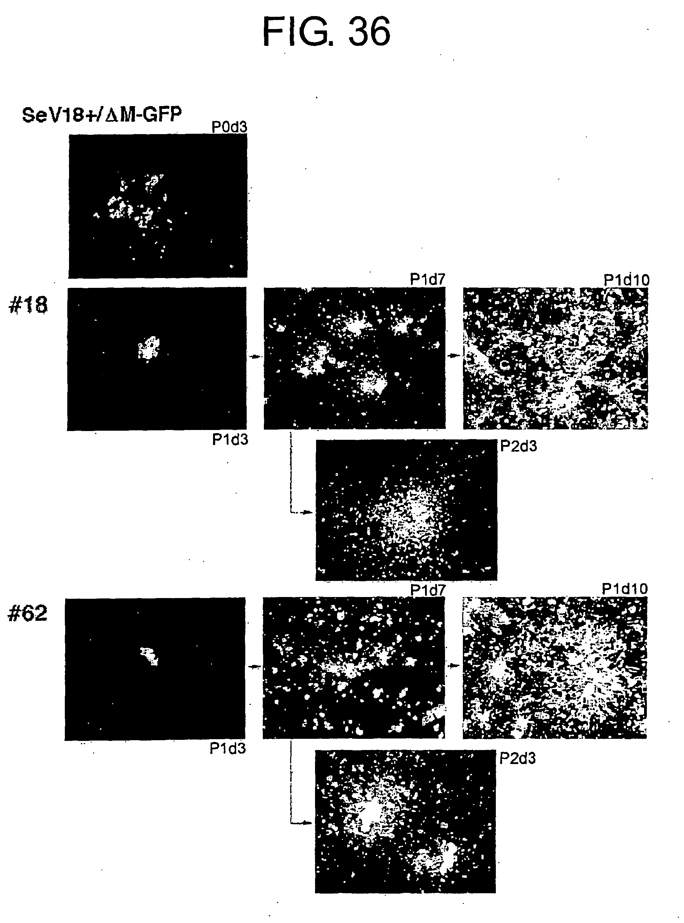

Reconstitution of M gene-deficient SeV (SeV18+/ΔM-GFP) was carried out in conjunction with assessment of the clones described in Example 21. That is, P0 lysate of SeV18+/ΔM-GFP was added to each clone, and whether or not GFP protein spread was observed (whether or not the trans-supply of M protein was achieved) was examined. P0 lysate was prepared according to the method described in Example 2, as follows: LLC-MK2 cells were plated on 100-mm diameter Petri dishes at 5x10⁶ cells/dish, cultured for 24 hours, and then infected at MOI=2 with PLWUV-VacT7 at room temperature for one hour. Plasmids pSeV18+/ΔM-GFP, pGEM/NP, pGEM/P, pGEM/L, pGEM/F-HN and pGEM/M were suspended in Opti-MEM at weight ratios of 12 µg, 4 µg, 2 µg, 4 µg, 4 µg and 4 µg/dish, respectively. To these suspensions, the equivalent of 1 µg DNA/5 µl of SuperFect transfection reagent was added and mixed. The mixture was allowed to stand at room temperature for 15 minutes, and finally added to 3 ml of Opti-MEM containing 3% FBS. This mixture was added to the cells, which were then cultured. After culturing for five hours, the cells were washed twice with serum-free MEM, and cultured in MEM containing 40 µg/ml AraC and 7.5 µg/ml trypsin. After 24 hours of culture, LLC-MK2/F7/A cells were layered at 8.5x10⁶ cells/dish, and further cultured in MEM containing 40 µg/ml AraC and 7.5 µg/ml trypsin at 37°C for two days. These cells were recovered, the pellet was suspended in 2 ml/dish Opti-MEM, and P0 lysate was prepared by repeating three cycles of freezing and thawing. At the same time, ten different clones were plated on 24-well plates. When nearly confluent, they were infected with AxCANCre at MOI=5, and cultured at 32°C for two days. These cells were transfected with P0 lysate of SeV18+/ΔM-GFP at 200 µl/well, and cultured using serum-free MEM containing 40 µg/ml AraC and 7.5 µg/ml trypsin at 32°C. GFP protein spread due to SeV18+/ΔM-GFP was observed in clones #18 and #62 (Fig. 36). This spread was especially rapid in clone #62, which was used in subsequent experiments. Hereafter, these cells prior to induction with AxCANCre are referred to as LLC-MK2/F7/M62. After induction, cells which continuously express F and M proteins are referred to as LLC-MK2/F7/M62/A. Preparation of SeV18+/ΔM-GFP cells was continued using LLC-MK2/F7/M62/A cells. Six days after P2 infection, 9.5x10⁷ GFP-CIU viruses were prepared. Five days after P4 infection, 3.7x10⁷ GFP-CIU viruses were prepared.

It is thought that, in this experiment, recovery of the SeV18+/ΔM-GFP virus became possible after the modification indicated in Example 3 (e.g., culturing at 32°C after the P1 stage). In SeV18+/ΔM-GFP, *in trans* supply of M protein from expression cells (LLC-MK2/F7/M62/A) is thought to be a cause, however, spread was extremely slow, and was finally observed seven days after P1 infection (Fig. 36). Thus, also in viral reconstitution experiments, "culturing at 32°C after the P1 stage" is supported as being very effective in reconstituting SeV which has inefficient transcription-replication or poor ability to form infectious virions.

### [Example 23] Productivity of an M gene-deficient virus

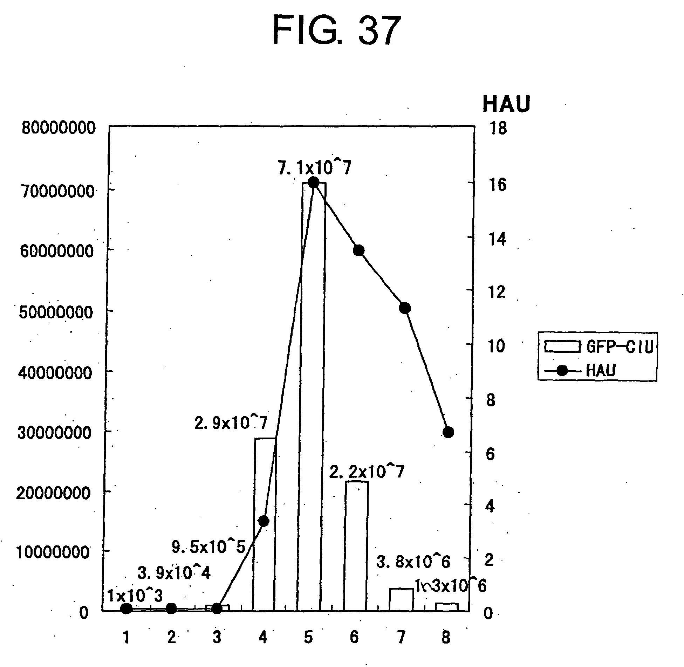

The productivity of this virus was also investigated. LLC-MK2/F7/M62/A cells were plated on 6-well plates and cultured at 37°C. When the cells were nearly confluent, they were shifted to 32°C. One day later, these cells were infected at MOI=0.5 with SeV18+/ΔM-GFP. The culture supernatant was recovered over time, and replaced with fresh medium. Supernatants thus recovered were assayed for CIU and HAU. Most viruses were recovered four to six days after infection (Fig. 37). HAU was maintained for six or more days after infection, however cytotoxicity was strongly exhibited at this point, indicating the cause was not HA protein originating in viral particles, but rather the activity of HA protein free or bound to cell debris. Therefore for virus collection, the culture supernatant should be recovered by the fifth day after infection.

### [Example 24] Structural confirmation of M gene-deficient SeV

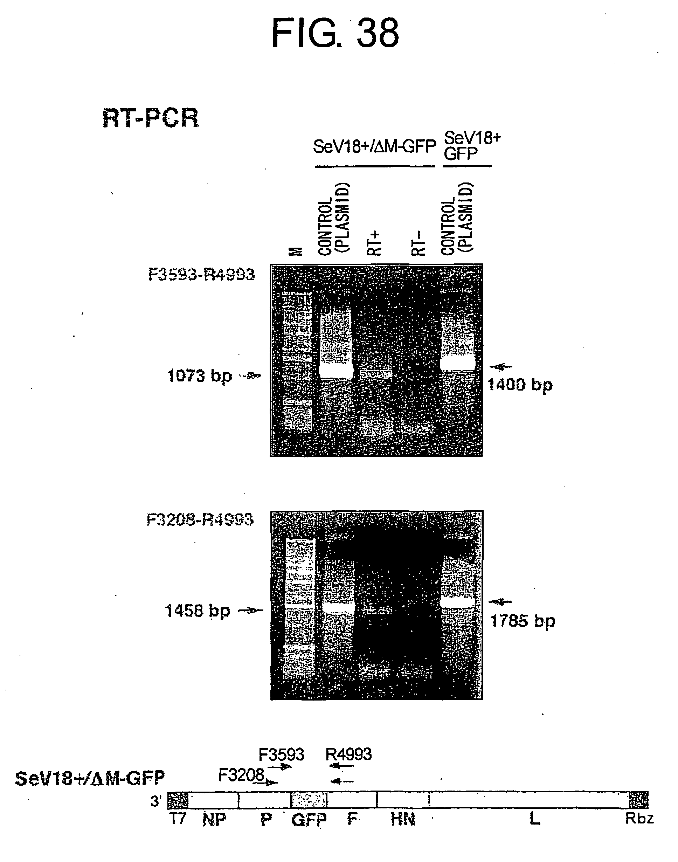

SeV18+/ΔM-GFP's viral genes were confirmed by RT-PCR, and the viral proteins by Western-blotting. In RT-PCR, the P2 stage virus six days after infection was used. QIAamp Viral RNA Mini Kit (QIAGEN, Bothell, WA) was used in the recovery of RNA from the viral solution. Thermoscript RT-PCR System (Gibco-BRL, Rockville, MD) was used to prepare the cDNA. Both systems were performed using kit protocol methods. The random hexamer supplied with the kit was used as the primer for cDNA preparation. To confirm that the product was formed starting from RNA, RT-PCR was performed in the presence or absence of reverse transcriptase. PCR was performed with the above-prepared cDNA as the template, using two pairs of primers: one combination of F3593 (5'-ccaatctaccatcagcatcagc-3'/SEQ ID NO: 31) on the P gene and R4993 (5'-ttcccttcatcgactatgacc-3'/SEQ ID NO: 32) on the F gene, and another combination of F3208 (5'-agagaacaagactaaggctacc-3'/SEQ ID NO: 33) on the P gene and R4993. As expected from the gene structure of SeV18+/ΔM-GFP, amplifications of 1073 bp and 1458 bp DNAs were observed from the former and latter combinations respectively (Fig. 38). Where reverse transcriptase was omitted (RT-), gene amplification did not occur. Where the M gene was inserted instead of the GFP gene (pSeV18+GFP), 1400 bp and 1785 bp DNAs were amplified respectively. These DNAs are clearly different in size from those described above, supporting the fact that this virus is M gene-deficient in structure.

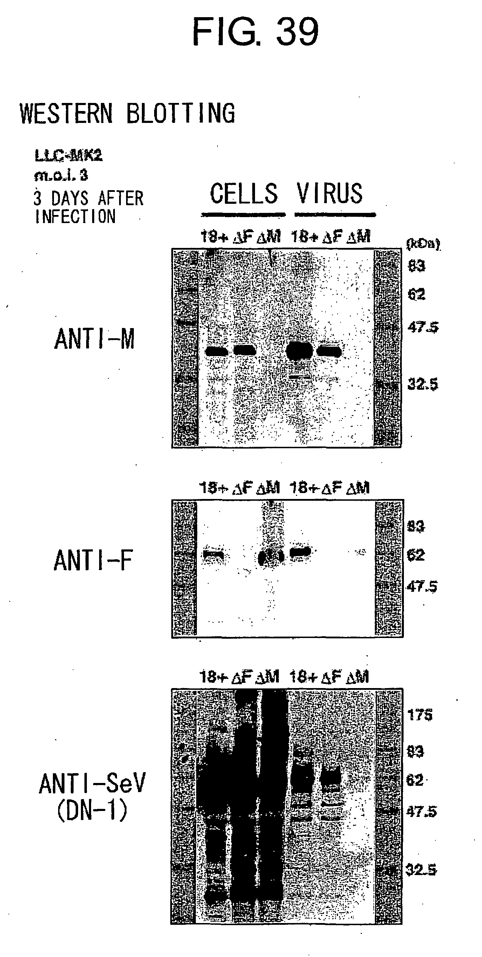

Protein confirmation was performed using Western- blotting. LLC-MK2 cells were infected at MOI=3 with SeV18+/ΔM-GFP, SeV18+/ΔF-GFP and SeV18+GFP, respectively, and the culture supernatant and cells were recovered three days after infection. The culture supernatant was centrifuged at 48,000 g for 45 minutes to recover viral proteins. After SDS-PAGE, Western-blotting was performed to detect proteins using anti-M protein antibody, anti-F protein antibody, and DN-1 antibody (rabbit polyclonal) which mainly detects NP protein, according to the method described in Examples 3 and 4. In cells infected with SeV18+/ΔM-GFP, M protein was not detected while F or NP protein was observed. Therefore this virus was also confirmed to have the SeV18+/ΔM-GFP structure from the point of view of proteins (Fig. 39). F protein was not observed in cells infected with SeV18+/ΔF-GFP, while all viral proteins examined were detected in cells infected with SeV18+GFP. In addition, very little NP protein was observed in the culture supernatant in the case of infection with SeV18+/ΔM-GFP, indicating that there were no or very few secondarily released particles.

### [Example 25] Quantitative analysis concerning the presence or absence of secondarily released particles of M gene-deficient SeV

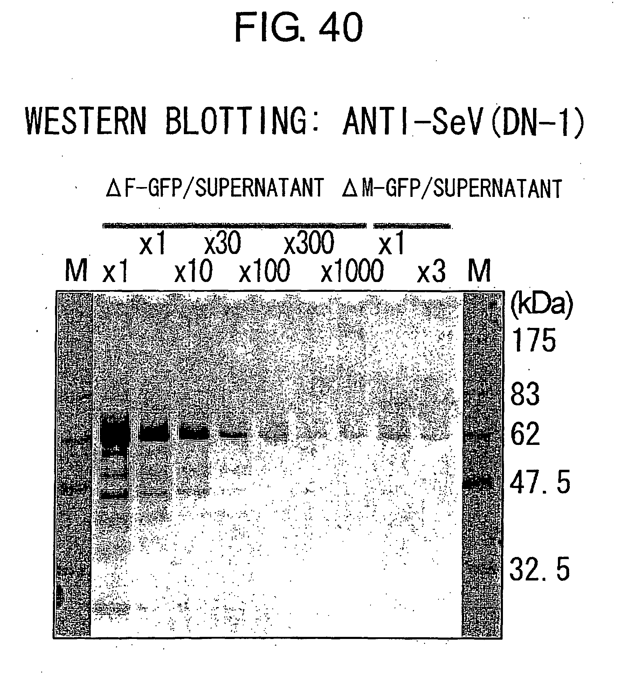

As described in Example 24, LLK-MK2 cells were infected with SeV18+/ΔM-GFP at MOI=3, the culture supernatant was recovered three days after infection, filtered through an 0.45 µm pore diameter filter, and then centrifuged at 48,000 g for 45 minutes to recover viral proteins. Western-blotting was then used to semi-quantitatively detect viral proteins in the culture supernatant. Samples similarly prepared from cells infected with SeV18+/ΔF-GFP were used as the control. Serial dilutions of respective samples were prepared and subjected to Western-blotting to detect proteins using the DN-1 antibody (primarily recognizing NP protein). The viral protein level in the culture supernatant of cells infected with SeV18+/ΔM-GFP was estimated to be about 1/100 that of cells infected with SeV18+/ΔF-GFP (Fig. 40). Sample HA activities were 64 HAU for SeV18+/ΔF-GFP, compared to less than 2 HAU for SeV18+/ΔM-GFP.

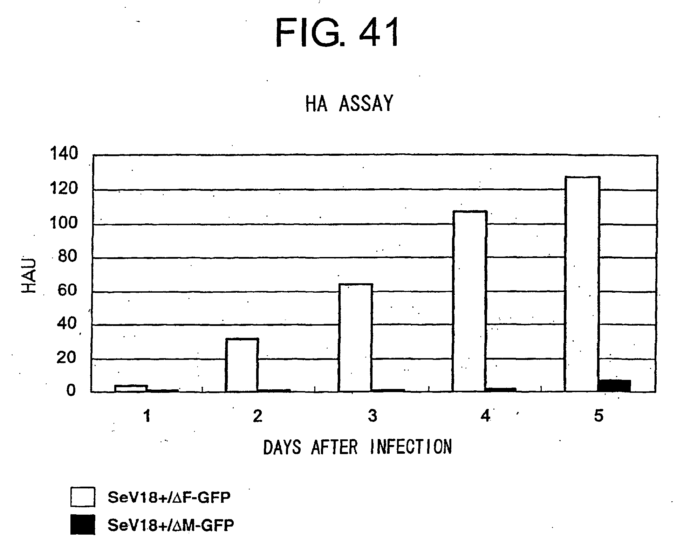

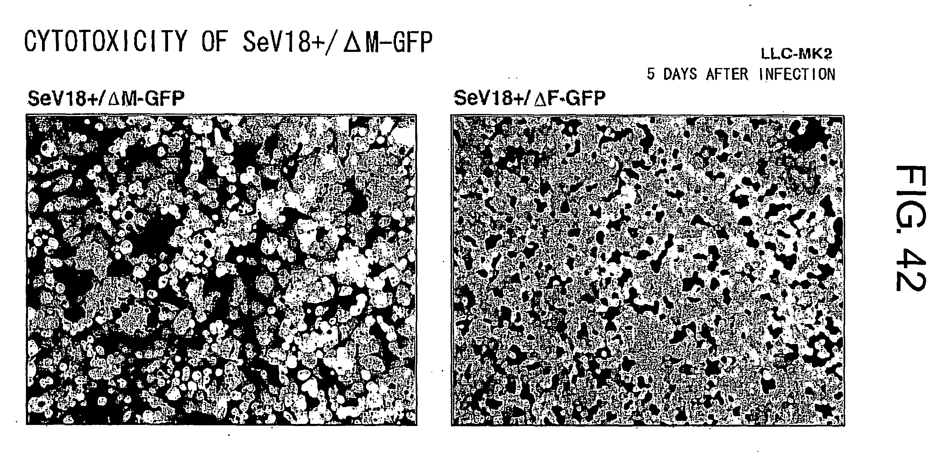

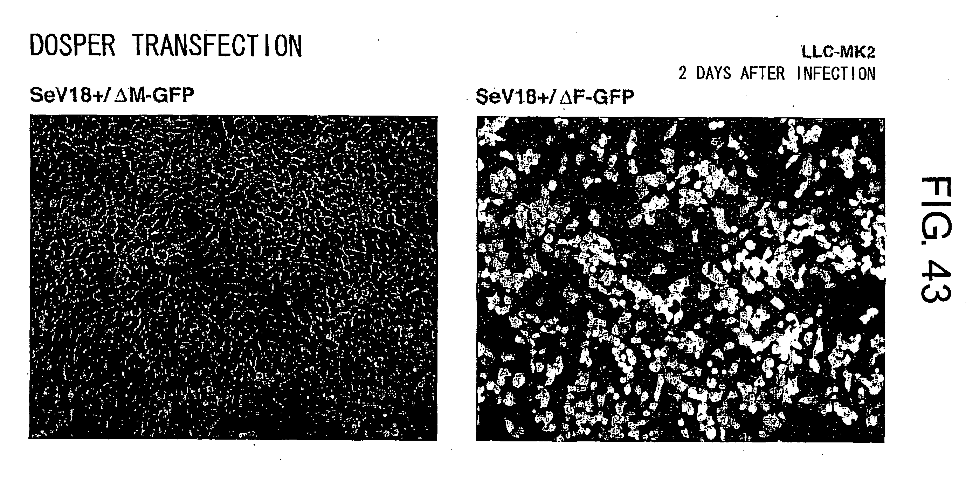

Time courses were examined for the same experiments. That is, LLC-MK2 cells were infected at MOI=3 with SeV18+/ΔM-GFP, and the culture supernatant was recovered over time (every day) to measure HA activity (Fig. 41). Four days or more after infection, slight HA activity was detected. However, measurements of LDH activity, an indicator of cytotoxicity, revealed clear cytotoxicity four or more days after infection in the SeV18+/ΔM-GFP-infected cells (Fig. 42). This indicated the strong possibility that elevated HA activity was not due to VLPs, but to the activity of HA protein bound to or free from cell debris. Furthermore, the culture supernatant obtained five days after infection was examined using Dosper Liposomal Transfection Reagent, a cationic liposome (Roche, Basel, Switzerland). The culture supernatant (100 µl) was mixed with Dosper (12.5 µl), allowed to stand at room temperature for ten minutes, and then transfected to LLC-MK2 cells cultured to confluency on 6-well plates. Inspection under a fluorescence microscope two days after transfection revealed that many GFP-positive cells were observed in the supernatant of cells infected with SeV18+/ΔF-GFP which contained secondarily released particles, while very few or almost no GFP-positive cells were observed in the supernatant of cells infected with SeV18+/ΔM-GFP (Fig. 43). From the above results, the secondary release of particles was concluded to be almost completely suppressed by M protein deficiency.

### [Example 26] Reconstitution of SeV deficient in both F and M genes

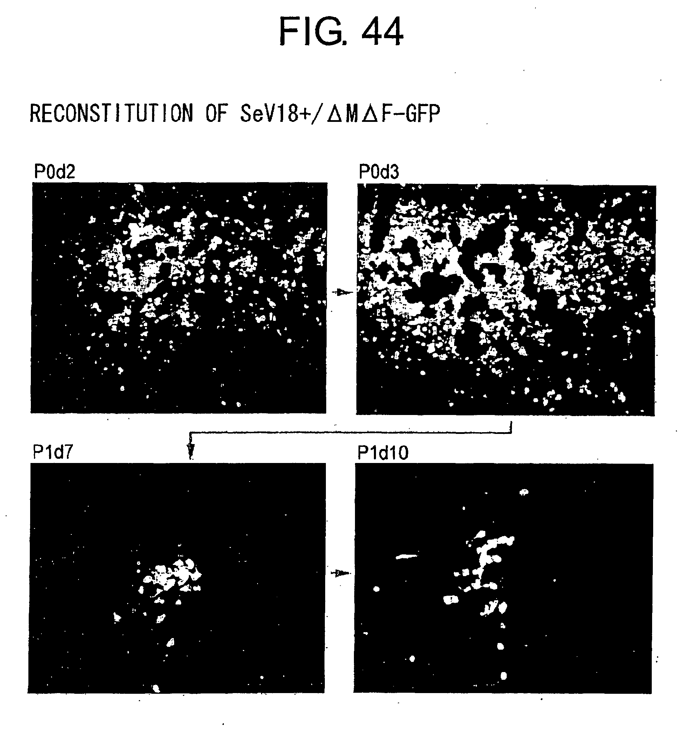

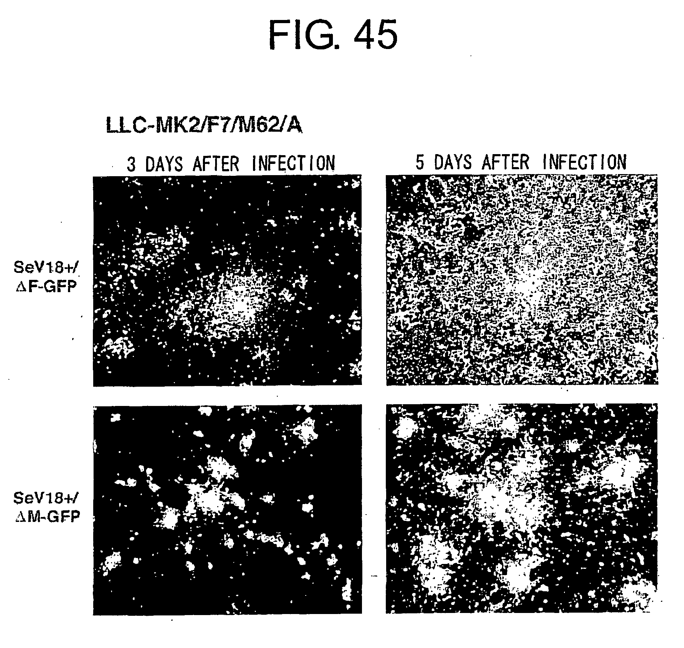

Reconstitution of SeV deficient in both F and M genes (SeV18+/ΔMΔF-GFP) was performed by the same method used for the reconstitution of SeV18+/ΔM-GFP, as described in Example 22. That is, LLC-MK2 cells were plated on 100-mm diameter Petri dishes at 5x10⁶ cells/dish, cultured for 24 hours, and then infected at MOI=2 with PLWUV-VacT7 at room temperature for one hour. Plasmids pSeV18+/ΔMΔF-GFP, pGEM/NP, pGEM/P, pGEM/L, pGEM/F-HN and pGEM/M were suspended in Opti-MEM at weight ratios of 12 µg, 4 µg, 2 µg, 4 µg, 4 µg and 4 µg/dish, respectively. One µg DNA/5 µl equivalent of SuperFect transfection reagent were added to the suspension and mixed. The mixture was allowed to stand at room temperature for 15 minutes before 3 ml of Opti-MEM containing 3% FBS was added. The mixture was added to the cells after washing with serum-free MEM, and the cells were cultured. After five hours of culture, the cells were washed twice with serum-free MEM, and cultured in MEM containing 40 µg/ml AraC and 7.5 µg/ml trypsin. After culturing for 24 hours, LLC-MK2/F7/M62/A cells were layered at 8.5x 10⁶ cells/dish, and further cultured in MEM containing 40 µg/ml AraC and 7.5 µg/ml trypsin at 37°C for two days. These cells were recovered, the pellet was suspended in 2 ml/dish of Opti-MEM, and P0 lysate was prepared by repeating three cycles of freezing and thawing. Meanwhile, LLC-MK2/F7/M62/A cells were plated on 24-well plates until nearly confluent, and then moved to 32°C, and cultured for one day. Cells thus prepared were transfected with P0 lysate of SeV18+/ΔMΔF-GFP at 200 µl/well, and cultured using serum-free MEM containing 40 µg/ml AraC and 7.5 µg/ml trypsin at 32°C. With P0, well spread GFP positive cells were observed. In the case of P1, spread of GFP positive cells was also observed, although very weak (Fig. 44). However, viral solution comprising a detectable titer could not be recovered. Where LLC-MK2/F7/M62/A cells were infected with SeV18+/ΔF-GFP or SeV18+/ΔM-GFP, the smooth spread of GFP positive cells was observed for both viruses (Fig. 45). Cells expressing both F and M (LLC-MK2/F7/M62/A cells) were infected with SeV18+/ΔF-GFP or SeV18+/ΔM-GFP at MOI=0.5. Sampling was carried out three and six days later. Samples were mixed with 1/6.5 volume of 7.5 % BSA (final concentration=1%) and stored. Vector productivity was investigated by measuring titers. As a result, SeV18+/ΔF-GFP was recovered as viral solution of 10⁸ or more GFP-CIU/ml and SeV18+/ΔM-GFP was recovered as viral solution of 10⁷ or more GFP-CIU/ml (Table 1). Thus, these results indicate that both M and F proteins can be successfully supplied *in trans.*

**Table 1**

| | 3 days after infection | 6 days after infection | |
|---|---|---|---|
| SeV18+/ΔF-GFP | 1.0x10⁸ | 1.7x10⁸ | |
| SeV18+/ΔM-GFP | 1.0x10⁷ | 3.6x10⁷ | GFP-CIU/ml |

### [Example 27] Improvement in helper cells expressing SeV-F and M proteins

When using the M/F-expressing LLC-MK2/F7/M62/A helper cells, M/F double-deficient SeV (SeV18+/ΔMΔF-GFP) viral particles could not be recovered. However, F-deficient SeV (SeV18+/ΔF-GFP) and M-deficient SeV (SeV18+/ΔM-GFP) could be both reconstructed and produced. Therefore, M and F protein trans-supply, as a basic ability of the Cre/loxP expression-inducing system utilizing the same helper cells, was considered to be sufficiently possible. Thus, the use of the Cre/loxP expression-inducing system was judged effective, and further increases in the levels of M and F proteins expressed using this system was judged to be required in order to enable reconstruction of the M/F double-deficient SeV.

### <1> Construction of a M and F-expressing plasmid

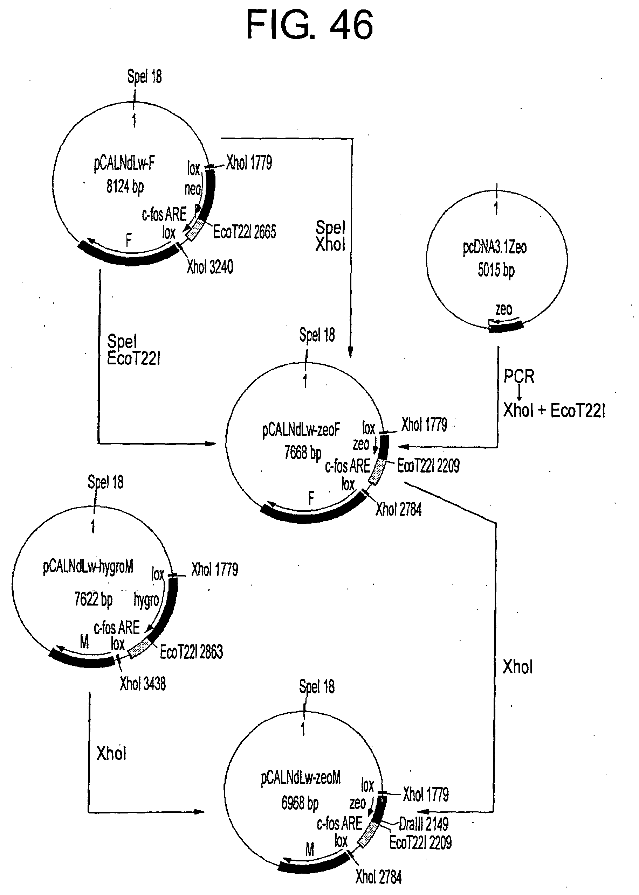

To improve the helper cells that simultaneously induce the expression of M and F proteins, the above-described system was used to re-transduce M and F genes into above-described LLC-MK2/F7/M62 cells (produced earlier). Since the pCALNdLw/F used to transduce the F gene comprised a neomycin resistant gene, and the pCALNdLw/hygroM used to transduce the M gene comprised a hygromycin resistant gene, different resistance genes needed to be transduced in order to use these cells. Accordingly, the neomycin resistant gene of the F gene-comprising plasmid (pCALNdLw/F: M gene transduced into the SwaI site of pCALNdLw) was replaced with a zeocin resistant gene, in accordance with the scheme shown in Fig. 46. Thus, the pCALNdLw/F was digested with SpeI and EcoT22I, an F gene-comprising fragment (5477 bp) was separated using agarose electrophoresis, and the relevant band was cut out and recovered using a QIAEXII Gel Extraction System. At the same time, pCALNdLw/F was cleaved with XhoI, and a fragment without the neomycin resistant gene (6663 bp) was recovered, and then further cleaved with SpeI to recover a 1761 bp fragment. The zeocin resistant gene was prepared as follows: PCR was carried out using pcDNA3.1Zeo(+) (Invitrogen, Groningen, Netherlands) as a template and two primers, zeo-5'(5'-TCTCGAGTCGCTCGGTACGatggccaagttgaccagtgccgttccggtgctcac-3'/SEQ ID No: 34); and zeo-3'(5'-AATGCATGATCAGTAAATTACAATGAACATCGAACCCCAGAGTCCCGCtcagtcctgctcctcg gccacgaagtgcacgcagttg-3'/SEQ ID NO: 35). The amplification product was recovered using a QIAquick PCR Purification Kit, and digested with XhoI and EcoT22I. These three fragments were then ligated to prepare pCALNdLw-zeoF. An XhoI fragment was then used to recombine the drug resistant gene-comprising fragment, and the pCALNdLw-zeoM gene was constructed.

### <2> Cloning of helper cells

Transfection was conducted according to the method described in the protocol using a LipofectAMINE PLUS reagent (Invitrogen Corp. , Groningen, Netherlands). Thus, the following procedures were performed: The LLC-MK2/F7/M62 cells were inoculated to 60 mm petri dishes at a density of 5x10⁵ cells/dish, and cultured in 10% FBS-comprising D-MEM for 24 hours. Each 1 µg (2 µg in total) of the pCALNdLw-zeoF and pCALNdLw-zeoM was diluted in FBS- and antibiotic-free D-MEM (242 µL in total), agitated, combined with 8 µl of a LipofectAMINE PLUS reagent, re-agitated, and allowed to stand at room temperature for 15 minutes. Subsequently, 12 µL of the LipofectAMINE reagent diluted preliminarily with an FBS- and antibiotic-free D-MEM (250 µL in total) was added, and the mixture was allowed to stand at room temperature for 15 minutes. Subsequently, 2 ml of the FBS- and antibiotic-free D-MEM was added and agitated. This transfection mixture was added to the LLC-MK2/F7/M62 cells, which had been washed once with PBS. After culturing in a 37°C in 5% CO₂ incubator for three hours, 2.5 ml of 20% FBS-containing D-MEM was added without removing the transfection mixture. After culturing for 24 hours, the cells were scraped using trypsin, dispensed to a 96-well plate at a density of about 5 cells/well or 25 cells/well, and cultured for about two weeks in 500 µg/mL zeocin (Gibco-BRL, Rockville, MD)-containing 10% FBS-supplemented D-MEM. Clones spreading from a single cell were propagated up to 6-well plates. A total of 98 clones thus prepared were analyzed.

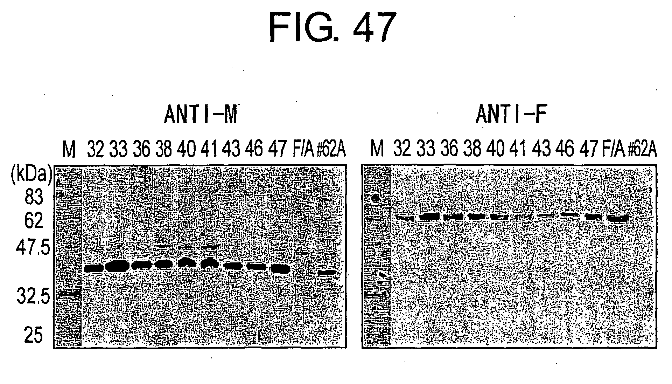

The resultant 98 clones were semi-quantitatively examined for M and F protein expression levels using Western-blotting. Each clone was inoculated into a 12-well plate, and when almost confluent, was infected with a Cre DNA recombinase-expressing recombinant adenovirus (AxCANCre) , diluted with a 5% FBS-containing MEM at MOI=5 using the method of Saito *et al*. (Saito, I. *et al*., Nucl. Acid. Res. 23, 3816-3821 (1995); and Arai, T. *et al*., J. Virol. 72, 1115-1121 (1998)). After culturing at 32°C for two days, the culture supernatant was removed. The cells were washed once with PBS, and recovered by scraping with a cell scraper. A 1/5 volume aliquot was applied per lane, subjected to SDS-PAGE, and then Western blotting using an anti-M antibody and an anti-F antibody (f236: Segawa, H. *et al*., J. Biochem. 123, 1064-1072 (1998)). The results of nine clones of about 98 evaluated are indicated in Fig. 47.

### [Example 28] Reconstitution of M/F double-deficient SeV (2)

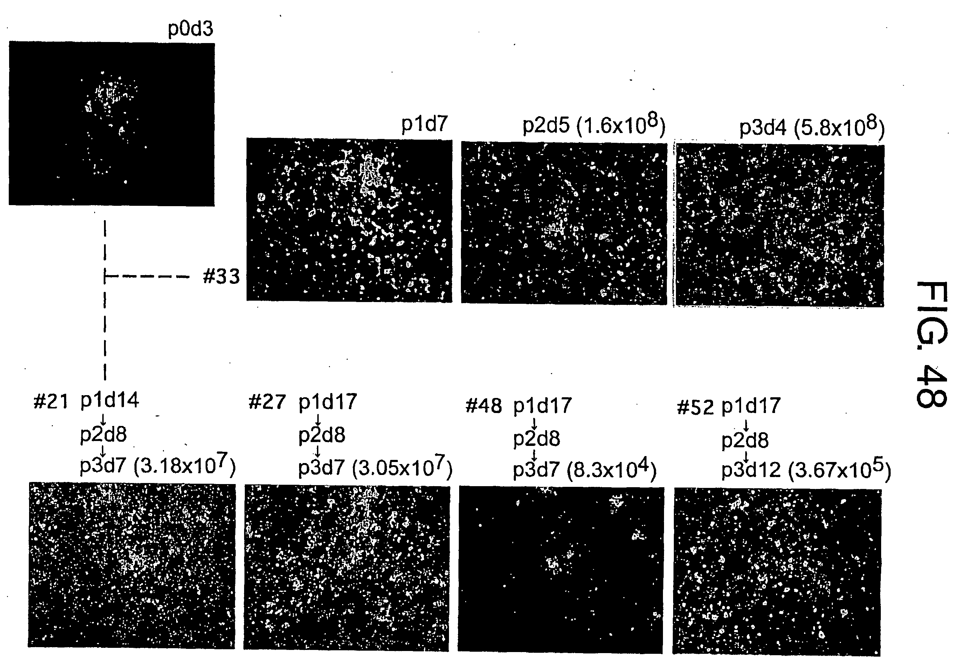

The reconstitution of M/F double-deficient SeV (SeV18+/ΔMΔF-GFP) was conducted in conjunction with the evaluation of the clones described in Example 27. Thus, the possibility of reconstitution was evaluated using P0 Lysate (lysate of the transfected cells) of the M/F double-deficient SeV reconstitution, . P0 lysate was prepared as described below, in a method analogous to that described in Example 2. LLC-MK2 cells were inoculated at a density of 5x10⁶ cells/dish to a 100 mm petri dish, cultured for 24 hours, and then infected with aPLWUV-VacT7 at room temperature for one hour (MOI=2). After washing with serum-free MEM, the plasmids pSeV18+/ΔMΔF-GFP, pGEM/NP, pGEM/P, pGEM/L, pGEM/F-HN and pGEM/M were suspended in Opti-MEM in the ratios of 12 µg, 4 µg, 2 µg, 4 µg, 4 µg and 4 µg/dish, respectively. SuperFect transfection reagent was added at a concentration of 5 µL reagent to 1 µg DNA. The samples were mixed, allowed to stand at room temperature for 15 minutes, and finally combined with 3 ml of a 3% FBS-containing Opti-MEM. The mixture was added to the cells, and they were cultured for five hours. The cells were then washed twice with serum-free MEM, and cultured in MEM containing 40 µg/mL AraC and 7.5 µg/mL Trypsin. After culturing for 24 hours, 8.5x 10⁶ cells/dish were overlaid with LLC-MK2/F7/A, and cultured for a further two days at 37°C in MEM containing 40 µg/mL AraC and 7.5 µg/mL Trypsin. These cells were recovered, the pellet was suspended in 2 ml/dish of Opti-MEM, and then frozen and thawed three times repeatedly, thus preparing P0 lysate. Meanwhile, freshly cloned cells were inoculated to a 24-well plate. When nearly confluent, these cells were infected with AxCANCre at MOI=5, and then cultured at 32°C for two days. The resultant cells were transfected with 200 µl/well of the P0 lysate of SeV18+/ΔMΔF-GFP, and cultured in serum-free MEM comprising 40 µg/mL AraC and 7.5 µg/mL Trypsin at 32°C. Twenty clones of those evaluated exhibited GFP protein spread, and M/F double-deficient SeVs were successfully recovered. Reconstitution conditions for several of these clones are indicated (Fig. 48). However, in clone #33 (LLC-MK2/F7/M62/#33), 10⁸ GFP-CIU/mL or more infected viral particles were recovered at the p3 stage (the third subculture). Therefore, clone #33 was regarded as an extremely promising cell for production. Transduction of LLC-MK2/F7/M62 cells with both M and F genes resulted in the preparation cells enabling high yield recovery of M/F double-deficient SeV. These findings suggest that expression is extremely satisfactory at the LLC-MK2/F7/M62 cell stage, and slightly upregulated in both M/F proteins (via transduction of both the M and F genes), enabling recovery of the M/F double-deficient SeV.

### [Example 29] Ability to produce M/F double-deficient SeV

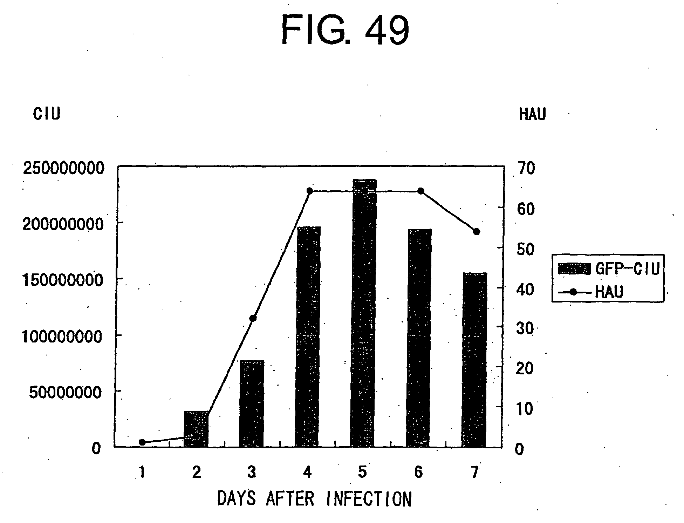

This virus was also examined from the point of view of ease of production. LLC-MK2/F7/M62/#33 were inoculated in a 6-well plate, and cultured at 37°C. When nearly confluent, the cells were infected with AxCANCre at MOI=5 (LLC-MK2/F7/M62/#33/A), and cultured at 32°C for two days. The cells were then infected with SeV18+/ΔMΔF-GFP at MOI=0.5, and culture supernatant was recovered over time as fresh medium was added. The recovered supernatant was examined for CIU and HAU. Two days and beyond after infection, the virus was recovered continuously at 10⁸ CIU/mL or more (Fig. 49). Virus production was considered efficient because changes in CIU and HAU occurred in parallel, and most of the produced particles were infectious.

### [Example 30] Structural confirmation of M/F double-deficient SeV

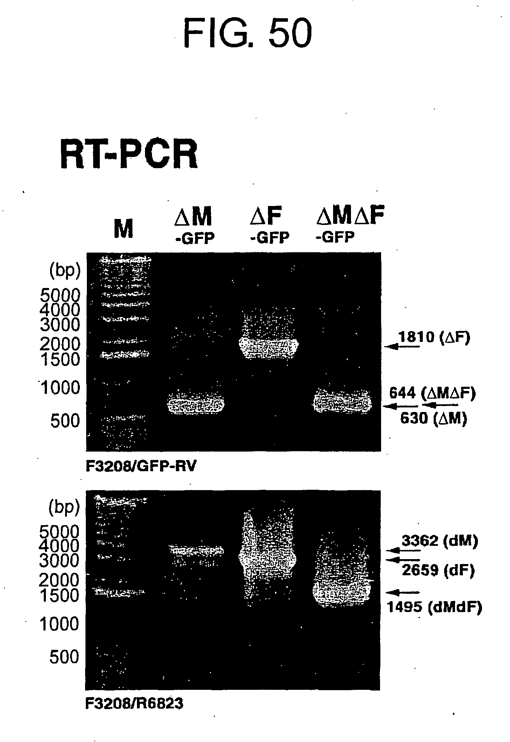

The SeV18+/ΔMΔF-GFP viral gene was confirmed by RT-PCR, and the viral proteins were confirmed using Western-blotting. In RT-PCR, the virus at the P2 stage five days after infection (P2d5) was used. A QIAamp Viral RNA Mini Kit (QIAGEN, Bothell, WA) was used to recover RNA from the viral solution. A Superscript One-Step RT-PCR System (Gibco-BRL, Rockville, MD) was utilized in the cDNA preparation. Both systems were used according to methods described in the protocols attached thereto. Two combinations of primers were used in PCRs for cDNA preparation and RT-PCR: the combination of F3208 on the P gene (5'-agagaacaagactaaggctacc-3'/SEQ ID No: 33) and GFP-RV on the GFP gene (5'-cagatgaacttcagggtcagcttg-3'/SEQ ID No: 36); and the combination of this same F3208, and R6823 on the HN gene (5'-tgggtgaatgagagaatcagc-3'/SEQ ID No: 37). As predicted from the gene structure of the SeV18+/ΔMΔF-GFP, PCR using the former pair amplified a 644bp fragment, and that using the latter amplified a 1495bp fragment (Fig. 50). Amplification using SeV18+/ΔM-GFP and SeV18+/ΔF-GFP resulted in genes of respectively predicted size, indicating a clear difference in size compared to that obtained using the SeV18+/ΔMΔF-GFP. Based on the findings described above, this virus is suggested to comprise a M/F double-deficient gene structure.

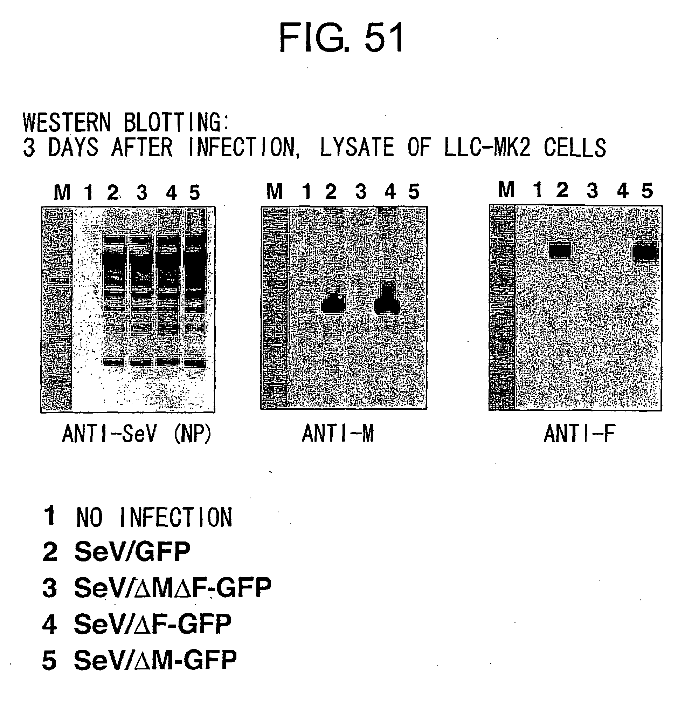

Furthermore, Western-blotting was used to confirm structure from the point of view of the proteins. LLC-MK2 cells were infected at MOI=3 with SeV18+/ΔMΔF-GFP, SeV18+/ΔM-GFP, SeV18+/ΔF-GFP and SeV18+GFP, and recovered two days after the infection. After performing SDS-PAGE, Western-Blotting was performed using an anti-M antibody, anti-F antibody, and a DN-1 antibody (rabbit polyclonal) which mainly recognizes NP protein. The methods are as described in Example 3 and Example 4. Neither M protein nor F protein was observed in SeV18+/ΔMΔF-GFP-infected cells, but NP was observed. Therefore the structure of SeV18+/ΔMΔF-GFP was also confirmed from the viewpoint of proteins (Fig. 51). At this time, SeV18+/ΔF-GFP-infected cells did not exhibit F protein, SeV18+/ΔM-GFP-infected cells did not exhibit M protein, and all of the tested viral proteins were observed in SeV18+GFP.

### [Example 31] Quantitative analysis of the presence or absence of M/F double-deficient SeV secondarily released particles

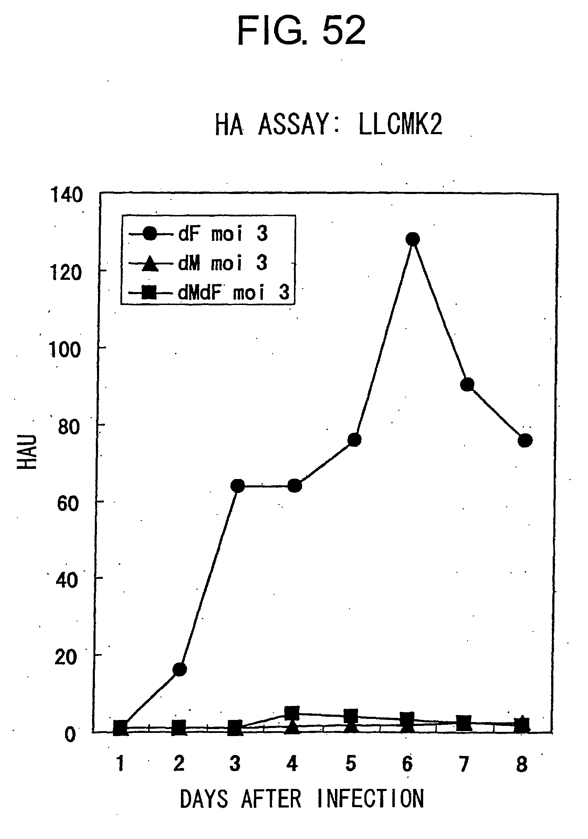

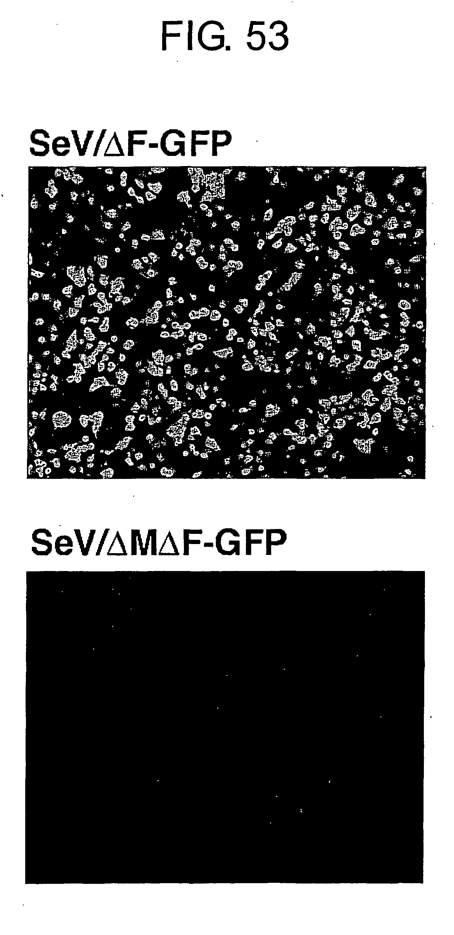

This experiment was conducted over time. Thus, LLC-MK2 cells were infected at MOI=3 with SeV18+/ΔMΔF-GFP, and culture supernatant was recovered at certain time intervals (every 24 hours) and examined for HA activity (Fig. 52). Four days after infection, HA activity was observed, although at low levels. The increased HA activity for SeV18+/ΔMΔF-GFP was assumed to be attributable to HA protein bound to or liberated from cell debris, rather than to VLPs. Furthermore, culture supernatant obtained five days after infection was investigated using a cationic liposome, namely, Dosper Liposomal Transfection Reagent (Roche, Basel, Switzerland). Thus, 100 µl of the culture supernatant and 12.5 µl of Dosper were mixed and allowed to stand at room temperature for ten minutes, and then transfected to LLC-MK2 cells which had been grown confluently in a 6-well plate. Two days later, fluorescence microscopic observation revealed a large number of GFP-positive cells in the culture supernatant of the SeV18+/ΔF-GFP-infected cells containing secondarily released particles, while almost no GFP-positive cells were found in the SeV18+/ΔMΔF-GFP-infected cell culture supernatant (Fig. 53). Based on these findings, it was concluded that in the case of SeV18+/ΔMΔF-GFP, secondary release of particles from infected cells was virtually absent.

### [Example 32] Evaluation of the viral infectivity of M/F double-deficient SeV and M-deficient SeV (in vitro)

The efficiency of transduction and expression in non-dividing cells is an important factor for evaluating the performance of a gene transfer vector. Therefore, assessment is essential. Accordingly, cerebral cortex nerve cells were prepared from the brain of a rat fetus on day 17 of pregnancy. These cells were cultured as a first generation for investigating infectivity in non-dividing cells.

The first generation nerve cell culture derived from the rat cerebral cortex was obtained as follows: On day 17 of pregnancy, a pregnant SD rat was decapitated under ether anesthesia. The abdomen was sterilized using Isodine and 80% ethanol, the uterus removed and placed onto a 10 cm petri dish, and the fetuses were taken from the uterus. Then, the fetal scalp and cranial bone were opened using INOX 5 forceps, and the brain was excised to a 35 mm petri dish. The cerebellum and a part of the brain stem were removed using ophthalmic scissors, the cerebrum was divided into semispheres, and the remainder of the brain stem was removed. The olfactory bulb and the meninx were clipped off using forceps. Finally, the diencephalon and hippocampus were removed using ophthalmic scissors, and the cortex was collected in a petri dish, cut into small pieces using a surgical scalpel, and transferred into a 15 mm centrifuge tube. The cells were treated with 0.3 mg papain/ml at 37°C for ten minutes, treated and then washed with 5 ml of serum-containing medium, and then dispersed. After passing through a 70 µm strainer, the cells were collected by centrifugation and dispersed by gentle pipetting. The cells were then counted. The cells were inoculated onto a poly-L-lysine (PLL) -coated 24 well plate at a density of 2x10⁵ or 4x10⁵ cells/well, and two days later were infected with an M/F double-deficient SeV (SeV18+/ΔMΔF-GFP) and an M-deficient SeV (SeV18+/ΔM-GFP) at MOI=3. Thirty-six hours after infection, the cells were immunostained with MAP2 as a neurocyte-specific marker, and infected cells were identified on the basis of overlap with the GFP-expressing cells (SeV-infected cells) .

MAP2 immunostaining was conducted as follows: The infected cells were washed with PBS, fixed with 4% paraformaldehyde at room temperature for ten minutes, again washed with PBS, and then blocked with a 2% normal goat serum-containing PBS at room temperature for 60 minutes. The cells were reacted with a 200-fold-diluted anti-MAP2 antibody (Sigma, St.Louis, MO) at 37°C for 30 minutes, washed with PBS, and reacted with a 200-fold-diluted secondary antibody (goat anti mouse IgG Alexa568: Molecular Probes Inc., Eugene, OR) at 37°C for 30 minutes. After washing with PBS, cell fluorescence was observed using a fluorescence microscope (DM IRB-SLR: Leica, Wetzlar, Germany).

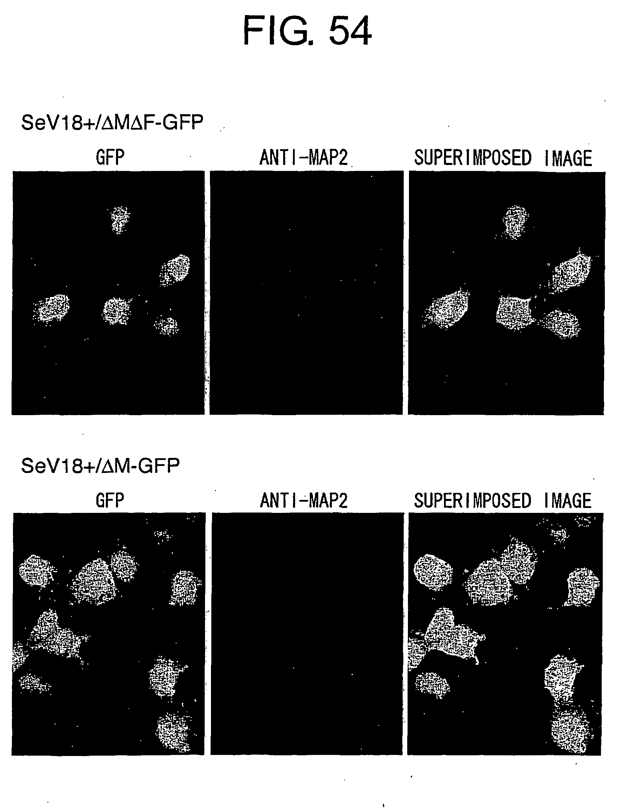

In both the M/F double-deficient SeV (SeV18+/ΔMΔF-GFP) and the M-deficient SeV (SeV18+/ΔM-GFP) , most of the MAP2-positive cells were GFP positive (Fig. 54). That is, most of the prepared nerve cells exhibited efficient SeV infection, and therefore both M/F double-deficient SeV and M-deficient SeV were confirmed to have efficient transduction and expression in non-dividing cells.

### [Example 33] Evaluation of the viral infectivity of M/F double-deficient SeV and M-deficient SeVs (in vivo)

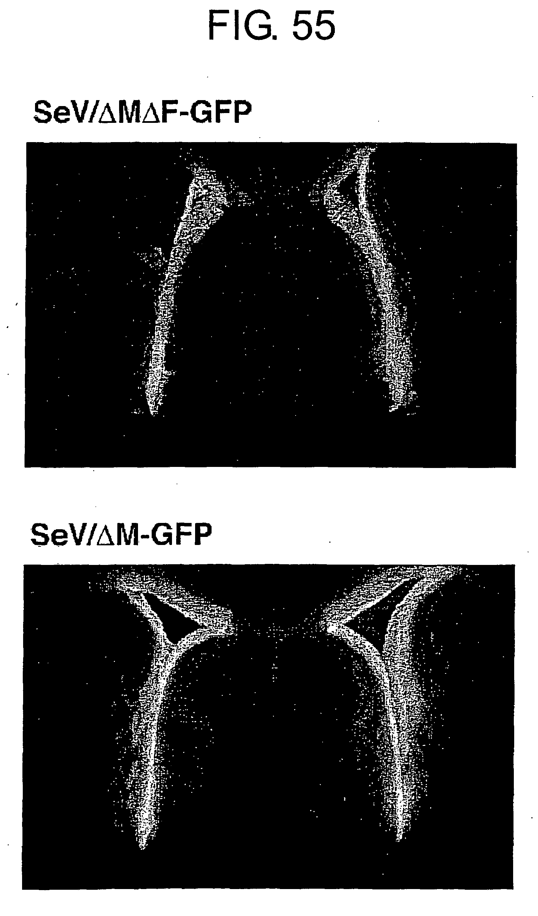

Infectivity *in vivo* was evaluated. 5 µl (1x10⁹ p.f.u/ml) of each of M/F double-deficient SeV (SeV18+/ΔMΔF-GFP) and M-deficient SeV (SeV18+/ΔM-GFP) was administered to the left lateral ventricule of a jird mouse using stereo method. Two days after administration, the mice were sacrificed, their brains removed, and frozen sections prepared. Specimens were observed using a fluorescence microscope, and examined for infection on the basis of GFP fluorescence intensity. Both the M/F double-deficient SeV (SeV18+/ΔMΔF-GFP) and M-deficient SeV (SeV18+/ΔM-GFP) resulted in numerous positive cells in the ependymal cells of both lateral ventricles (Fig. 55). Thus, it was revealed that both M/F double-deficient SeV and M-deficient SeV were capable of achieving efficient gene transduction and expression *in vivo*.

### [Example 34] Evaluation of M/F double-deficient SeV and M-deficient SeV cytotoxicity

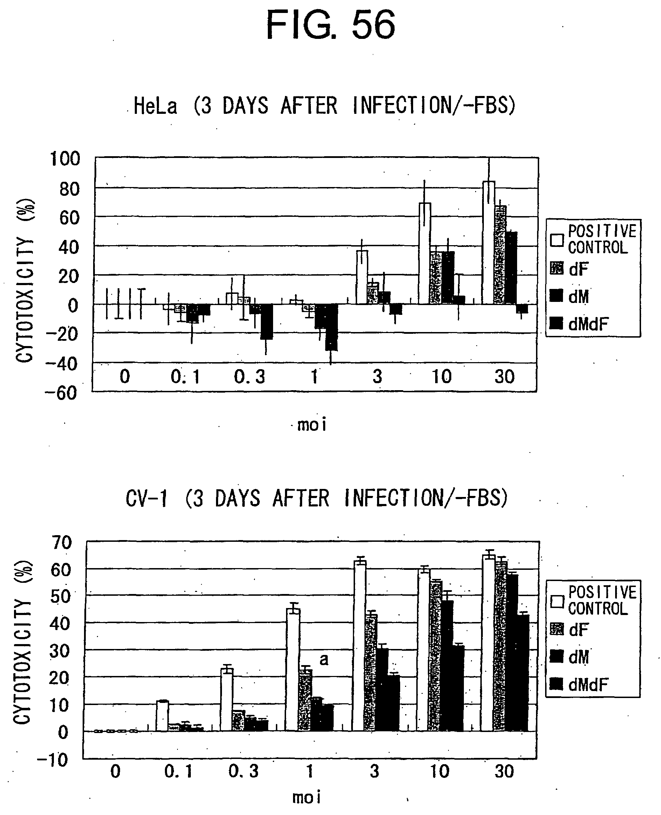

Cytotoxicity was evaluated utilizing cells that enabled observation of SeV-dependent cytotoxicity, namely, using CV-1 cells and HeLa cells. As controls, an added-type SeV (native type: SeV18+GFP) (having replicative ability) and an F-deficient SeV (SeV18+/ΔF-GFP) were also measured at the same time. The experimental method is detailed in Example 6, Example 12 and Example 17. Briefly, CV-1 cells or HeLa cells were inoculated to a 96-well plate at 2.5x10⁴ cells/well (100 µL/well) and cultured. Both cultures employed 10% FBS-containing MEM. After culturing for 24 hours, the SeV18+GFP, SeV18+/ΔF-GFP, SeV18+/ΔM-GFP or SeV18+/ΔMΔF-GFP solution was diluted with 1% BSA-containing MEM in a volume of 5 µl/well, and the solution was added to affect infection. After six hours, the viral solution-containing medium was removed, and replaced with FBS-free MEM medium. Three days after infection, the culture supernatant was sampled, and subjected to cytotoxicity quantification using a Cytotoxicity Detection Kit (Roche, Basel, Switzerland) according to kit instructions. Compared to the added-type SeV, the deletion of the M gene or F gene (in SeV18+/ΔF-GFP and SeV18+/ΔM-GFP) resulted in reduced cytotoxicity, and the combination of these two deletions (in SeV18+/ΔMΔF-GFP) resulted in an additive effect, further reducing cytotoxicity (Fig. 56).

As described above, "the M/F double-deficient SeV vector", successfully reconstituted for the first time in this invention, is a highly versatile gene transfer vector comprising the ability to infect various cells, including non-dividing cells, eliminating almost all secondarily released particles, and exhibiting reduced cytotoxicity.

### Effects of the Invention

The present invention provides methods of testing for, screening for, and producing (-)strand RNA viruses in which particle formation ability is reduced or eliminated. The viruses produced by this invention are useful as gene transfer vectors with fewer side effects against hosts, since they reduce both cytotoxicity and immunoresponse induction caused by secondary release (VLP release) from gene-transferred cells. Vectors provided by the present invention are especially expected to have various applications as vectors for *in vivo* and *ex vivo* gene therapy.

## Claims

1. A method for testing particle formation ability of a (-)strand RNA virus vector, wherein the method comprises detecting localization of M protein in cells in which the vector has been introduced.

2. A method of screening for a (-)strand RNA virus vector whose particle formation ability has been reduced or eliminated, comprising the steps of:
(a) detecting localization of M protein in cells into which the vector has been introduced; and
(b) selecting the vector by which localization has been reduced or eliminated.

3. The method according to claim 1 or 2, wherein the localization of M protein is an aggregation of M proteins on the cell surface.

4. A method of screening for a gene which reduces or eliminates particle formation ability of a (-)strand RNA virus vector, comprising the steps of:
(a) detecting localization of M protein in cells into which the (-)strand RNA virus vector comprising a test gene has been introduced; and
(b) selecting the gene which reduces or eliminates localization.

5. The method according to claim 4, wherein the localization of M protein is an aggregation of M proteins on the cell surface.

6. The method according to claim 4 or 5, wherein the test gene is a mutant of a gene selected from the group consisting of M, F, and HN genes of a (-)strand RNA virus.

7. A method for producing a recombinant (-)strand RNA virus vector whose particle formation ability has been reduced or eliminated, wherein the method comprises reconstituting the (-)strand RNA virus vector comprising a gene which can be identified or isolated by a method according to any one of claims 4 to 6, under a condition where the reduction or elimination of M protein localization by the gene is continuously complemented.

8. A method for producing a recombinant (-)strand RNA virus vector whose particle formation ability has been reduced or eliminated, wherein the method comprises reconstituting the (-)strand RNA virus vector by which the localization of the M gene expression product is reduced or eliminated as a result of the deletion or mutation of the M gene, under a condition where functional M protein is continuously expressed.

9. The method according to claim 8, wherein the step comprises reconstituting, at a permissive temperature, the (-)strand RNA virus vector comprising a temperature-sensitive mutant M gene by which the aggregation of gene products on the cell surface has been reduced or eliminated.

10. The method according to claim 9, wherein the temperature-sensitive mutant M gene is a gene encoding a (-)strand RNA virus M protein, in which an amino acid corresponding to at least one amino acid position selected from the group consisting of G69, T116 and A183 of a Sendai virus M protein has been substituted with another amino acid.

11. The method according to claim 8, wherein the step comprises reconstituting the (-)strand RNA virus vector whose M gene is deleted, under a condition where the M gene, which has been introduced in the chromosome of the cells used for reconstitution, is expressed.

12. A method according to any one of claims 7 to 11, wherein the (-)strand RNA virus vector further comprises the deletion of HN and/or F genes, or comprises a temperature-sensitive mutant HN and/or F genes.

13. The method according to claim 12, wherein the temperature-sensitive mutant HN gene is a gene encoding a (-)strand RNA virus HN protein, in which an amino acid corresponding to at least one amino acid position selected from the group consisting of A262, G264, and K461 of a Sendai virus HN protein, has been substituted with another amino acid.

14. A method according to any one of claims 7 to 13, wherein the (-)strand RNA virus vector further comprises a mutation in the P and/or L gene.

15. The method according to claim 14, wherein the mutation in the P gene is a substitution of an amino acid position of the (-)strand RNA virus P protein, corresponding to E86 and/or L511 of a Sendai virus P protein, with another amino acid.

16. The method according to claim 14 or 15, wherein the mutation in the L gene is a substitution of an amino acid position of the (-)strand RNA virus L protein, corresponding to N1197 and/or K1795 of a Sendai virus L protein, with another amino acid.

17. A method according to any one of claims 7 to 16, wherein the method comprises reconstituting a vector at 35°C or a lower temperature.

18. A method according to any one of claims 1 to 17, wherein the (-)strand RNA virus is a paramyxovirus.

19. The method according to claim 18, wherein the paramyxovirus is a Sendai virus.

20. A recombinant (-)strand RNA virus vector produced by a method according to any one of claims 7 to 14, wherein the particle formation ability of the vector has been reduced or eliminated.

21. A recombinant (-)strand RNA virus, comprising a functional M protein, but whose M protein-encoding sequence is deleted in the genome of the virus.

22. A recombinant (-)strand RNA virus comprising at least one feature selected from the group consisting of the following (a) to (d) :
(a) the M protein encoded in the genome of the virus comprises a substitution of an amino acid, corresponding to at least one amino acid position selected from the group consisting of G69, T116 and A183 of a Sendai virus M protein, with another amino acid;
(b) the HN protein encoded in the genome of the virus comprises a substitution of an amino acid, corresponding to at least one amino acid position selected from the group consisting of A262, G264, and K461 of a Sendai virus HN protein, with another amino acid;
(c) the P protein encoded in the genome of the virus comprises a substitution of an amino acid, corresponding to the amino acid position of E86 or L511 of a Sendai virus P protein, with another amino acid;
(d) the L protein encoded in the genome of the virus comprises a substitution of an amino acid, corresponding to the amino acid position of N1197 and/or K1795 of a Sendai virus L protein or an amino acid of another (-)strand RNA virus M protein homologous thereto, with another amino acid.

23. The virus according to claim 22 comprising the features of at least (a) and (b).

24. The virus according to claim 22 comprising the features of at least (c) and (d).

25. The virus according to claim 22 comprising the features of all of (a) to (d).

26. A virus according to any one of claims 21 to 25, wherein at least one sequence encoding a spike protein in the genome of the virus is further deleted.

27. ' The virus according to claim 26, wherein the spike protein is an F protein.

28. A virus according to any one of claims 21 to 27, wherein the (-)strand RNA virus is a paramyxovirus.

29. The virus according to claim 28, wherein the paramyxovirus is a Sendai virus.

30. A recombinant virus according to any one of claims 21 to 29, which is used for reducing cytotoxicity upon gene introduction.

31. A recombinant virus according to any one of claims 21 to 30, which is used for inhibiting the reduction in the expression level of an introduced gene upon gene introduction.

32. A recombinant virus according to any one of claims 21 to 31, which is used for inhibiting the release of a virus-like particle (VLP) from a cell into which a virus has been introduced upon gene transduction.

33. An aqueous solution comprising a recombinant virus according to any one of claims 21 to 32 at a level of 10⁶ CIU/ml or higher.
